# EUROPEAN PATENT APPLICATION

(11) **EP 4 631 941 A1**
(43) Date of publication of application: **15.10.2025**
(21) Application number: 23899451.1
(22) Date of filing: 09.08.2023
(51) Int. Cl.: C07D 417/14, A61K 31/506, A61P 35/00, A61P 35/02

(54) **BENZOTHIAZOLE PYRIMIDINEAMINE DYRK2 INHIBITOR, PREPARATION METHOD THEREFOR, AND USE THEREOF**

(30) Priority: 06.12.2022 CN 202211553361
(71) Applicant: Jiangsu Tasly Diyi Pharmaceutical Co., Ltd., Huai'an, Jiangsu 223003 (CN)
(72) Inventor: YANG, Peng, Huaian, Jiangsu 223003 (CN); XIAO, Yibei, Huaian, Jiangsu 223003 (CN); YUAN, Kai, Huaian, Jiangsu 223003 (CN); HAO, Haiping, Huaian, Jiangsu 223003 (CN); XIA, Fei, Huaian, Jiangsu 223003 (CN); ZHENG, Mingming, Huaian, Jiangsu 223003 (CN); CHEN, Weijiao, Huaian, Jiangsu 223003 (CN); JI, Minghui, Huaian, Jiangsu 223003 (CN); YANG, Huanaoyu, Huaian, Jiangsu 223003 (CN); ZHUANG, Xujie, Huaian, Jiangsu 223003 (CN)
(74) Representative: Gulde & Partner
(86) International application number: PCT/CN2023/111890
(87) International publication number: WO 2024/119866

(57) **Abstract**

Disclosed in the present invention are a benzothiazole pyrimidineamine DYRK2 inhibitor, a preparation method therefor, and a use thereof. The compound of the present invention can inhibit the proliferation of cancer cells, and can be used for preparing drugs for treating cancer or tumor related diseases.

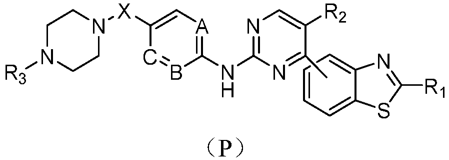

## Description

### TECHNICAL FIELD

The invention is related to the field of pharmaceutical chemistry, in particular to a benzothiazole pyrimidineamine DYRK2 inhibitor, a preparation method therefor and use thereof in preparing a medicament for treating related diseases.

### BACKGROUND ARTS

The human bispecific tyrosine-phosphorylation-regulated kinase DYRKs is an evolutionarily conservative kinase family characterized by kinase activity at its own tyrosine residues and serine/threonine sites of other proteins. DYRKs are a five-member serine/threonine CMGC kinase family. Double-specific tyrosine phosphorylation-regulated kinase 2 (Dual-specificity tyrosine phosphorylation-regulated kinase 2, DYRK2) belongs to the DYRK family and has both tyrosine kinase activity and serine/threonine kinase activity. It has multiple functions of regulating cell proliferation, apoptosis and differentiation.

Bispecific tyrosine phosphorylation-regulated kinases (DYRK) belong to the CMGC kinase group together with CDK and MAPK family kinases. In the human body, DYRK family kinases have five subtypes. Among them, DYRK2 is an important regulator of 26S protein kinase, which regulates the Thr25 site of Rpt3 subunit in the granule by phosphorylating 26S proteasome, thereby enhancing the activity of proteasome. DYRK2 is found to have a significantly increased expression in tumors and plays an important role in tumor proliferation and growth. At present, small molecule inhibitors of DYRK2 have shown remarkable anticancer effects in cancers such as prostate cancer, triple negative breast cancer and multiple myeloma. Recently, a research group reported on the specific small-molecule inhibition of DYRK2, LDN192960 (Banerjee et al., 2019). The crystal structure analysis and biochemical studies revealed the molecular mechanism of selective inhibition of DYRK2 by LDN192960. LDN192960 was able to reduce the proteasome activity by inhibiting the activity of DYRK2, thereby alleviating the progression of triple negative breast cancer and multiple myeloma, indicating that targeted DYRK2 is expected to become an effective regimen for the treatment of these two kinds of tumors.

However, the activity of DYRK2 inhibitors in the prior art is still unsatisfactory, and there is an urgent need to provide a DYRK2 inhibitor with better activity.

### SUMMARY OF THE INVENTION

The purpose of the invention is to provide a small molecule DYRK2 inhibitor, or a stereoisomer thereof, or a pharmaceutically acceptable salt thereof, a pharmaceutical composition comprising the small molecule DYRK2 inhibitor and the preparation method therefor, and use thereof in the preparation of a medicament for treating related cancer or tumor disease.

The invention provides a compound represented by Formula (P), or a stereoisomer thereof, or a pharmaceutically acceptable salt thereof, 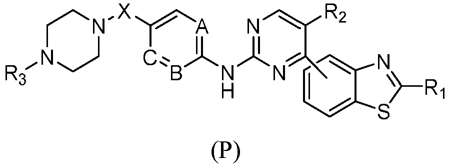 wherein
the said X is each independently selected from: O, NH, S(O)₂, C(O) or -(CH₂)ₙ-, n is 0, or 1, or 2;
the said A, B, C is each independently selected from C or N;
the said R₁ is each independently selected from: hydrogen, deuterium, halogen, hydroxy, mercapto, cyano, nitro, C₂-C₈ alkenyl, C₂-C₈ alkynyl, C₁-C₈ alkyl, halo-C₁-C₈ alkyl, C₁-C₈ alkoxy, C₃-C₈ cycloalkyl, C₆-C₁₀ aryl, C₅-C₁₀ heteroaryl, C₄-C₈ heterocyclyl or -NR₄R₅, wherein the said R₄, R₅ is each independently selected from: H, C₁-C₈ alkyl, C₃-C₈ cycloalkyl, C₆-C₁₀ aryl, C₅-C₁₀ heteroaryl or C₄-C₈ heterocyclyl;
the said R₂ is each independently selected from hydrogen, deuterium, hydroxy, mercapto, cyano, halogen, nitro, C₃-C₈ cycloalkyl, C₁-C₈ alkyl or C₁-C₈ alkoxy ;
the said R₃ is each independently selected from hydrogen, deuterium, C₁-C₈ alkyl, halo-C₁-C₈ alkyl, C₁-C₈ alkoxy, C₃-C₈ cycloalkyl, -C(O)OC₁-C₈ alkyl, -C(O)C₃-C₈ cycloalkyl, C₆-C₁₀ aryl, C₃-C₁₀ heteroaryl, C₄-C₈ heterocyclyl or -C(O) R₆;
the said R₆ is each independently selected from C₁-C₈ alkyl, C₁-C₈ alkoxy, C₃-C₈ cycloalkyl or C₆-C₁₀ aryl.

Preferably, the compound represented by Formula (P), or a stereoisomer thereof, or a pharmaceutically acceptable salt thereof is a compound represented by Formula (P-I) or (P-II), or a stereoisomer thereof, or a pharmaceutically acceptable salt thereof as shown below: 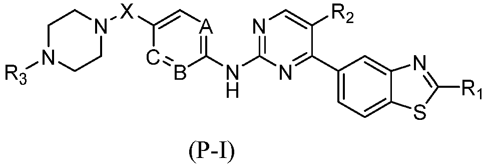 or 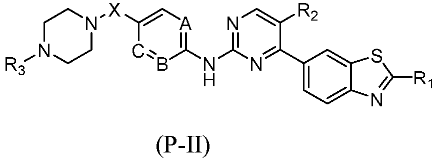 wherein
X is each independently selected from O, NH, S(O)₂, C(O) or -(CH₂)ₙ-, n is 0, or 1, or 2;
the A, B, C is each independently selected from C or N;
the R₁ is each independently selected from: hydrogen, deuterium, halogen, hydroxy, mercapto, cyano, nitro, C₂-C₈ alkenyl, C₂-C₈ alkynyl, C₁-C₈ alkyl, halo-C₁-C₈ alkyl, C₁-C₈ alkoxy, C₃-C₈ cycloalkyl, C₆-C₁₀ aryl, C₃-C₁₀ heteroaryl, C₄-C₈ heterocyclyl or -NR₄R₅, wherein R₄, R₅ is each independently selected from: H, C₁-C₈ alkyl, C₃-C₈ cycloalkyl, C₆-C₁₀ aryl, C₃-C₁₀ heteroaryl or C₄-C₈ heterocyclyl;
the R₂ is each independently selected from hydrogen, deuterium, hydroxy, mercapto, cyano, halogen, nitro, C₃-C₈ cycloalkyl, C₁-C₈ alkyl or C₁-C₈ alkoxy ;
the R₃ is each independently selected from hydrogen, deuterium, C₁-C₈ alkyl, halo-C₁-C₈ alkyl, C₁-C₈ alkoxy, C₃-C₈ cycloalkyl, -C(O)OC₁-C₈ alkyl, -C(O)C₃-C₈ cycloalkyl, C₆-C₁₀ aryl, C₃-C₁₀ heteroaryl, C₄-C₈ heterocyclyl or -C(O) R₆;
the R₆ is each independently selected from C₁-C₈ alkyl, C₁-C₈ alkoxy, C₃-C₈ cycloalkyl or C₆-C₁₀ aryl.

Preferably, a compound represented by Formula (P-I) or (P-II), or a stereoisomer thereof, or a pharmaceutically acceptable salt thereof:
wherein
the X is each independently selected from C(O) or -(CH₂)ₙ-, n is 1;
the A, B, C is each independently selected from C or N;
the R₁ is each independently selected from hydrogen, halogen, C₁-C₈ alkyl, hydroxy, -NR₄R₅, wherein R₄, R₅ is selected from H, C₁-C₆ alkyl or C₄-C₈ heterocyclyl;
the R₂ is each independently selected from hydrogen, halogen, hydroxy, C₁-C₆ alkyl or C₁-C₆ alkoxy ;
the R₃ is each independently selected from hydrogen, C₁-C₆ alkyl, -C(O)OC₁-C₆ alkyl, -C(O)C₃-C₆ cycloalkyl, C₆-C₁₀ aryl, or -C(O) R₆;
the R₆ is each independently selected from C₁-C₆ alkyl, C₁-C₆ alkoxy, C₃-C₆ cycloalkyl or C₆-C₁₀ aryl.

More preferably, a compound represented by Formula (P-I) or (P-II), or a stereoisomer thereof, or a pharmaceutically acceptable salt thereof:
wherein
the X is each independently selected from C(O) or -CH₂-;
the A, B, C is each independently selected from C or N;
the R₁ is selected from hydrogen, methyl or -NR₄R₅, wherein R₄, R₅ is each independently selected from hydrogen, methyl, ethyl, propyl, isopropyl, cyclopentyl or cyclohexyl;
the R₂ is each independently selected from hydrogen, methyl, ethyl, propyl, methoxy, fluoro or isopropyl;
the R₃ is each independently selected from hydrogen, methyl, ethyl, propyl, isopropyl, n-butyl, isobutyl, -C(O)OC₁-C₆ alkyl, phenyl or -C(O)R₆;
the R₆ is each independently selected from methyl, ethyl, methoxy, ethoxy or cyclopropyl.

Most preferably, the compound represented by Formula (P-I) or (P-II), or a stereoisomer thereof, or a pharmaceutically acceptable salt thereof is selected from the compounds with following structures, or a stereoisomer thereof, or a pharmaceutically acceptable salt thereof:

| Code Name | Chemical Name | Structure Formula |
|---|---|---|
| P-1 | 4-(benzothiazole-5-yl)-N-( 5-((4-isopropylpiperazine-1-yl)methyl)pyridine-2-yl) -5-methylpyrimidine-2-am ine | 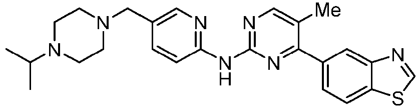 |
| P-2 | 4-(benzothiazole-5-yl)-5-methyl-N-(5-(piperazine-1 -methylene)piperidine-2-yl )pyrimidine-2-amine hydrochloride | 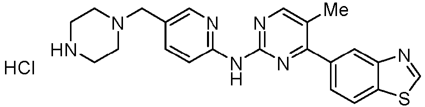 |
| P-3 | (6-((4-(benzothiazole-5-yl) -5-methylpyrimidine-2-yl) amino)pyridine-3-yl)(4-eth ylpiperazine-1-yl)ketone | 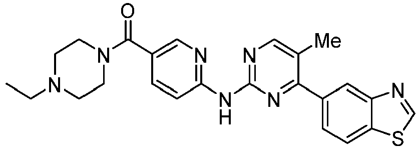 |
| P-4 | tert-butyl 4-(6-((4-(benzothiazole-5-yl)-5-methylpyrimidine-2-yl)amino)nicotinoyl)pipera zine-1-carboxylate | 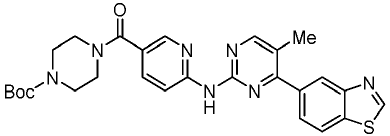 |
| P-5 | (6-((4-(benzothiazole-5-yl) -5-methylpyrimidine-2-yl) amino)pyridine-3-yl)(piper azine-1-yl)ketone hydrochloride | 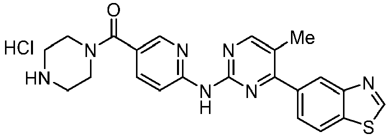 |
| P-6 | (6-((4-(benzothiazole-5-yl) -5-methoxypyrimidine-2-y l)amino)pyridine-3-yl)(pip erazine-1-yl)ketone hydrochloride | 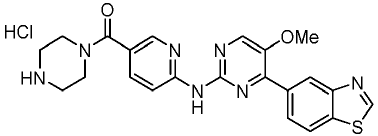 |
| P-7 | 4-(benzothiazole-5-yl)-5-methoxy-N-(5-(piperazine -1-ylmethyl)pyridine-2-yl) pyrimidine-2-amine hydrochloride | 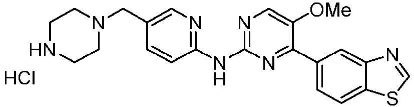 |
| P-8 | 4-(benzothiazole-5-yl)-N-( 5-(piperazine-1-methylene )pyridine-2-yl)pyrimidine-2-amine hydrochloride | 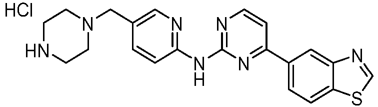 |
| P-9 | (6-((4-(benzothiazole-5-yl) pyrimidine-2-yl)amino)pyr idine-3-yl)(4-ethylpiperazi ne-1-yl)ketone | 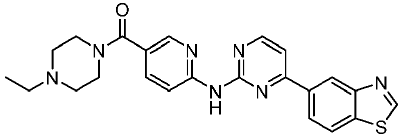 |
| P-10 | tert-butyl 4-(6-((4-(benzothiazole-5-yl)pyrimidine-2-yl)amino) nicotinoyl)piperazine-1-ca rboxylate | 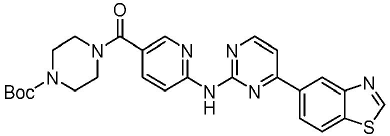 |
| P-11 | (6-((4-(benzothiazole-5-yl) pyrimidine-2-yl)amino)pyr idine-3-yl)(piperazine-1-yl )ketone hydrochloride | 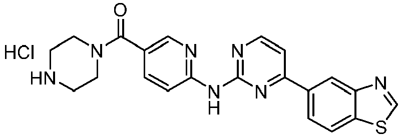 |
| P-12 | 4-(benzothiazole-5-yl)-N-( 5-((4-ethylpiperazine-1-yl) methyl)pyridine-2-yl)-5-fl uoropyridine-2-amine | 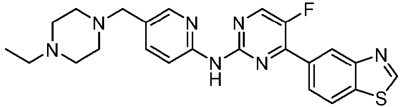 |
| P-13 | 4-(benzothiazole-5-yl)-5-fl uoro-N-(5-(piperazine-1-m ethylene)pyridine-2-yl)pyr imidine-2-amine hydrochloride | 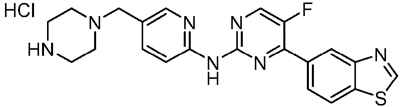 |
| P-14 | (6-((4-(benzothiazole-5-yl) -5-fluoropyrimidine-2-yl)a mino)pyridine-3-yl)(4-eth ylpiperazine-1-yl)ketone | 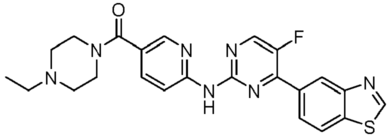 |
| P-15 | 1-(4-(6-((4-(benzothiazole-5-yl)-5-fluoropyrimidine-2 -yl)amino)nicotinoyl)piper azine-1-yl)ethyl-1-one | 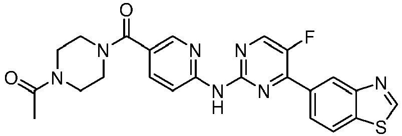 |
| P-16 | (4-(6-((4-(benzothiazole-5-yl)-5-fluoropyrimidine-2-y l)amino)nicotinoyl)piperaz ine-1-yl)(cyclopropyl)keto ne | 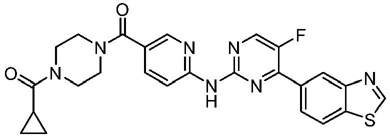 |
| P-17 | (6-((4-(benzothiazole-5-yl) -5-fluoropyrimidine-2-yl)a mino)pyridine-3-yl)(4-phe nylpiperazine-1-yl)ketone | 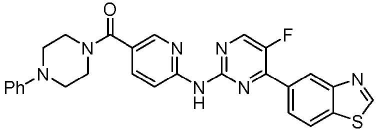 |
| P-18 | ethyl 4-(6-((4-(benzothiazole-5-yl)-5-fluoropyrimidine-2-y l)amino)nicotinoyl)piperaz ine-1-carboxylate | 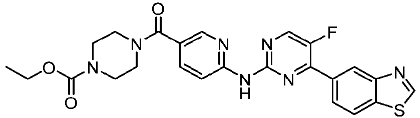 |
| P-19 | tert-butyl 4-(6-((4-(benzothiazole-5-yl)-5-fluoropyrimidine-2-y l)amino)nicotinoyl)piperaz ine-1-carboxylate | 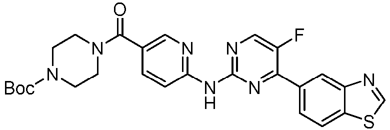 |
| P-20 | (6-((4-(benzothiazole-5-yl) -5-fluoropyrimidine-2-yl)a mino)pyridine-3-yl)(pipera zine-1-yl)ketone hydrochloride | 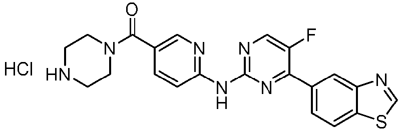 |
| P-21 | (2-((4-(benzothiazole-5-yl) -5-fluoropyrimidine-2-yl)a mino)pyrimidine-5-yl)(4-e thylpiperazine-1-yl)ketone | 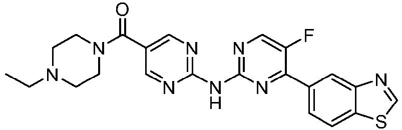 |
| P-22 | tert-butyl 4-(2-((4-(benzothiazole-5-yl)-5-fluoropyrimidine-2-y l)amino)pyrimidine-5-carb onyl)piperazine-1-carboxy late | 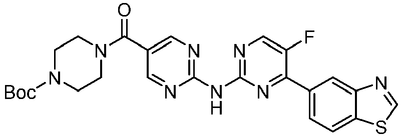 |
| P-23 | (2-((4-(benzothiazole-5-yl) -5-fluoropyrimidine-2-yl)a mino)pyrimidine-5-yl)(pip erazine-1-yl)ketone hydrochloride | 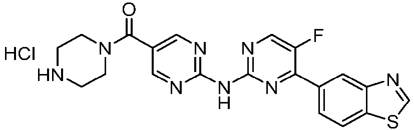 |
| P-24 | (5-((4-(benzothiazole-5-yl) -5-fluoropyrimidine-2-yl)a mino)pyridine-2-yl)(4-eth ylpiperazine-1-yl)ketone | 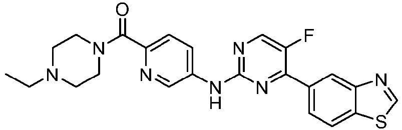 |
| P-25 | tert-butyl 4-(5-((4-(benzothiazole-5-yl)-5-fluoropyrimidine-2-y l)amino)pyridine-2-yl)pipe razine-1-carboxylate | 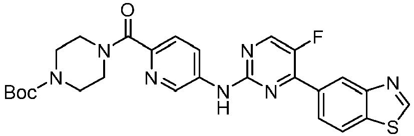 |
| P-26 | (5-((4-(benzothiazole-5-yl) -5-fluoropyrimidine-2-yl)a mino)pyridine-2-yl)(pipera zine-1-yl)ketone hydrochloride | 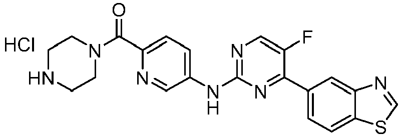 |
| P-27 | (6-((4-(2-(dimethylamino) benzothiazole-5-yl)-5-fluo ropyrimidine-2-yl)amino)p yridine-3-yl)(piperazine-1-yl)ketone hydrochloride | 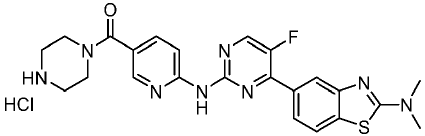 |
| P-28 | (6-((4-(2-(diethylamino)be nzothiazole-5-yl)-5-fluoro pyrimidine-2-yl)amino)pyr idine-3-yl)(piperazine-1-yl )ketone hydrochloride | 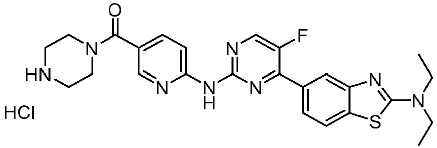 |
| P-29 | (5-((4-(2-(dimethylamino) benzothiazole-5-yl)-5-fluo ropyrimidine-2-yl)amino)p yridine-2-yl)(4-ethylpipera zine-1-yl)ketone | 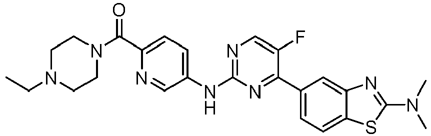 |
| P-30 | (5-((4-(2-(dimethylamino) benzothiazole-5-yl)-5-fluo ropyrimidine-2-yl)amino)p yridine-2-yl)(4-isopropylpi perazine-1-yl)ketone | 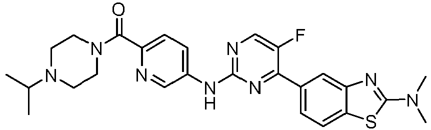 |
| P-31 | tert-butyl 4-(5-((4-(2-(dimethylamin o)benzothiazole-5-yl)-5-fl uoropyrimidine-2-yl)amin o) pyridine-1-yl)piperazine-1 -carboxylate | 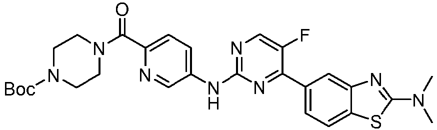 |
| P-32 | (5-((4-(2-(dimethylamino) benzothiazole-5-yl)-5-fluo ropyrimidine-2-yl)amino)p yridine-2-yl)(piperazine-1-yl)ketone hydrochloride | 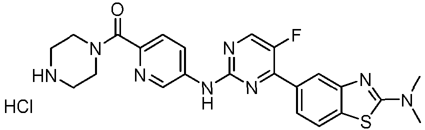 |
| P-33 | (5-((4-(2-(diethylamino) benzothiazole-5-yl)-5-fluo ropyrimidine-2-yl)amino)p yridine-2-yl)(4-ethylpipera zine-1-yl)ketone | 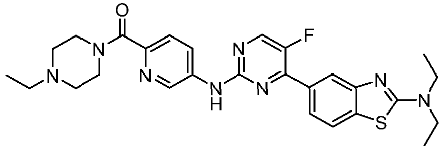 |
| P-34 | (5-((4-(2-(diethylamino)be nzothiazole-5-yl)-5-fluoro pyrimidine-2-yl)amino)pyr idine-2-yl)(4-isopropylpip erazine-1-yl)ketone | 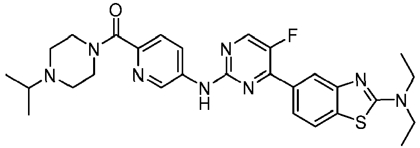 |
| P-35 | 1-(4-(6-((4-(2-(dimethyla mino) benzothiazole-6-yl)-5 -fluo ropyrimidine-2-yl)amino)n icotinoyl)piperazine-1-yl)e thyl-1-one | 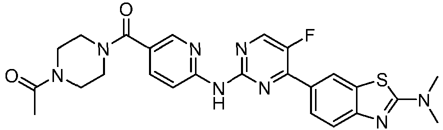 |
| P-36 | (4-(cyclopropylcarbonyl)p iperazine-1-yl)(6-((4-(2-(di methylamino) benzothiazole-6-yl)-5-fluo ropyrimidine-2-yl)amino)p yridine-3-yl)ketone | 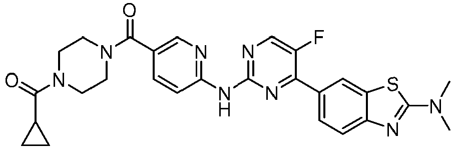 |
| P-37 | (6-((4-(2-(dimethylamino) benzothiazole-6-yl)-5-fluo ropyrimidine-2-yl)amino)p yridine-3-yl)(4-aryl piperazine-1-yl)ketone | 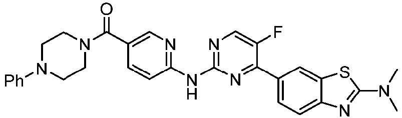 |
| P-38 | ethyl 4-(6-((4-(2-(dimethylamin o)benzothiazole-6-yl)-5-fl uoropyrimidine-2-yl)amin o)nicotinoyl)piperazine-1-carboxylate | 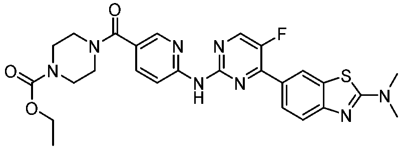 |
| P-39 | 1-(4-(6-((4-(2-(diethylami no)benzothiazole-6-yl)-5-f luoropyrimidine-2-yl)amin o)nicotinoyl)piperazine-1-yl)ethyl-1-one | 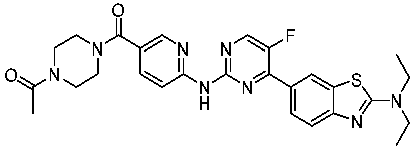 |
| P-40 | (4-(cyclopropylcarbonyl)p iperazine-1-yl)(6-((4-(2-(di ethylamino) benzothiazole-6-yl)-5-fluo ropyrimidine-2-yl)amino)p yridine-3-yl)ketone | 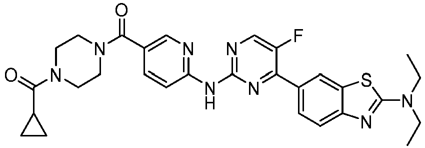 |
| P-41 | 1-(4-(6-((4-(2-(cyclopenyl amino)benzothiazole-6-yl) -5-fluoropyrimidine-2-yl)a mino)nicotinoyl)piperazin e-1-yl)ethyl-1-one | 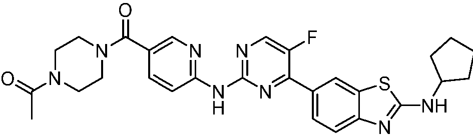 |
| P-42 | (4-(6-((4-(2-(cyclopenyla mino) benzothiazole-6-yl)-5-fluo ropyrimidine-2-yl)amino) nicotinoyl)piperazine-1-yl) (cyclopropyl)ketone | 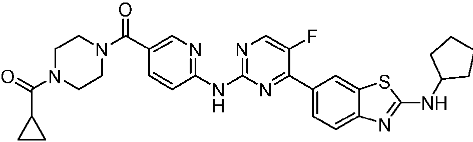 |
| P-43 | (6-((4-(benzothiazole-6-yl) -5-fluoropyrimidine-2-yl)a mino)pyridine-3-yl)(4-isop ropylpiperazine-1-yl)keton e | 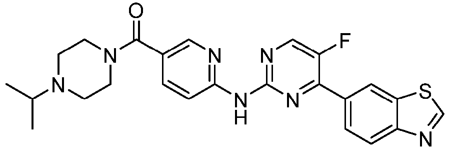 |
| P-44 | 1-(4-(6-((4-(benzothiazole-6-yl)-5-fluoropyrimidine-2 -yl)amino) nicotinoyl)piperazine-1-yl) ethyl-1-one | 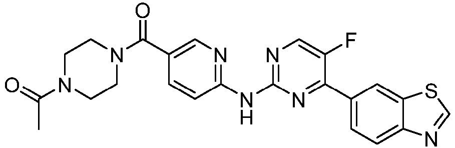 |
| P-45 | (4-(6-((4-(benzothiazole-6-yl)-5-fluoropyrimidine-2-y l)amino) nicotinoyl)piperazine-1-yl) (cyclopropyl)ketone | 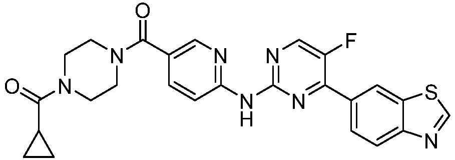 |
| P-46 | 4-(benzothiazole-6-yl)-N-( 5-((4-isopropylpiperazine-1-yl)methyl)pyridine-2-yl) -5-methylpyrimidine-2-am ine | 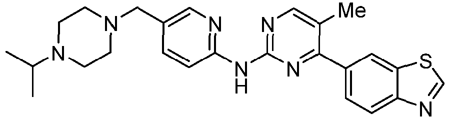 |
| P-47 | tert-butyl 4-(6-((4-(benzothiazole-6-yl)-5-methylpyrimidine-2-yl)amino) nicotinoyl)piperazine-1-ca rboxylate | 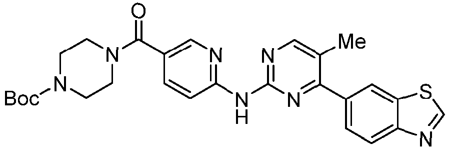 |
| P-48 | (6-((4-(benzothiazole-6-yl) -5-methylpyrimidine-2-yl) amino)pyridine-3-yl)(4-iso propylpiperazine-1-yl)keto ne | 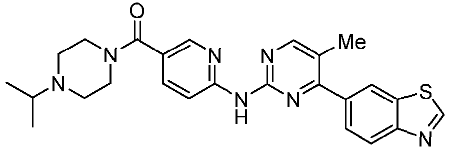 |
| P-49 | (6-((4-(benzothiazole-6-yl) -5-methylpyrimidine-2-yl) amino) pyridine-3-yl)(piperazine-1-yl)ketone hydrochloride | 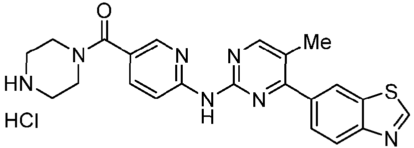 |
| P-50 | 4-(benzothiazole-6-yl)-N-( 5-((4-isopropylpiperazine-1-yl)methyl)pyridine-2-yl) -5-methoxypyrimidine-2-a mine | 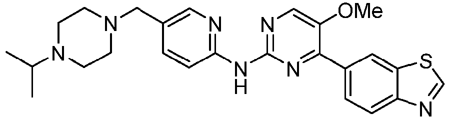 |
| P-51 | tert-butyl 4-(6-((4-(benzothiazole-6-yl)-5-methoxypyrimidine-2-yl)amino) nicotinoyl)piperazine-1-ca rboxylate | 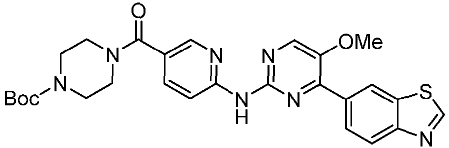 |
| P-52 | (6-((4-(benzothiazole-6-yl) -5-methoxypyrimidine-2-y l)amino)pyridine-3-yl)(4-i sopropylpiperazine-1-yl)k etone | 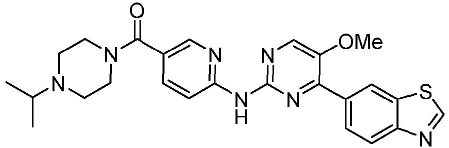 |
| P-53 | (6-((4-(benzothiazole-6-yl) -5-methoxypyrimidine-2-y l)amino) pyridine-3-yl)(piperazine-1-yl)ketone hydrochloride | 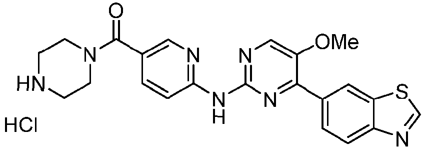 |
| P-54 | (6-((4-(benzothiazole-6-yl) pyrimidine-2-yl)amino)pyr idine-3-yl)(4-isopropylpip erazine-1-yl)ketone | 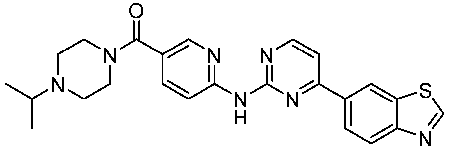 |
| P-55 | 5-fluoro-N-(5-((4-isopropy lpiperazine-1-yl)methyl)py ridine-2-yl)-4-(2-methylbe nzothiazole-6-yl)pyrimidin e-2-amine | 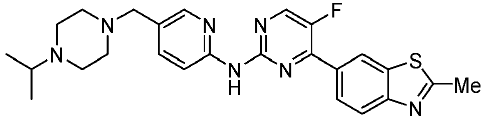 |
| P-56 | (6-((5-fluoro-4-(2-methylb enzothiazole-6-yl)pyrimidi ne-2-yl)amino) pyridine-3-yl)(4isopropylp iperazine-1-yl)ketone | 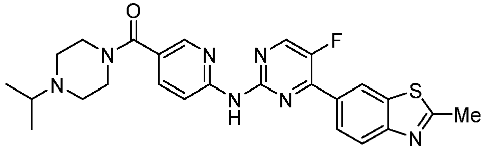 |
| P-57 | 1-(4-(6-((5-fluoro-4-(2-me thylbenzothiazole-6-yl)pyr imidine-2-yl)amino) nicotinoyl)piperazine-1-yl) ethyl-1-one | 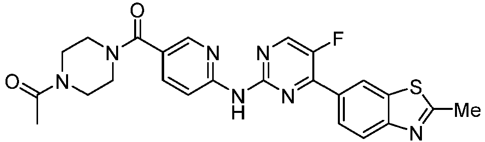 |
| P-58 | (4-(cyclopropylcarbonyl)p iperazine-1-yl)(6-((5-fluor o-4-(2-methylbenzothiazol e-6-yl)pyrimidine-2-yl)am ino)pyridine-3-yl)ketone | 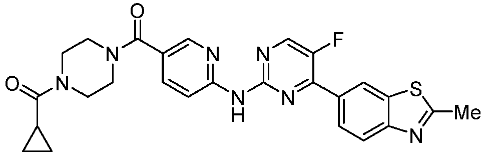 |

The invention further provides a compound represented by Formula (P), or a stereoisomer thereof, or a pharmaceutically acceptable salt thereof:
wherein the pharmaceutically acceptable salt is a salt formed by the compound represented by Formula (P) and hydrochloric acid, sulfuric acid, phosphoric acid, methanesulfonic acid, acetic acid, trichloroacetic acid, propionic acid, butyric acid, maleic acid, p-toluenesulfonic acid, malic acid, malonic acid, cinnamic acid, citric acid, fumaric acid, camphoric acid, gluconic acid, aspartic acid and tartaric acid, and preferably, the pharmaceutically acceptable salt is a hydrochloride salt of the compound represented by Formula (P).

The invention further provides a preparation method for the compound represented by Formula (P), or a stereoisomer thereof, or a pharmaceutically acceptable salt thereof, including a step of subjecting Intermediate Compound (A) and Intermediate Compound (B) to a coupling reaction under a catalyst: 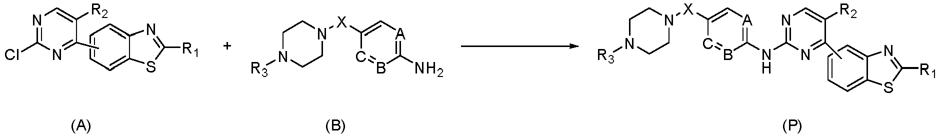 wherein, the definitions of the substituents X, A, B, C, R₁, R₂ and R₃ are identical to the above;

Preferably, the catalyst is a palladium catalyst;

Preferably, the reaction is conducted under argon protection atmosphere; and the reaction temperature is 55-125°C, and the reaction time is 5-24 hours.

The invention further provides a pharmaceutical composition, comprising the compound represented by Formula (P), or a stereoisomer thereof, or a pharmaceutically acceptable salt thereof.

The invention further provides the use of the compound represented by Formula (P), or a stereoisomer thereof, or a pharmaceutically acceptable salt thereof, or the pharmaceutical composition comprising the compound represented by Formula (P), or a stereoisomer thereof, or a pharmaceutically acceptable salt thereof in the preparation of a medicament of an inhibitor targeting DYRK2,
wherein the medicament of an inhibitor targeting DYRK2 is a medicament for treating cancer or tumor related disease, preferably a medicament for treating or preventing lung cancer, leukemia, breast cancer, prostate cancer, multiple myeloma, liver cancer, gastric cancer, bone cancer, brain cancer, head and neck cancer, intestinal cancer, pancreatic cancer, bladder cancer, testicular cancer, ovarian cancer or endometrial cancer.

The invention has the beneficial technical effects that, the invention provides a benzothiazole pyrimidineamine DYRK2 inhibitor, which has strong inhibitory activity on DYRK2 kinase activity, the IC₅₀ (nM) value of which can reach 0.1nM-1000nM, preferably the IC₅₀ (nM) value of 1nM-100nM, and the IC₅₀ (nM) value of the inhibitory activity of some compounds on DYRK2 of less than 10nM.

The following is an explanation and explanation of the terminology of the present invention:
The "cancer" or "malignant tumor" refers to any of a variety of diseases characterized by uncontrolled abnormal proliferation of cells, the body (i.e., metastasis) of the ability of affected cells to spread to other sites locally or through the bloodstream and lymphatic system, and any of a number of characteristic structural and/or molecular features. The "cancer cells" refer to cells that undergo early, middle or advanced stages of multistep tumor progression. The cancer is lung cancer, leukemia, breast cancer, prostate cancer, multiple myeloma, liver cancer, stomach cancer, bone cancer, brain cancer, head and neck cancer, intestinal cancer, pancreatic cancer, bladder cancer, testicular cancer, ovarian cancer, or endometrial cancer.

The compounds and derivatives provided in the present invention may be named according to the IUPAC (International Union for Pure and Applied Chemistry) or CAS (Chemical Abstract Service, Columbus, OH) nomenclature system.

Definition of terms used in connection with the present invention: the initial definition of a group or term provided herein applies to that group or term throughout the specification unless otherwise indicated. Terms not specifically defined herein should be given the meanings given to them by those skilled in the art based on the disclosure and context.

The "substituted" means that a hydrogen atom in a molecule is replaced by a different atom or molecule.

The minimum and maximum values of the carbon atom content in the hydrocarbon group are indicated by a prefix, for example, the prefix C_{a~b} alkyl denotes any alkyl group containing from "a" to "b" carbon atoms. Thus, for example, "C_{1~6} alkyl" refers to an alkyl group containing 1 to 6 carbon atoms. "C_{1~8} alkyl" refers to an alkyl group containing 1 to 8 carbon atoms.

The "alkyl" refers to a saturated hydrocarbon chain having a specified number of member atoms. For example, C_{1~8} alkyl refers to an alkyl group having 1 to 8 member atoms, and C_{1~6} alkyl refers to an alkyl group having 1 to 6 member atoms, for example 1 to 4 member atoms, and 1 to 3 member atoms. The alkyl group may be linear or branched. Representative branched alkyl groups have one, two or three branches. The alkyl group may optionally be substituted with one or more substituents as defined herein. Alkyl groups include methyl, ethyl, propyl (n-propyl and isopropyl), butyl (n-butyl, isobutyl and tert-butyl), pentyl (n-pentyl, isopentyl and neopentyl), hexyl, and the like. The alkyl group may also be part of other groups such as C₁~C₈ alkoxy, C₁~C₆ alkoxy.

The "cycloalkyl" and "cycloalkane" refer to saturate or partially saturated cyclic group having carbon atoms and no cyclic heteroatoms and having a single ring or multiple rings (including fused, and). For polycyclic systems having aromatic and non-aromatic rings without cyclic heteroatoms, the term "cycloalkyl" (e.g., 5,6,7,8,-tetrahydronaphthalene-5-yl) is used when the point of attachment is at a non-aromatic carbon atom. The term "cycloalkyl" includes cycloalkenyl groups such as cyclohexenyl. Examples of the cycloalkyl group include, for example, adamantyl, cyclopropyl, cyclobutyl, cyclohexyl, cyclopentyl, cyclooctyl, cyclopentenyl, cyclohexenyl, and the like. Examples of cycloalkyl groups that include polycyclic alkyl ring systems are bicyclohexyl, bicyclopentyl, bicyclooctyl, and the like.

The "alkenyl" refers to a straight or branched chain hydrocarbyl group having 2 to 10 carbon atoms and in some embodiments 2 to 6 carbon atoms or 2 to 4 carbon atom and having at least one olefinically unsaturation site (> C=C <). For example, (Cₐ~C_{b}) alkenyl refers to an alkenyl group having a to b carbon atoms and is intended to include, for example, vinyl, propenyl, isopropenyl, 1,3- butadienyl, and the like.

The "alkynyl" refers to a linear monovalent hydrocarbon group or a branched monovalent hydrocarbon group containing at least one triple bond. The term "alkynyl" is also intended to include those hydrocarbyl groups having a triple bond and a double bond. For example, C₂~C₆ alkynyl is intended to include ethynyl, propynyl, and the like.

The "halogen" is fluorine, chlorine, bromine or iodine.

The "haloalkyl" or "halo-alkyl" means that the hydrogen atom in the alkyl group may be substituted with one or more halogen atoms. For example, C_{1~8} haloalkyl refers to an alkyl group having 1 to 8 carbon atoms in which hydrogen atoms are substituted by one or more halogen atoms; C_{1~4} haloalkyl refers to an alkyl group having 1 to 4 carbon atoms in which hydrogen atoms are substituted by one or more halogen atoms.

The "heterocycle", "heterocycloalkyl", "heterocycloalkane" refers to a saturated or non-aromatic unsaturated ring containing at least one heteroatom; wherein the heteroatom refers to a nitrogen atom, an oxygen atom, a sulfur atom. The term "heterocyclyl" is a heterocycloalkyl group and represents a monocyclic or fused ring containing one or more heteroatoms of N, O or S. The term "C₄~C₈ heterocyclic group" refers to a heterocyclic group having from 4 to 8 carbon atoms in its ring. C₄~C₈ heterocyclic group include, but are not limited to, piperazinyl, morpholino, piperidinyl, pyrrolidinyl, and that like.

The "heteroaryl ring" refers to an aromatic unsaturated ring containing at least one heteroatom; wherein the heteroatom refers to a nitrogen atom, an oxygen atom, a sulfur atom. The term "heteroaryl" mean a monocyclic or fused cyclyl group containing one, two, three or four groups selected from that group consist of N, O or S ring heteroatoms, with the rest of the ring atoms being C, in addition with fully conjugated π electronic system. The term "C₃~C₁₀ heteroaryl" refers to a heteroaryl group having from 3 to 10 carbon atoms in its ring. C₃~C₁₀ heteroaryl include, but are not limited to, pyrrole, furan, thiophene, imidazole, oxazole, thiazole, pyrazole, pyrimidine, pyridine.

The term "C₆~C₁₀ aryl" refers to a full carbon monocyclic or fused polycyclic group of 6 to 10 carbon atoms having a fully conjugated π electronic system. It typically includes, but are not limit to, phenyl, naphthyl.

The term "pharmaceutically acceptable" means that a carrier, vector, diluent, excipient, and/or salt formed is generally chemically or physically compatible with the other components comprising the pharmaceutical dosage form and physiologically compatible with the receptor.

The pharmaceutical composition can be any reusable pharmaceutical preparation form, such as oral, injection, topical administration, and that like, and the oral dosage form include but not limited to tablet, capsule, oral liquid, granule, pill, suspension, injection selected from water acupuncture and powder acupuncture, and topical preparation selected from patch and ointment. All formulation may be prepared according to conventional techniques of pharmacy, for example, that compounds of the present invention, or stereoisomers thereof, or pharmaceutically acceptable salt thereof, are used as the pharmaceutically active ingredient, and pharmaceutically acceptable carriers are added if necessary, to prepare the above pharmaceutical dosage forms suitable for administration, wherein the unit dose of the pharmaceutically active ingredient may be 0.1mg to 1000mg, for example, each tablet contains 0.1mg to 1000mg, preferably 5 to 5-500mg, of the pharmaceutically active ingredient.

The terms "salt", "pharmaceutically acceptable salt", "pharmaceutically acceptable salt" refer to the acidic and/or basic salts of the above compounds or stereoisomers thereof with inorganic and/or organic acids and bases, and also include zwitterionic salts (internal salts) and also quaternary ammonium salts such as alkyl ammonium salts. These salts may be obtained directly in the final isolation and purification of the compound. It may also be obtained by suitably (e. g ., equiv .) mixing the above compounds, or stereoisomers thereof, with an amount of acid or base. These salts may be obtained by precipitation in solution and collection by filtration, or by recovery after evaporation of the solvent, or by reaction in an aqueous medium followed by freeze-drying. The salt of that present invention may be hydrochloride, sulfate, citrate, besylate, hydrobromide, hydrofluoride, phosphate, acetate, propionate, succinate, oxalate, malate, succinate, fumarate, maleate, tartrate or trifluoroacetate of the compound.

### BRIEF DESCRIPTION OF THE DRAWINGS

Fig. 1 is a schematic representation of that significant inhibition of prostate cancer DU145 and 22Rv1 cell proliferation by the compound of Example 20 of the present invention;
Fig. 2 is a graph showing changes in body weight and organ weight of animals in the acute toxicity test of Example 20 of the present invention;
Fig. 3 is a schematic representation of that significant inhibition of prostate cancer DU145 and 22Rv1 cell proliferation by the compound of Example 40 of the present invention;
Fig. 4 is a graph showing changes in body weight and organ weight of animals in the acute toxicity test of Example 40 of the present invention;
Fig. 5 is the result of *in vivo* activity of the compound of Example 20 of the present invention against prostate cancer;
Fig. 6 is the result of *in vivo* activity of the compound of Example 40 of the present invention against prostate cancer;

### DETAILED DESCRIPTION OF THE INVENTION

The following examples facilitate a better understanding of the invention but do not limit it. Experimental methods in the following examples are conventional methods unless otherwise specified. The test materials used in the examples below, unless otherwise specified, were purchased from the conventional biochemical reagent store. The present application is described in detail below with reference to specific embodiments.

### Intermediate Preparation Example 1:

### Synthesis of 5-(4,4,5,5-tetramethyl-1,3,2-dioxaborolane-2-yl)benzothiazole (A-1)

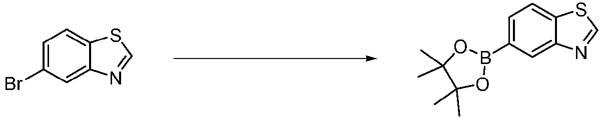

5-bromobenzothiazole (0.43 g, 2.0 mmol) was dissolved in 1,4-dioxane (10 mL), and then was added with pinacol boronate (0.53 g, 2.1 mmol), Pd (dppf)Cl₂ (22 mg, 0.06 mmol), potassium acetate (0.59 g, 6.0 mmol). The system was replaced with argon three times, and was heated to 100°C to react for 8 h. The mixture was cooled, filtrated, and concentrated, and was purified by flash silica column chromatography to give the compound 5-(4,4,5,5-tetramethyl-1,3,2-dioxaborolane-2-yl)benzothiazole (0.44 g, 86% yield). ¹HNMR (400 MHz, CDCl₃) *δ* 9.00 (s, 1H), 8.60 - 8.59 (m, 1H), 7.97 (dd, *J=* 8.0, 0.7 Hz, 1H), 7.86 (dd, *J =* 8.0, 1.0 Hz, 1H), 1.39 (s, 12H).

### Intermediate Preparation Example 2:

### Synthesis of 5-(2-chloro-5-methylpyrimidine-4-yl)benzothiazole (A-2)

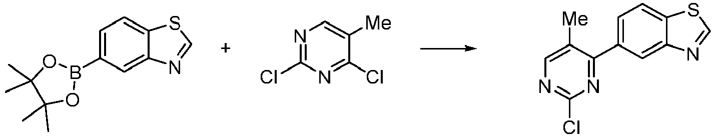

Compound 2,4-dichloro-5-methylpyrimidine (0.28 g, 1.4 mmol) was weighed and added into a 250 mL three-necked flask, and then was added with Pd(PPh₃)₂Cl₂(21 mg, 0.03 mmol), sodium carbonate (0.27 g, 2.5 mmol), ethylene glycol dimethyl ether (10 mL) and water (0.25 mL). The system was replaced with argon three times, and was heated to 80 °C. Compound 5-(4,4,5,5-tetramethyl-1,3,2-dioxaborolane-2-yl)benzothiazole A-1(0.26 g, 1.0 mmol) was dissolved in ethylene glycol dimethyl ether (5 mL), and was added dropwisely into the three-necked flask to react for 16 h. The mixture was cooled, filtrated, and concentrated, and was purified by flash silica column chromatography to give the compound 5-(2-chloro-5-methylpyrimidine-4-yl)benzothiazole A-2(0.21 g, 80% yield). ¹HNMR (300 MHz, CDCl₃) δ 9.10 (s, 1H), 8.56 (s, 1H), 8.39 (dd, *J =* 1.7, 0.6 Hz, 1H), 8.11 (dd, *J =* 8.4, 0.6 Hz, 1H), 7.78 (dd, *J =* 8.4, 1.7 Hz, 1H), 2.47 (s, 3H).

### Intermediate Preparation Example 3:

### Synthesis of 5-(2-chloro-5-methoxypyrimidine-4-yl)benzothiazole (A-3)

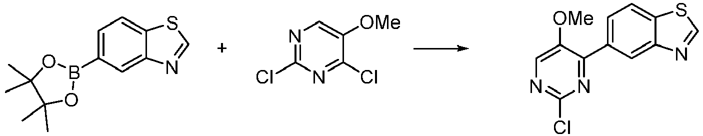

Referring to the synthesis method of Compound A-2, the yield was 78%. ¹HNMR (300 MHz, CDCl₃) *δ* 9.07 (s, 1H), 8.98 (dd, *J* = 1.7, 0.6 Hz, 1H), 8.37 (s, 1H), 8.26 (dd, *J* = 8.6, 1.7 Hz, 1H), 8.07 (dd, *J =* 8.6, 0.6 Hz, 1H), 4.05 (s, 3H).

### Intermediate Preparation Example 4:

### Synthesis of 5-(2-chloropyrimidine-4-yl)benzothiazole (A-4)

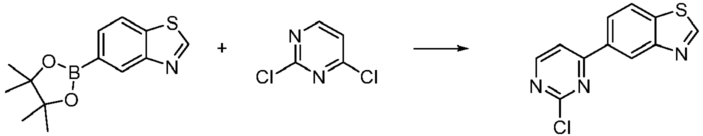

Referring to the synthesis method of Compound A-2, the yield was 73%. ¹HNMR (300 MHz, CDCl₃) *δ* 9.11 (s, 1H), 8.83 (d, *J =* 1.7 Hz, 1H), 8.71 (d, *J =* 5.3 Hz, 1H), 8.29 (dd, *J* = 8.5, 1.7 Hz, 1H), 8.12 (d, *J =* 8.5 Hz, 1H), 7.79 (d, *J =* 5.3 Hz, 1H).

### Intermediate Preparation Example 5:

### Synthesis of 5-(2-chloro-5-fluoropyrimidine-4-yl)benzothiazole (A-5)

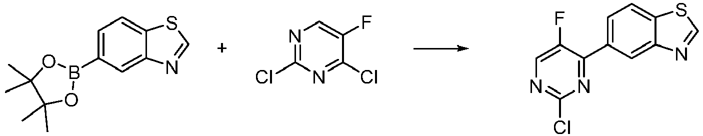

Referring to the synthesis method of Compound A-2, the yield was 78%. ¹HNMR (400 MHz, CDCl₃) *δ* 9.11 (s, 1H), 8.94 (s, 1H), 8.58 (d, *J =* 3.1 Hz, 1H), 8.29 (dd, *J =* 8.5, 1.7 Hz, 1H), 8.13 (d, *J =* 8.5 Hz, 1H).

### Intermediate Preparation Example 6:

### Synthesis of 6-bromo-N,N-dimethylbenzothiazole-2-amine (A-6)

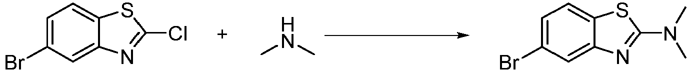

5-bromo-2-chlorobenzothiazole (1 g, 4 mmol) and dimethylamine (0.72 g, 16 mmol) were dissolved in THF(15 mL), and were heated to 75 °C for refluxing, in order to react for 6 h. The mixture was purified by flash silica column chromatography to give the compound 5-bromo-N,N-dimethylbenzothiazole-2-amine A-6 (0.84 g, 82% yield). ¹HNMR (400 MHz, CDCl₃) *δ* 7.69 (d, *J=* 1.9 Hz, 1H), 7.42 (d, *J =* 8.3 Hz, 1H), 7.14 (dd, *J =* 8.3, 1.9 Hz, 1H), 3.19 (s, 6H).

### Intermediate Preparation Example 7:

### Synthesis of N,N-dimethyl-5-(4,4,5,5-tetramethyl-1,3,2-dioxaborolane-2-yl)benzothiazole (A-7)

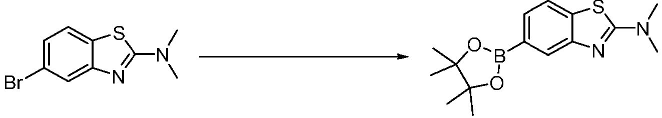

Referring to the synthesis method of Compound A-1, the yield was 75%. ¹H NMR (400 MHz, CDCl₃) *δ* 8.03 - 8.02 (m, 1H), 7.60 (dd, *J =* 7.9, 0.6 Hz, 1H), 7.48 (dd, *J* = 7.9, 1.1 Hz, 1H), 3.20 (s, 6H), 1.34 (s, 12H).

### Intermediate Preparation Example 8:

### Synthesis of 5-(2-chloro-5-fluoropyrimidine-4-yl)-N,N-dimethylbenzothiazole-2-amine (A-8)

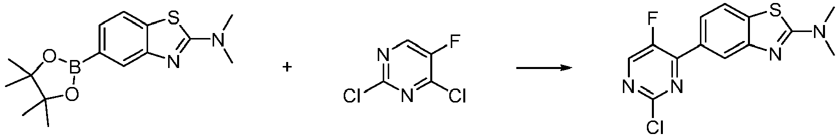

Referring to the synthesis method of Compound A-2, the yield was 74%. ¹H NMR (400 MHz, CDCl₃) *δ* 8.49 (d, *J =* 3.3 Hz, 1H), 8.36 - 8.34 (m, 1H), 7.90 - 7.87 (m, 1H), 7.72 (d, *J =* 8.4 Hz, 1H), 3.24 (s, 6H).

### Intermediate Preparation Example 9:

### Synthesis of 5-bromo-N,N-diethylbenzothiazole-2-amine (A-9)

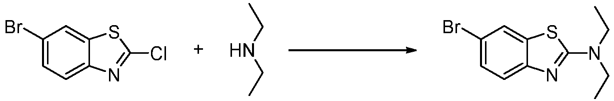

5-bromo-2-chlorobenzothiazole (2.49 g, 10 mmol), diethylamine (2.93 g, 40 mmol) was dissolved in THF (100 mL), and was heated to 75 °C for refluxing, in order to react for 12 h. The mixture was purified by flash silica column chromatography to give the compound 5-bromo-N,N-diethylbenzothiazole-2-amine A-9 (2.37 g, 83% yield). ¹H NMR (400 MHz, CDCl₃) *δ* 7.66 (d, *J* = 1.9 Hz, 1H), 7.39 (d, *J* = 8.3 Hz, 1H), 7.12 (dd, *J* = 8.3, 1.9 Hz, 1H), 3.55 (q, *J* = 7.1 Hz, 4H), 1.27 (t, *J =* 7.1 Hz, 6H).

### Intermediate Preparation Example 10:

### Synthesis of N,N-diethyl-5-(4,4,5,5-tetramethyl-1,3,2-dioxaborolane-2-yl)benzothiazole (A-10)

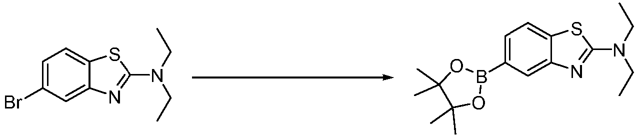

Referring to the synthesis method of Compound A-1, the yield was 72%. ¹H NMR (400 MHz, CDCl₃) *δ* 8.01 - 8.00 (m, 1H), 7.58 (d, *J =* 7.8 Hz, 1H), 7.45 (dd, *J =* 7.8, 1.1 Hz, 1H), 3.56 (q, *J =* 7.0 Hz, 4H), 1.34 (s, 12H), 1.28 (t, *J =* 7.0 Hz, 6H).

### Intermediate Preparation Example 11:

### Synthesis of 5-(2-chloro-5-fluoropyrimidine-4-yl)-N,N-diethylbenzothiazole-2-amine (A-11)

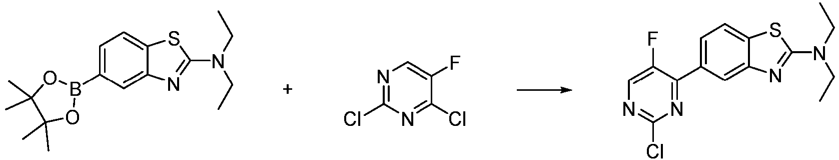

Referring to the synthesis method of Compound A-2, the yield was 72%. ¹H NMR (400 MHz, CDCl₃) *δ* 8.47 (d, *J =* 3.3 Hz, 1H), 8.32 (s, 1H), 7.87 - 7.84 (m, 1H), 7.69 (d, *J =* 8.4 Hz, 1H), 3.60 (q, *J =* 7.2 Hz, 4H), 1.31 (t, *J* = 7.2 Hz, 6H).

### Intermediate Preparation Example 12:

### Synthesis of 6-(2-chloro-5-fluoropyrimidine-4-yl)-N,N-dimethylbenzothiazole-2-amine (A-12) Step 1: Synthesis of 6-bromo-N,N-dimethylbenzothiazole-2-amine (A-12-1)

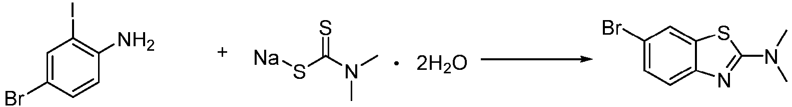

4-bromo-2-iodoaniline (0.60 g, 2.0 mmol), sodium dimethyldithiocarbamate dihydrate (0.72 g, 4.0 mmol), copper acetate (0.36 g, 2.0 mmol) and potassium carbonate (0.55g, 4.0 mmol) were weighed and dissolved in DMF(10 mL), and were heated to 120 °C to react for 6 h. The mixture was cooled, filtrated, and concentrated, and was purified by flash silica column chromatography to give the compound 6-bromo-N,N-dimethylbenzothiazole-2-amine (A-12-1) (0.44 g, 85% yield). ¹H NMR (400 MHz, CDCl₃) δ 7.69 (d, *J =* 1.9 Hz, 1H), 7.41 - 7.35 (m, 2H), 3.20 (s, 6H).

### Step 2: Synthesis of N,N-dimethyl-6-(4,4,5,5-tetramethyl-1,3,2-dioxaborolane-2-yl)benzothiazole-2-amine (A-12-2)

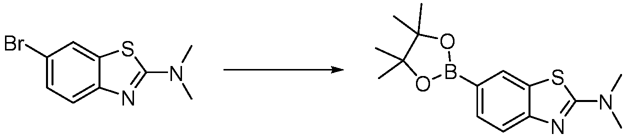

Intermediate A-12-1 (0.51 g, 2.0 mmol) was dissolved in DMF(10 mL), and then was added with pinacol boronate (0.53 g, 2.1 mmol), Pd (dppf)Cl₂ (22 mg, 0.06 mmol), potassium acetate (0.59 g, 6.0 mmol). The system was replaced with argon three times, and was heated to 80°C to react for 24 h. The mixture was cooled, filtrated, and concentrated, and was purified by flash silica column chromatography to give the compound N,N-dimethyl-6-(4,4,5,5-tetramethyl-1,3,2-dioxaborolane-2-yl)benzothiazole-2-amine A-12-2 (0.54 g, 88% yield). MS (M + H)⁺: found, 305.3.

### Step 3: Synthesis of 6-(2-chloro-5-fluoropyrimidine-4-yl)-N,N-dimethylbenzothiazole-2-amine (A-12)

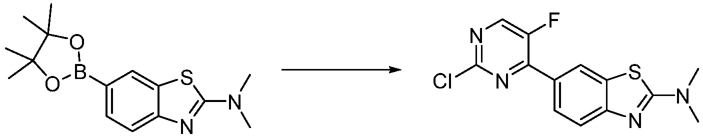

Compound 2,4-dichloro-5-fluoropyrimidine (0.23 g, 1.4 mmol) was weighed and added into a 250 mL three-necked flask, and then was added with Pd(PPh₃)₂Cl₂(21 mg, 0.03 mmol), sodium carbonate (0.27 g, 2.5 mmol), ethylene glycol dimethyl ether (10 mL) and H₂O(0.25 mL). The system was replaced with argon three times, and was heated to 80 °C. N,N-dimethyl-6-(4,4,5,5-tetramethyl-1,3,2-dioxaborolane-2-yl)benzothiazole-2-amine A-12-2 (0.3 g, 1.0 mmol) was dissolved in ethylene glycol dimethyl ether (5 mL), and was added dropwisely into the three-necked flask to react for 16 h. The mixture was cooled, filtrated, and concentrated, and was purified by flash silica column chromatography to give the compound 6-(2-chloro-5-fluoropyrimidine-4-yl)-N,N-dimethylbenzothiazole-2-amine A-12 (0.25 g, 80% yield). ¹H NMR (400 MHz, CDCl₃) δ 8.49 (d, *J =* 1.9 Hz, 1H), 8.45 (d, *J =* 3.6 Hz, 1H), 8.17 - 8.14 (m, 1H), 7.62 (d, *J =* 8.7 Hz, 1H), 3.27 (s, 6H).

### Intermediate Preparation Example 13:

### Synthesis of 6-(2-chloro-5-fluoropyrimidine-4-yl)-N,N-diethylbenzothiazole-2-amine (A-13) Step 1: Synthesis of 6-bromo-N,N-diethylbenzothiazole-2-amine (A-13-1)

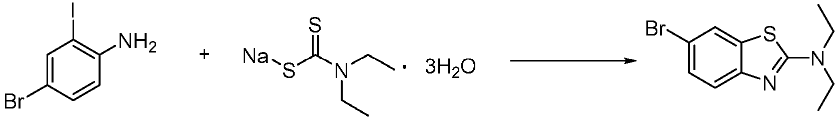

4-bromo-2-iodoaniline (0.60 g, 2.0 mmol), sodium diethyldithiocarbamate trihydrate (0.90 g, 4.0 mmol), copper acetate (0.36 g, 2.0 mmol) and potassium carbonate (0.55g, 4.0 mmol) were weighed and dissolved in DMF(10 mL), and were heated to 120 °C to react for 6 h. The mixture was cooled, filtrated, and concentrated, and was purified by flash silica column chromatography to give the compound 6-bromo-N,N-dimethylbenzothiazole-2-amine (A-13-1) (0.46 g, 80% yield). MS (M + H)⁺: found, 285.1.

### Step 2: Synthesis of N,N-diethyl-6-(4,4,5,5-tetramethyl-1,3,2-dioxaborolane-2-yl)benzothiazole-2-amine (A-13-2)

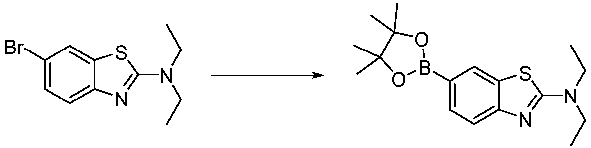

Referring to the synthesis method of Compound (A-12-2), the yield was 90%. MS (M + H)⁺: found, 333.3.

### Step 3: Synthesis of 6-(2-chloro-5-fluoropyrimidine-4-yl)-N,N-diethylbenzothiazole-2-amine (A-13)

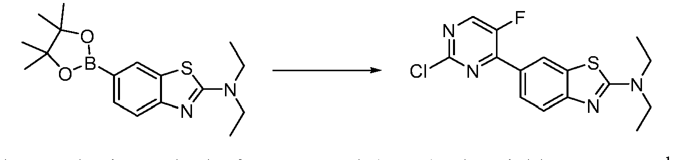

Referring to the synthesis method of Compound (A-12), the yield was 82%. ¹H NMR (300 MHz, CDCl₃) δ 8.44 (dd, *J =* 8.8, 2.7 Hz, 2H), 8.13 (d, *J =* 8.6 Hz, 1H), 7.58 (d, *J =* 8.7 Hz, 1H), 3.61 (q, *J =* 7.2 Hz, 4H), 1.32 (t, *J =* 7.2 Hz, 6H).

### Intermediate Preparation Example 14:

### Synthesis of (6-aminopyridine-3-yl)(4-ethylpiperazine-1-yl)ketone (B-1)

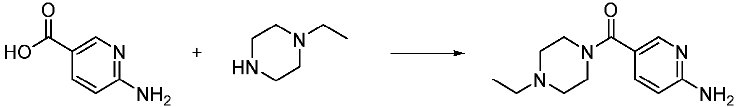

6-aminonicotinic acid (0.69 g, 5 mmol), N,N'-carbonyldiimidazole (1.62 g, 10 mmol) were weighed and dissolved in DMF(30 mL), and were heated to 70°C to react for 10 min, and further stirred under room temperature for 1 hour. The mixture was added with N-ethylpiperazine (0.69g, 6 mmol), and reacted under room temperature overnight, concentrated, and was purified by flash silica column chromatography to give the compound B-1 (0.97g, yield of 83%). ¹H NMR (400 MHz, CDCl₃) *δ* 8.18 (dd, *J =* 2.4, 0.8 Hz, 1H), 7.54 (dd, *J =* 8.5, 2.4 Hz, 1H), 6.49 (dd, *J =* 8.5, 0.9 Hz, 1H), 4.95 (s, 2H), 3.67 (brs, 4H), 2.48 - 2.43 (m, 6H), 1.10 (t, *J =* 7.2 Hz, 3H).

### Intermediate Preparation Example 15:

### Synthesis of tert-butyl 4-(6-aminonicotinoyl)piperazine-1-carboxylate (B-2)

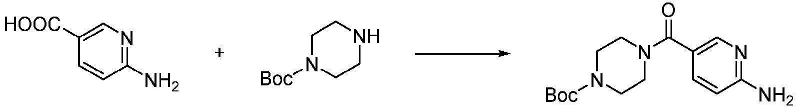

Referring to the synthesis method of Compound (B-1), the yield was 85%. ¹H NMR (400 MHz, CDCl₃) δ 8.19 (dd, *J =* 2.3, 0.8 Hz, 1H), 7.57 (dd, *J =* 8.5, 2.3 Hz, 1H), 6.51 (dd, *J =* 8.5, 0.8 Hz, 1H), 4.70 (s, 2H), 3.62 - 3.58 (m, 4H), 3.48 - 3.45 (m, 4H), 1.48 (s, 9H).

### Intermediate Preparation Example 16:

### Synthesis of 1-(4-(6-aminonicotinoyl)piperazine-1-yl)ethane-1-one (B-3)

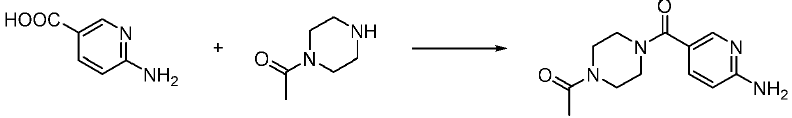

Referring to the synthesis method of Compound (B-1), the yield was 79%. ¹H NMR (400 MHz, CDCl₃) δ 8.20 (dd, *J =* 2.3, 0.8 Hz, 1H), 7.56 (dd, *J =* 8.5, 2.3 Hz, 1H), 6.52 (dd, *J =* 8.5, 0.8 Hz, 1H), 4.88 (s, 2H), 3.70 - 3.58 (m, 8H), 2.14 (s, 3H).

### Intermediate Preparation Example 17:

### Synthesis of 4-(6-aminonicotinoyl)piperazine-1-yl)(cyclopropyl)ketone (B-4)

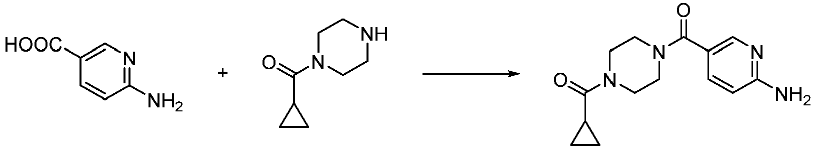

Referring to the synthesis method of Compound (B-1), the yield was 79%. ¹H NMR (400 MHz, CDCl₃) δ 8.19 (dd, *J =* 2.4, 0.8 Hz, 1H), 7.55 (dd, *J =* 8.6, 2.4 Hz, 1H), 6.52 (dd, *J =* 8.6, 0.8 Hz, 1H), 5.12 (s, 2H), 3.74 - 3.62 (m, 8H), 1.77 - 1.73 (m, 1H), 1.02 - 0.99 (m, 2H), 0.83 - 0.75 (m, 2H).

### Intermediate Preparation Example 18:

### Synthesis of (6-aminopyridine-3-yl)(4- aryl piperazine-1-yl)ketone (B-5)

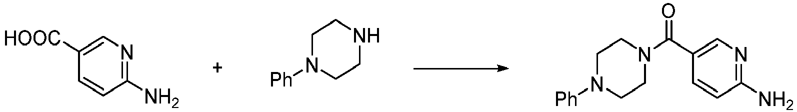

Referring to the synthesis method of Compound (B-1), the yield was 73%. ¹H NMR (400 MHz, CDCl₃) δ 8.22 (dd, *J =* 2.3, 0.8 Hz, 1H), 7.58 (dd, *J= 8.5,* 2.3 Hz, 1H), 7.30 - 7.27 (m, 2H), 6.95 - 6.92 (m, 2H), 6.90 - 6.85 (m, 1H), 6.52 (dd, *J =* 8.5, 0.8 Hz, 1H), 4.86 (s, 2H), 3.81 - 3.79 (m, 4H), 3.22 - 3.19 (m, 4H).

### Intermediate Preparation Example 19:

### Synthesis of ethyl 4-(6-aminonicotinoyl)piperazine-1-carboxylate (B-6)

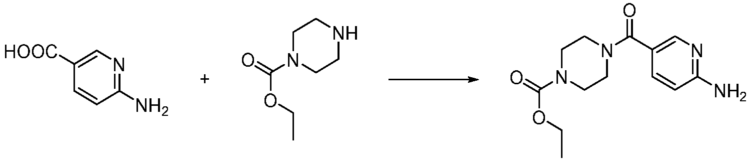

Referring to the synthesis method of Compound (B-1), the yield was 73%. ¹H NMR (400 MHz, CDCl₃) 6 8.18 (s, 1H), 7.53 (d, *J =* 8.6 Hz, 1H), 6.52 (d, *J =* 8.6 Hz, 1H), 5.13 (s, 2H), 4.18 - 4.13 (m, 2H), 3.63 - 3.44 (m, 8H), 1.30 - 1.25 (m, 3H).

### Intermediate Preparation Example 20:

### Synthesis of (5-aminopyridine-2-yl)(4-ethylpiperazine-1-yl)ketone (B-7)

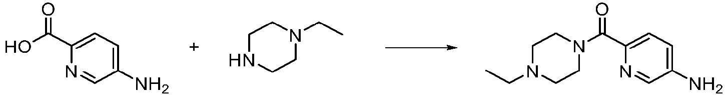

5-amino-2-pyridinecarboxylic acid (0.69 g, 5 mmol), N-ethylpiperazine (0.69g, 6 mmol), HATU (2.85 g, 7.5 mmol), N-methylmorpholine (1.26g, 12.5 mmol) were weighed and dissolved in dichloromethane (30 mL). The mixture was stirred under room temperature for 8 hours, concentrated, and was purified by flash silica column chromatography to give the compound B-7 (0.89g, yield of 76%). The yield was 82%. MS (M + H)⁺: found, 235.4.

### Intermediate Preparation Example 21:

### Synthesis of (5-aminopyridine-2-yl)(4-isopropylpiperazine-1-yl)ketone (B-8)

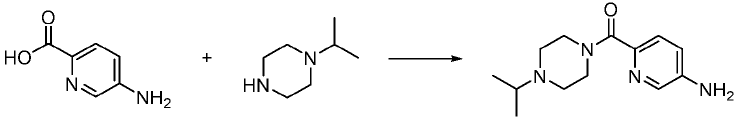

Referring to the synthesis method of Compound (B-7), the yield was 73%. MS (M + H)⁺: found, 249.4

### Intermediate Preparation Example 22:

### Synthesis of tert-butyl 4-(5-aminopyridine-2-yl)piperazine-1-carboxylate (B-9)

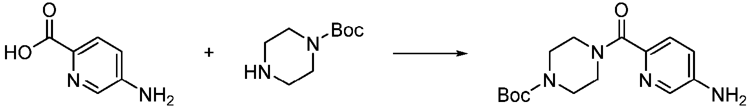

Referring to the synthesis method of Compound (B-7), the yield was 75%. MS (M + H)⁺: found, 307.4.

### Intermediate Preparation Example 23:

### Synthesis of (2-aminopyrimidine-5-yl)(4-ethylpiperazine-1-yl)ketone (B-10)

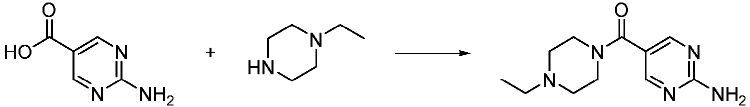

2-aminopyrimidine-5-carboxylic acid (0.60 g, 5 mmol), EDCI (2.88 g, 15 mmol), HOBt (1.01 g, 7.5 mmol), triethylamine (2.53 g, 25 mmol) were weighed and dissolved in DMF (30 mL). The mixture was stirred under room temperature for half an hour, and was added with N-ethylpiperazine (0.69 g, 6 mmol), and further stirred for 8 hours, concentrated, and was purified by flash silica column chromatography to give the compound B-10 (0.84 g, yield of 71%). ¹H NMR (300 MHz, CDCl₃) 6 8.41 (s, 2H), 5.55 (s, 2H), 3.70 - 3.58 (m, 4H), 2.48 - 2.41 (m, 6H), 1.09 (t, *J =* 7.2 Hz, 3H).

### Intermediate Preparation Example 24:

### Synthesis of tert-butyl 4-(2-aminopyrimidine-5-carbonyl)piperazine-1-carboxylate (B-11)

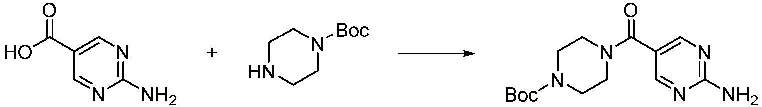

Referring to the synthesis method of Compound (B-10), the yield was 73%. MS (M + Na)⁺: found, 330.4.

### Intermediate Preparation Example 25:

### Synthesis of 5- ( (4-ethylpiperazine-1-yl)methyl)pyridine-2-amine (B-12)

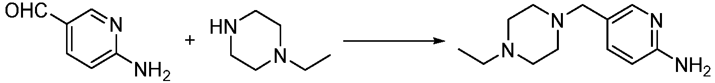

2-amino-5-formylpyridine(2.34 g, 10 mmol) and N-ethylpiperazine(1.37 g, 12 mmol) were dissolved in 1, 2-dichloroethane (50mL). The mixture was stirred under room temperature for 2 hours, and then was added with sodium triacetoxyborohydride (3.18 g, 15 mmol). The mixture was stirred under room temperature for 8 hours. The mixture was quenched by adding water, extracted by dichloromethane, dried by anhydrous sodium sulfate, concentrated, and then purified by column chromatography to give Compound B-12 (1.81g, yield of 82%). ¹H NMR (400 MHz, Chloroform-*d*) δ 7.91 (d, *J =* 2.3 Hz, 1H), 7.43 (dd, *J =* 8.4, 2.3 Hz, 1H), 6.48 (d, *J =* 8.4 Hz, 1H), 4.71 (s, 2H), 3.38 (s, 2H), 3.06 (brs, 4H), 2.52 - 2.44 (m, 6H), 1.10 (t, *J =* 7.2 Hz, 3H).

### Intermediate Preparation Example 26:

### Synthesis of 5-((4-isopropylpiperazine-1-yl)methyl)pyridine-2-amine (B-13)

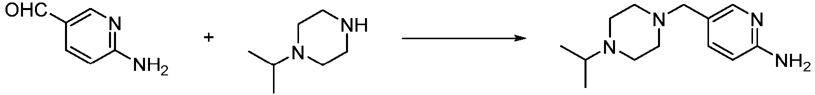

Referring to the synthesis method of Compound (B-12), the yield was 79%. MS (M + H)⁺: found, 235.2.

### Intermediate Preparation Example 27:

### Synthesis of tert-butyl 4-((6-aminopyridine-3-yl)methyl)piperazine-1-carboxylate (B-14)

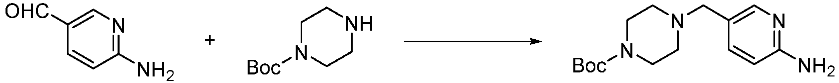

Referring to the synthesis method of Compound (B-12), the yield was 86%. MS (M + H)⁺: found, 293.3.

### Intermediate Preparation Example 28:

### Synthesis of 6-(4,4,5,5-tetramethyl-1,3,2-dioxaborolane-2-yl)benzothiazole (C-1)

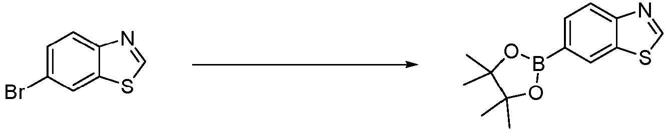

Referring to the synthesis method of Compound (A-1), the yield was 90%. ¹H NMR (400 MHz, CDCl₃) *δ* 9.05 (s, 1H), 8.45 (d, *J* = 1.1 Hz, 1H), 8.13 (d, *J* = 8.2 Hz, 1H), 7.94 (dd, *J* = 8.2, 1.1 Hz, 1H), 1.38 (s, 12H).

### Intermediate Preparation Example 29:

### Synthesis of 6-(2-chloro-5-fluoropyrimidine-4-yl)benzothiazole (C-2)

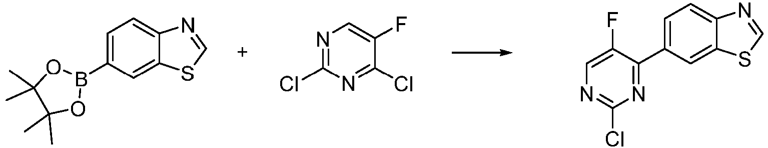

Referring to the synthesis method of Compound (A-2), the yield was 82%. ¹H NMR (400 MHz, CDCl₃) *δ* 9.17 (s, 1H), 8.85 (d, *J =* 1.7 Hz, 1H), 8.58 (d, *J =* 3.1 Hz, 1H), 8.35 - 8.26 (m, 2H).

### Intermediate Preparation Example 30:

### Synthesis of 6-(2-chloro-5-methylpyrimidine-4-yl)benzothiazole (C-3)

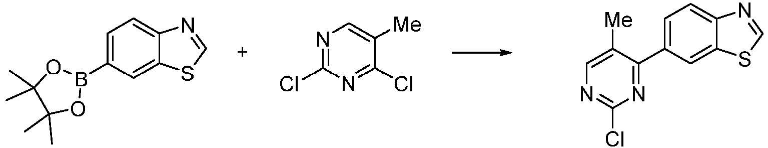

Referring to the synthesis method of Compound (A-2), the yield was 77%. ¹H NMR (400 MHz, DMSO) *δ* 9.55 (s, 1H), 8.76 (s, 1H), 8.57 (d, *J =* 1.7 Hz, 1H), 8.23 (d, *J=* 8.5 Hz, 1H), 7.87 (dd, *J =* 8.5, 1.7 Hz, 1H), 2.41 (s, 3H).

### Intermediate Preparation Example 31:

### Synthesis of 6-(2-chloro-5-methoxypyrimidine-4-yl)benzothiazole (C-4)

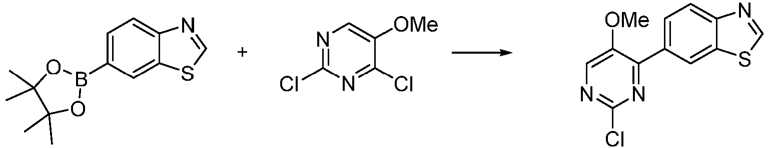

Referring to the synthesis method of Compound (A-2), the yield was 81%. ¹H NMR (400 MHz, DMSO) *δ* 9.55 (s, 1H), 8.91 - 8.90 (m, 1H), 8.73 (s, 1H), 8.25 - 8.20 (m, 2H), 4.06 (s, 3H).

### Intermediate Preparation Example 32:

### Synthesis of 6-(2-chloropyrimidine-4-yl)benzothiazole (C-5)

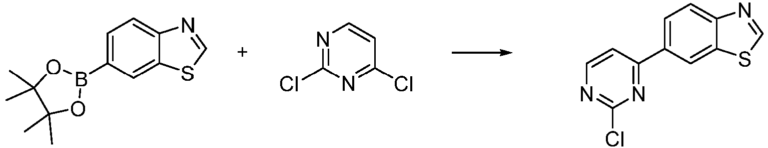

Referring to the synthesis method of Compound (A-2), the yield was 81%. ¹H NMR (400 MHz, CDCl₃) *δ* 9.14 (s, 1H), 8.85 (d, *J =* 1.7 Hz, 1H), 8.69 (d, *J =* 5.3 Hz, 1H), 8.27 - 8.17 (m, 2H), 7.75 (d, *J* = 5.3 Hz, 1H).

### Intermediate Preparation Example 33:

### Synthesis of 2-methyl-6-(4,4,5,5-tetramethyl-1,3,2-dioxaborolane-2-yl)benzothiazole (C-6)

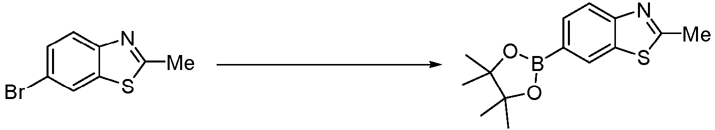

Referring to the synthesis method of Compound (A-1), the yield was 90%. ¹H NMR (400 MHz, CDCl₃) *δ* 8.31 - 8.30 (m, 1H), 7.94 - 7.85 (m, 2H), 2.84 (s, 3H), 1.36 (s, 12H).

### Intermediate Preparation Example 34:

### Synthesis of 6-(2-chloro-5-fluoropyrimidine-4-yl)-2-methylbenzothiazole (C-7)

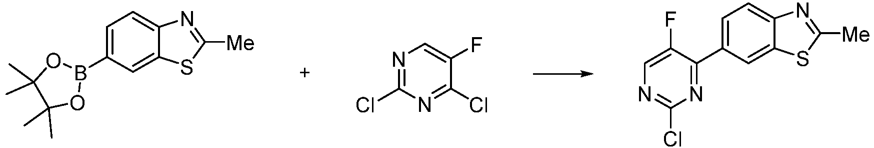

Referring to the synthesis method of Compound (A-2), the yield was 84%. ¹H NMR (300 MHz, DMSO) *δ* 9.00 (d, *J =* 3.4 Hz, 1H), 8.80 - 8.79 (m, 1H), 8.17 - 8.08 (m, 2H), 2.87 (s, 3H).

### Intermediate Preparation Example 35:

### Synthesis of 6-bromo-N -cyclopentylbenzothiazole-2-amine (C-8)

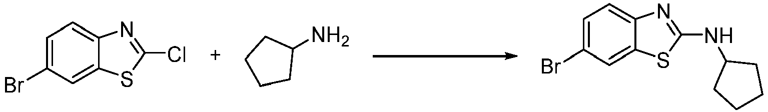

6-bromo-2-chlorobenzothiazole (0.5 g, 2 mmol), cyclopenylamino (0.19 g, 2.2 mmol), DIPEA(0.39 g, 3 mmol) was dissolved in DMSO (10 mL). The system was replaced with argon three times, and was heated to 80°C to react for 12 h. The mixture was purified by flash silica column chromatography to give the compound 6-bromo-N -cyclopentylbenzothiazole-2-amine C-8 (0.53 g, 90% yield). ¹H NMR (400 MHz, CDCl₃) *δ* 7.68 (d, *J =* 1.7 Hz, 1H), 7.38 - 7.33 (m, 2H), 6.28 (s, 1H), 3.98 - 3.93 (m, 1H), 2.14 - 2.04 (m, 2H), 1.72 - 1.54 (m, 6H).

### Intermediate Preparation Example 36:

### Synthesis of N-cyclopentyl-6-(4,4,5,5-tetramethyl-1,3,2-dioxaborolane-2-yl)benzothiazole-2-amine (C-9)

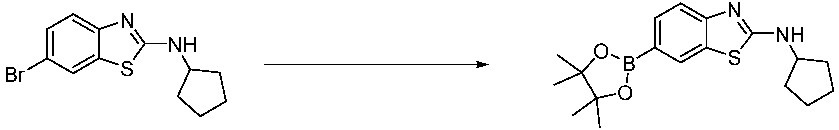

Referring to the synthesis method of Compound (A-1), the yield was 88%. ¹H NMR (400 MHz, CDCl₃) *δ* 8.05 (d, *J =* 1.2 Hz, 1H), 7.73 (dd, *J =* 8.0, 1.2 Hz, 1H), 7.49 (d, *J=* 8.0 Hz, 1H), 5.74 (s, 1H), 4.03 - 4.00 (m, 1H), 2.13 - 2.07 (m, 2H), 1.76 - 1.56 (m, 6H), 1.35 (s, 12H).

### Intermediate Preparation Example 37:

### Synthesis of 6-(2-chloro-5-fluoropyrimidine-4-yl)-2-methylbenzothiazole (C-10)

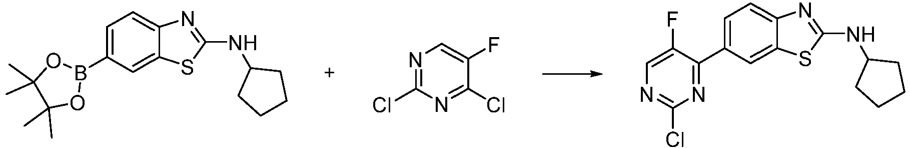

Referring to the synthesis method of Compound (A-2), the yield was 83%. ¹H NMR (400 MHz, CDCl₃) *δ* 8.49 (d, *J =* 1.9 Hz, 1H), 8.46 (d, *J =* 3.5 Hz, 1H), 8.17 - 8.14 (m, 1H), 7.58 (d, *J =* 8.6 Hz, 1H), 5.88 (s, 1H), 4.13 - 4.07 (m, 1H), 2.19 - 2.11 (m, 2H), 1.77 - 1.61 (m, 6H).

### Example 1: Synthesis of

### 4-(benzothiazole-5-yl)-N-(5-((4-isopropylpiperazine-1-yl)methyl)pyridine-2-yl)-5-methylpyri midine-2-amine (P-1)

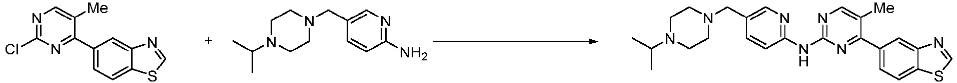

Compound A-2 (261.7mg, 1.0mmol) and B-13281.2mg, 1.2mmol) were dissolved in dioxane(10mL), and then were added with Pd₂(dba)₃(45.8mg, 0.05mmol), BINAP(62.3mg, 0.1mmol), cesium carbonate (651.6mg, 2.0mmol). The system was replaced with argon three times, and was heated to 100°C to react for 12h. The mixture was cooled, filtrated, and concentrated, and purified by column chromatography to give Compound P-1(193.0 mg, 42% yield), as a white solid. ¹H NMR (400 MHz, CDCl₃) *δ* 9.10 (s, 1H), 8.45 - 8.43 (m, 3H), 8.21 (d, *J =* 2.3 Hz, 1H), 8.12 (d, *J* = 8.4 Hz, 1H), 8.04 (s, 1H), 7.78 (dd, *J =* 8.4, 1.6 Hz, 1H), 7.61 - 7.58 (m, 1H), 3.55 (s, 2H), 3.38 - 2.89 (m, 9H), 2.37 (s, 3H), 1.44 - 1.40 (m, 6H).

### Example 2: Synthesis of 4-(benzothiazole-5-yl)-5-methyl-N-(5-(piperazine-1-methylene)piperidine-2-yl)pyrimidine-2-a mine hydrochloride (P-2)

### Step 1: Synthesis of Intermediate P-2-1

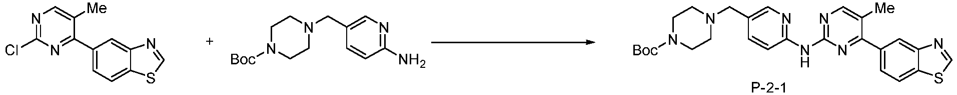

Intermediate P-2-1 was synthesized by referring to Example 1, with a yield of 45%, as a white solid. ¹H NMR (400 MHz, CDCl₃) *δ* 9.10 (s, 1H), 8.43 - 8.42 (m, 3H), 8.19 (d, *J =* 2.3 Hz, 1H), 8.11 (dd, *J =* 8.3, 0.6 Hz, 1H), 7.98 (s, 1H), 7.79 (dd, *J =* 8.3, 1.7 Hz, 1H), 7.66 - 7.64 (m, 1H), 3.46 (s, 2H), 3.43 - 3.40 (m, 4H), 2.39 - 2.37 (m, 7H), 1.45 (s, 9H). HRMS (ESI) calcd 518.2333; found, 518.2326.

### Step 2: Synthesis of Final Product P-2

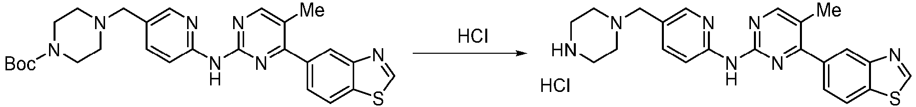

Intermediate P-2-1 (100 mg) was dissolved in dichloromethane (15 mL). The mixture was added dropwisely with 3 mL of 2 M EA-HCl solution, and stirred for 8 hours, concentrated to give Compound P-2, yield of 100%, as a light yellow solid. ¹H NMR (400 MHz, DMSO) *δ* 11.85 (s, 1H), 9.86 (s, 2H), 9.54 (s, 1H), 8.73 (d, *J =* 0.8 Hz, 1H), 8.64 (d, *J =* 2.2 Hz, 1H), 8.47 (d, *J =* 1.6 Hz, 1H), 8.40 (dd, *J =* 10.3, 7.6 Hz, 2H), 7.91 (d, *J =* 8.9 Hz, 1H), 7.88 - 7.85 (m, 1H), 4.47 (s, 2H), 3.50 - 3.34 (m, 8H), 2.43 (s, 3H). HRMS (ESI) calcd 418.1808; found, 418.1807.

### Example 3: Synthesis of (6-((4-(benzothiazole-5-yl)-5-methylpyrimidine-2-yl)amino)pyridine-3-yl)(4-ethylpiperazine-1 -yl)ketone (P-3)

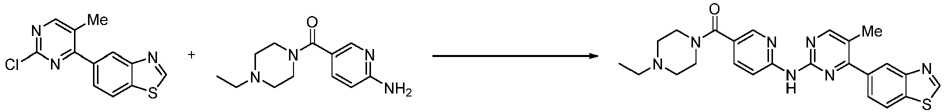

Referring to the synthesis method of Example 1, the yield was 37%, as a white solid. ¹H NMR (400 MHz, CDCl₃) *δ* 9.19 (s, 1H), 9.10 (s, 1H), 8.56 - 8.53 (m, 3H), 8.43 (d, *J =* 1.6 Hz, 1H), 8.12 (d, *J =* 8.3 Hz, 1H), 7.80 - 7.75 (m, 2H), 3.77 - 3.60 (m, 4H), 2.48 - 2.42 (m, 6H), 2.36 (s, 3H), 1.10 (t, *J =* 7.2 Hz, 3H). HRMS (ESI): calcd 460.1914; found, 460.1904.

### Example 4: Synthesis of tert-butyl 4-(6-((4-(benzothiazole-5-yl)-5-methylpyrimidine-2-yl)amino)nicotinoyl)piperazine -1-carboxylate (P-4)

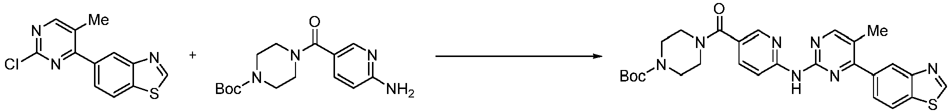

Referring to the synthesis method of Example 1, the yield was 38%, as a white solid. ¹H NMR (400 MHz, CDCl₃) *δ* 9.10 (s, 1H), 8.85 (s, 1H), 8.56 (dd, *J* = 8.8, 0.8 Hz, 1H), 8.51 - 8.50 (m, 1H), 8.48 (dd, *J* = 2.4, 0.8 Hz, 1H), 8.43 (d, *J* = 1.6 Hz, 1H), 8.12 (d, *J* = 8.4 Hz, 1H), 7.80 - 7.74 (m, 2H), 3.66 - 3.44 (m, 8H), 2.38 (s, 3H), 1.48 (s, 9H). HRMS (ESI) calcd 532.2125; found, 532.2124.

### Example 5: Synthesis of (6-((4-(benzothiazole-5-yl)-5-methylpyrimidine-2-yl)amino)pyridine-3-yl)(piperazine-1-yl)ket one hydrochloride (P-5)

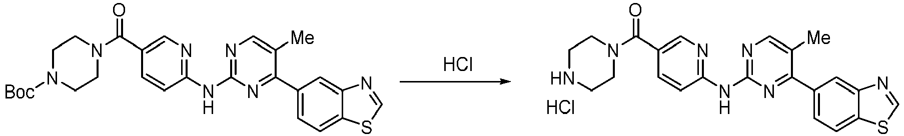

P-4 (100 mg) was dissolved in dichloromethane (15 mL). The mixture was added dropwisely with 3 mL of 2 M EA-HCl solution, and stirred for 8 hours, concentrated to give Compound P-5, yield of 100%, as a light yellow solid. ¹H NMR (400 MHz, DMSO) *δ* 12.03 (s, 1H), 9.80 (s, 2H), 9.55 (s, 1H), 8.75 (s, 1H), 8.59 (d, *J =* 2.0 Hz, 1H), 8.47 (d, *J =* 1.5 Hz, 1H), 8.39 (d, *J =* 8.3 Hz, 1H), 8.24 (dd, *J =* 8.9, 2.0 Hz, 1H), 7.93 (d, *J =* 8.9 Hz, 1H), 7.88 (dd, *J =* 8.3, 1.6 Hz, 1H), 3.83 - 3.74 (m, 4H), 3.18 - 3.16 (m, 4H), 2.43 (s, 3H). HRMS (ESI) calcd 432.1601; found, 432.1595.

### Example 6: Synthesis of (6-((4-(benzothiazole-5-yl)-5-methoxypyrimidine-2-yl)amino)pyridine-3-yl)(piperazine-1-yl)k etone hydrochloride (P-6)

### Step 1: Synthesis of Intermediate P-6-1

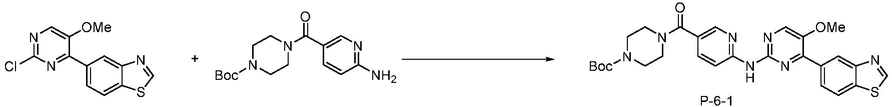

Intermediate P-6-1 was synthesized by referring to Example 1, with a yield of 41%, as a white solid. ¹H NMR (400 MHz, CDCl₃) *δ* 9.08 (s, 1H), 8.96 (dd, *J =* 1.7, 0.6 Hz, 1H), 8.52 - 8.50 (m, 1H), 8.40 (dd, *J* = 2.3, 0.8 Hz, 1H), 8.37 (s, 1H), 8.25 (dd, *J* = 8.5, 1.7 Hz, 1H), 8.17 (s, 1H), 8.09 (dd, *J* = 8.5, 0.6 Hz, 1H), 7.80 (dd, *J =* 8.7, 2.4 Hz, 1H), 3.98 (s, 3H), 3.66 - 3.47 (m, 8H), 1.48 (s, 9H). HRMS (ESI) calcd 548.2074; found, 548.2072.

### Step 2: Synthesis of Final Product P-6

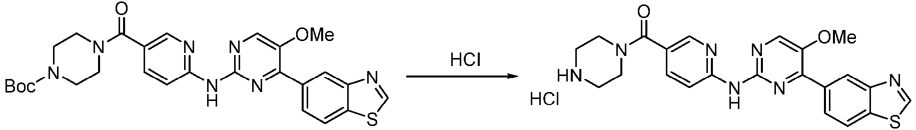

Intermediate P-6-1 (100 mg) was dissolved in dichloromethane (15 mL). The mixture was added dropwisely with 3 mL of 2 M EA-HCl solution, and stirred for 8 hours, concentrated to give Compound P-6, yield of 100%, as a light yellow solid. ¹H NMR (400 MHz, DMSO) *δ* 11.93 (s, 1H), 9.75 (s, 2H), 9.53 (s, 1H), 8.83 (d, *J =* 1.5 Hz, 1H), 8.66 (s, 1H), 8.56 - 8.55 (m, 1H), 8.37 (d, *J =* 8.5 Hz, 1H), 8.23 (dd, *J =* 8.3, 1.7 Hz, 2H), 7.94 (d, *J =* 8.9 Hz, 1H), 4.07 (s, 3H), 3.82 - 3.76 (m, 4H), 3.20 - 3.16 (m, 4H). HRMS (ESI) calcd 448.1550; found, 448.1554.

### Example 7: Synthesis of 4-(benzothiazole-5-yl)-5-methoxy-N-(5-(piperazine-1-ylmethyl)pyridine-2-yl)pyrimidine-2-am ine hydrochloride (P-7)

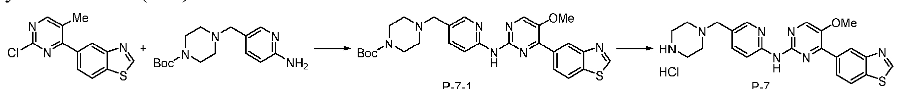

Step 1: Intermediate P-7-1 was synthesized by referring to Example 1, with a yield of 45%, as a white solid. ¹H NMR (400 MHz, CDCl₃) *δ* 9.07 (s, 1H), 8.97 (dd, *J =* 1.6, 0.6 Hz, 1H), 8.41 (d, *J* = 8.5 Hz, 1H), 8.34 (s, 1H), 8.26 (dd, *J =* 8.5, 1.6 Hz, 1H), 8.20 - 8.19 (m, 1H), 8.08 (dd, *J =* 8.5, 0.6 Hz, 1H), 7.93 (s, 1H), 7.68 (d, *J =* 8.6 Hz, 1H), 3.96 (s, 3H), 3.48 (s, 2H), 3.45 - 3.41 (m, 4H), 2.42 - 2.38 (m, 4H), 1.45 (s, 9H). HRMS (ESI) calcd 534.2282; found, 534.2277.

Step 2: Intermediate P-7-1 (100 mg) was dissolved in dichloromethane (15 mL). The mixture was added dropwisely with 3 mL of 2 M EA-HCl solution, and stirred for 8 hours, concentrated to give Compound P-7, yield of 100%, as a light yellow solid. ¹H NMR (400 MHz, DMSO) *δ* 12.26 (s, 1H), 10.13 (s, 2H), 9.53 (s, 1H), 8.83 (d, *J =* 1.6 Hz, 1H), 8.70 (d, *J =* 2.1 Hz, 1H), 8.66 (s, 1H), 8.54 (dd, *J* = 9.1, 2.1 Hz, 1H), 8.37 (d, *J* = 8.5 Hz, 1H), 8.23 (dd, *J* = 8.5, 1.7 Hz, 1H), 7.85 (d, *J* = 9.1 Hz, 1H), 4.57 (s, 2H), 4.08 (s, 3H), 3.55 - 3.44 (m,8H). HRMS (ESI) for C₂₄H₂₄FN₇O₂S (M + H)⁺: calcd 434.1758; found, 434.1750.

### Example 8: Synthesis of 4-(benzothiazole-5-yl)-N-(5-(piperazine-1-methylene)pyridine-2-yl)pyrimidine-2-amine hydrochloride (P-8)

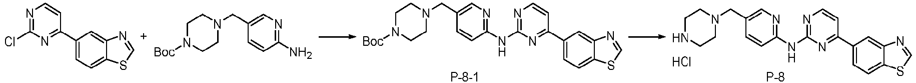

Step 1: Intermediate P-8-1 was synthesized by referring to Example 1, with a yield of 47%, as a white solid. ¹H NMR (400 MHz, CDCl₃) *δ* 9.10 (s, 1H), 8.85 (d, *J =* 1.7 Hz, 1H), 8.60 (d, *J =* 5.2 Hz, 1H), 8.53 (d, *J =* 8.5 Hz, 1H), 8.25 - 8.21 (m, 2H), 8.15 (s, 1H), 8.11 (d, *J =* 8.4 Hz, 1H), 7.75 (dd, *J =* 9.0, 2.2 Hz, 1H), 7.35 (d, *J =* 5.2 Hz, 1H), 3.50 (s, 2H), 3.46 - 3.42 (m, 4H), 2.43 - 2.39 (m, 4H), 1.46 (s, 9H). HRMS (ESI) calcd 504.2176; found, 504.2159.

Step 2: Intermediate P-8-1 (100 mg) was dissolved in dichloromethane (15 mL). The mixture was added dropwisely with 3 mL of 2 M EA-HCl solution, and stirred for 8 hours, concentrated to give Compound P-8, yield of 100%, as a light yellow solid. ¹H NMR (400 MHz, DMSO) *δ* 11.79 (s, 1H), 9.88 (s, 2H), 9.55 (s, 1H), 8.99 (d, *J =* 1.6 Hz, 1H), 8.82 (d, *J =* 5.5 Hz, 1H), 8.67 (d, *J* = 2.2 Hz, 1H), 8.44 - 8.36 (m, 3H), 8.04 - 8.01 (m, 2H), 4.50 (s, 2H), 3.51 - 3.43 (m, 8H). HRMS (ESI) calcd 404.1652; found, 404.1650.

### Example 9: Synthesis of (6-((4-(benzothiazole-5-yl)pyrimidine-2-yl)amino)pyridine-3-yl)(4-ethylpiperazine-1-yl)keton e (P-9)

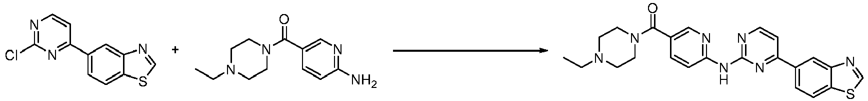

Referring to the synthesis method of Example 1, the yield was 36%, as a white solid. ¹H NMR (400 MHz, CDCl₃) *δ* 9.10 (s, 1H), 8.84 (d, *J =* 1.7 Hz, 1H), 8.63 (s, 1H), 8.61 (d, *J =* 2.7 Hz, 1H), 8.45 - 8.44 (m, 1H), 8.22 (dd, *J =* 8.5, 1.7 Hz, 2H), 8.12 (d, *J* = 8.5 Hz, 1H), 7.86 (dd, *J* = 8.8, 2.4 Hz, 1H), 7.41 (d, *J =* 5.3 Hz, 1H), 3.86 - 3.62 (m, 4H), 2.56 - 2.45 (m, 6H), 1.16 - 1.11 (m, 3H). HRMS (ESI) calcd 446.1758; found, 446.1751.

### Example 10: Synthesis of tert-butyl 4-(6-((4-(benzothiazole-5-yl)pyrimidine-2-yl)amino)nicotinoyl)piperazine-1-carboxylate (P-10)

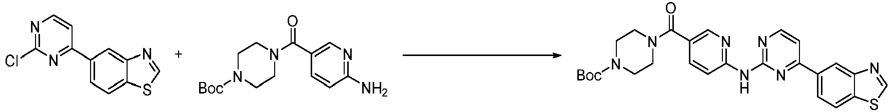

Referring to the synthesis method of Example 1, the yield was 39%, as a white solid. ¹H NMR (300 MHz, CDCl₃) *δ* 9.11 (s, 1H), 8.84 (d, *J =* 1.6 Hz, 1H), 8.66 - 8.63 (m, 2H), 8.45 (dd, *J* = 2.4, 0.8 Hz, 1H), 8.34 (s, 1H), 8.23 (dd, *J* = 8.4, 1.7 Hz, 1H), 8.12 (d, *J* = 8.4 Hz, 1H), 7.86 (dd, *J* = 8.7, 2.3 Hz, 1H), 7.42 (d, *J* = 5.2 Hz, 1H), 3.71 - 3.48 (m, 8H), 1.48 (s, 9H). HRMS (ESI) calcd 518.1969; found, 518.1970.

### Example 11: Synthesis of (6-((4-(benzothiazole-5-yl)pyrimidine-2-yl)amino)pyridine-3-yl)(piperazine-1-yl)ketone hydrochloride (P-11)

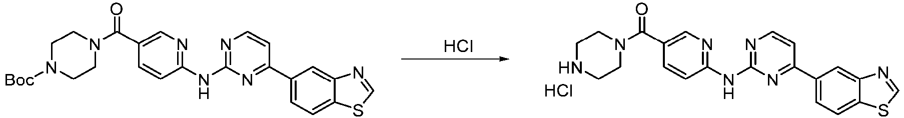

Intermediate P-10 (100 mg) was dissolved in dichloromethane (15 mL). The mixture was added dropwisely with 3 mL of 2 M EA-HCl solution, and stirred for 8 hours, concentrated to give Compound P-11, yield of 100%, as a light yellow solid. ¹H NMR (400 MHz, DMSO) *δ* 11.59 (s, 1H), 9.69 (s, 2H), 9.55 (s, 1H), 8.99 (d, *J =* 1.5 Hz, 1H), 8.81 (d, *J =* 5.4 Hz, 1H), 8.56 (d, *J =* 2.0 Hz, 1H), 8.43 - 8.37 (m, 2H), 8.20 - 8.11 (m, 2H), 7.99 (d, *J =* 5.3 Hz, 1H), 3.82 - 3.77 (m, 4H), 3.21 - 3.16 (m, 4H). HRMS (ESI) calcd 418.1445; found, 418.1440.

### Example 12: Synthesis of 4-(benzothiazole-5-yl)-N-(5-((4-ethylpiperazine-1-yl)methyl)pyridine-2-yl)-5-fluoropyridine-2 -amine (P-12)

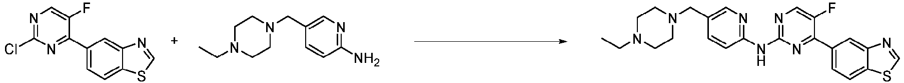

Referring to the synthesis method of Example 1, the yield was 41%, as a white solid. ¹H NMR (300 MHz, CDCl₃) *δ* 9.10 (s, 1H), 8.93 (s, 1H), 8.54 (s, 1H), 8.51 (d, *J* = 3.4 Hz, 1H), 8.41 (d, *J* = 8.6 Hz, 1H), 8.29 - 8.24 (m, 2H), 8.12 (d, *J* = 8.5 Hz, 1H), 7.73 (dd, *J =* 8.7, 2.2 Hz, 1H), 3.50 (s, 2H), 2.52 - 2.39 (m, 10H), 1.09 (t, *J =* 7.2 Hz, 3H). HRMS (ESI) calcd 450.1871; found, 450.1869.

### Example 13: Synthesis of 4-(benzothiazole-5-yl)-5-fluoro-N-(5-(piperazine-1-methylene)pyridine-2-yl)pyrimidine-2-ami ne hydrochloride (P-13)

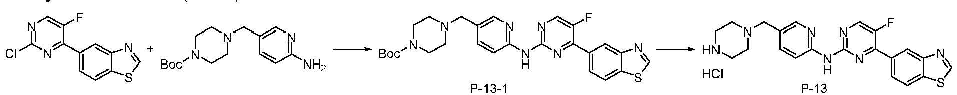

Step 1: Intermediate P-13-1 was synthesized by referring to Example 1, with a yield of 41%, as a white solid. ¹H NMR (400 MHz, CDCl₃) *δ* 9.11 (s, 1H), 8.93 (dd, *J =* 1.7, 0.8 Hz, 1H), 8.63 (s, 1H), 8.51 (d, *J =* 3.4 Hz, 1H), 8.43 (dd, *J =* 8.6, 0.8 Hz, 1H), 8.29 (dd, *J =* 2.3, 0.8 Hz, 1H), 8.26 - 8.23 (m, 1H), 8.12 (dd, *J =* 8.5, 0.6 Hz, 1H), 7.74 (dd, *J =* 8.6, 2.3 Hz, 1H), 3.49 (s, 2H), 3.45 - 3.43 (m, 4H), 2.42 - 2.40 (m, 4H), 1.46 (s, 9H). HRMS (ESI) calcd 522.2082; found, 522.2088.

Step 2: Intermediate P-13-1 (100 mg) was dissolved in dichloromethane (15 mL). The mixture was added dropwisely with 3 mL of 2 M EA-HCl solution, and stirred for 8 hours, concentrated to give Compound P-13, yield of 100%, as a light yellow solid. ¹H NMR (400 MHz, DMSO) *δ* 11.72 (s, 1H), 9.94 (s, 2H), 9.57 - 9.56 (m, 1H), 8.92 - 8.90 (m, 1H), 8.79 (s, 1H), 8.66 (s, 1H), 8.44 - 8.40 (m, 2H), 8.22 - 8.19 (m, 1H), 8.07 (dd, *J =* 8.9, 2.7 Hz, 1H), 4.51 (s, 2H), 3.53 - 3.44 (m, 8H). HRMS (ESI) calcd 422.1558; found, 422.1558.

### Example 14: Synthesis of (6-((4-(benzothiazole-5-yl)-5-fluoropyrimidine-2-yl)amino)pyridine-3-yl)(4-ethylpiperazine-1-yl)ketone (P-14)

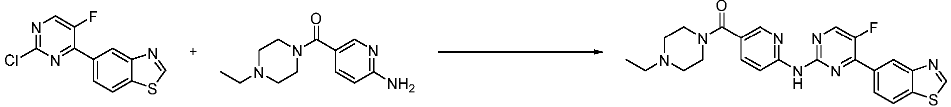

Referring to the synthesis method of Example 1, the yield was 33%, as a white solid. ¹H NMR (300 MHz, CDCl₃) *δ* 9.12 (s, 1H), 8.93 - 8.92 (m, 1H), 8.67 (s, 1H), 8.54 - 8.48 (m, 3H), 8.26 - 8.22 (m, 1H), 8.14 (d, *J =* 8.5 Hz, 1H), 7.84 (dd, *J= 8.7,* 2.4 Hz, 1H), 3.79 - 3.63 (m, 4H), 2.51 - 2.44 (m, 6H), 1.12 (t, *J =* 7.2 Hz, 3H). HRMS (ESI) calcd 464.1663; found, 464.1662.

### Example 15: Synthesis of 1-(4-(6-((4-(benzothiazole-5-yl)-5-fluoropyrimidine-2-yl)amino)nicotinoyl)piperazine-1-yl)eth yl-1-one (P-15)

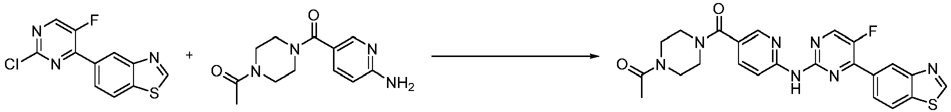

Referring to the synthesis method of Example 1, the yield was 38%, as a white solid. ¹H NMR (300 MHz, CDCl₃) *δ* 9.12 (s, 1H), 9.07 (s, 1H), 8.92 (s, 1H), 8.57 - 8.54 (m, 3H), 8.26 - 8.13 (m, 2H), 7.86 (dd, *J =* 8.6, 2.4 Hz, 1H), 3.72 - 3.50 (m, 8H), 2.15 (s, 3H). HRMS (ESI) calcd 478.1456; found, 478.1452.

### Example 16: Synthesis of (4-(6-((4-(benzothiazole-5-yl)-5-fluoropyrimidine-2-yl)amino)nicotinoyl)piperazine-1-yl)(cycl opropyl)ketone (P-16)

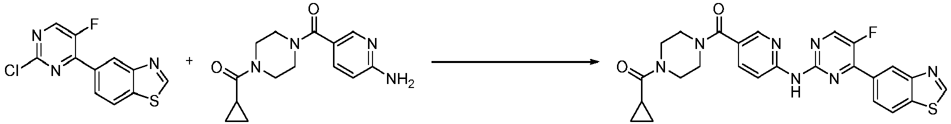

Referring to the synthesis method of Example 1, the yield was 41%, as a white solid. ¹H NMR (300 MHz, CDCl₃) *δ* 9.12 (s, 1H), 8.93 - 8.92 (m, 1H), 8.64 (s, 1H), 8.56 - 8.53 (m, 2H), 8.50 (dd, *J =* 2.4, 0.8 Hz, 1H), 8.26 - 8.22 (m, 1H), 8.16 - 8.13 (m, 1H), 7.86 (dd, *J =* 8.7, 2.3 Hz, 1H), 3.79 - 3.65 (m, 8H), 1.78 - 1.72 (m, 1H), 1.06 - 1.01 (m, 2H), 0.85 - 0.79 (m, 2H). HRMS (ESI) calcd 504.1612; found, 504.1603.

### Example 17: Synthesis of (6-((4-(benzothiazole-5-yl)-5-fluoropyrimidine-2-yl)amino)pyridine-3-yl)(4-phenylpiperazine-1-yl)ketone (P-17)

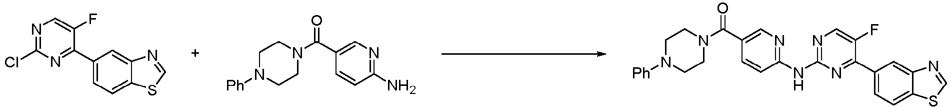

Referring to the synthesis method of Example 1, the yield was 39%, as a white solid. ¹H NMR (400 MHz, CDCl₃) *δ* 9.11 (s, 1H), 8.93 (d, *J =* 1.5 Hz, 1H), 8.54 - 8.51 (m, 2H), 8.48 (dd, *J =* 2.4, 0.8 Hz, 1H), 8.35 (s, 1H), 8.26 - 8.23 (m, 1H), 8.14 (d, *J =* 8.5 Hz, 1H), 7.87 (dd, *J =* 8.6, 2.4 Hz, 1H), 7.32 - 7.26 (m, 2H), 6.96 - 6.91 (m, 3H), 3.88 - 3.79 (m, 4H), 3.23 (brs, 4H). HRMS (ESI) calcd 512.1663; found, 512.1663.

### Example 18: Synthesis of ethyl 4-(6-((4-(benzothiazole-5-yl)-5-fluoropyrimidine-2-yl)amino)nicotinoyl) piperazine-1-carboxylate (P-18)

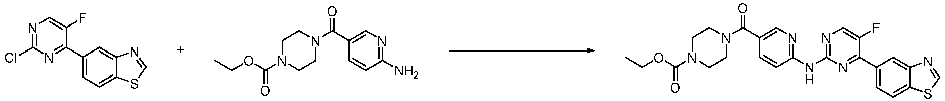

Referring to the synthesis method of Example 1, the yield was 37%, as a white solid. ¹H NMR (300 MHz, CDCl₃) *δ* 9.12 (s, 1H), 8.93 - 8.92 (m, 1H), 8.67 (s, 1H), 8.55 - 8.52 (m, 2H), 8.49 (dd, *J =* 2.4, 0.8 Hz, 1H), 8.26 - 8.22 (m, 1H), 8.14 (dd, *J =* 8.7, 0.8 Hz, 1H), 7.85 (dd, *J =* 8.7, 2.4 Hz, 1H), 4.18 (q, *J* = 7.1 Hz, 2H), 3.70 - 3.51 (m, 8H), 1.29 (t, *J* = 7.1 Hz, 3H). HRMS (ESI) calcd 508.1562; found, 508.1556.

### Example 19: Synthesis of tert-butyl 4-(6-((4-(benzothiazole-5-yl)-5-fluoropyrimidine-2-yl)amino)nicotinoyl)piperazine-1-carboxylate (P-19)

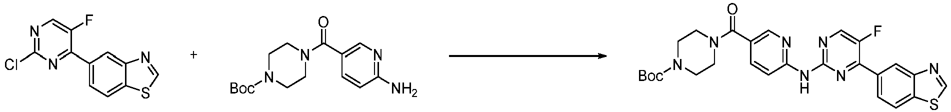

Referring to the synthesis method of Example 1, the yield was 41%, as a white solid. ¹H NMR (300 MHz, CDCl₃) *δ* 9.33 (s, 1H), 9.11 (d, *J =* 2.0 Hz, 1H), 8.92 (s, 1H), 8.58 - 8.53 (m, 3H), 8.24 (d, *J* = 8.6 Hz, 1H), 8.13 (dd, *J* = 8.6, 2.0 Hz, 1H), 7.86 (d, *J* = 8.7 Hz, 1H), 3.67 - 3.60 (m, 4H), 3.51 - 3.47 (m, 4H), 1.48 (s, 9H). HRMS (ESI) calcd 536.1875; found, 536.1871.

### Example 20: Synthesis of (6-((4-(benzothiazole-5-yl)-5-fluoropyrimidine-2-yl)amino)pyridine-3-yl)(piperazine-1-yl)keto ne hydrochloride (P-20)

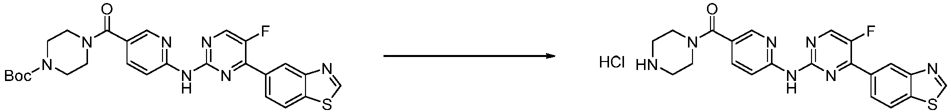

P-19 (100 mg) was dissolved in dichloromethane (15 mL). The mixture was added dropwisely with 3 mL of 2 M EA-HCl solution, and stirred for 8 hours, concentrated to give Compound P-20, yield of 100%, as a white solid. ¹H NMR (400 MHz, DMSO) *δ* 11.24 (s, 1H), 9.58 - 9.53 (m, 3H), 8.88 (d, *J =* 3.3 Hz, 1H), 8.78 (s, 1H), 8.51 (d, *J =* 2.2 Hz, 1H), 8.43 (d, *J =* 8.5 Hz, 1H), 8.21 - 8.17 (m, 2H), 8.11 - 8.08 (m, 1H), 3.79 - 3.76 (m, 3H), 3.19 - 3.15 (m, 3H). HRMS (ESI) calcd 436.1350; found, 436.1352.

### Example 21: Synthesis of (2-((4-(benzothiazole-5-yl)-5-fluoropyrimidine-2-yl)amino)pyrimidine-5-yl)(4-ethylpiperazine -1-yl)ketone (P-21)

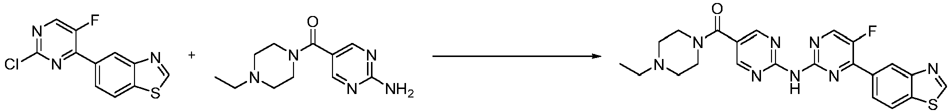

Referring to the synthesis method of Example 1, the yield was 37%, as a white solid. ¹H NMR (400 MHz, CDCl₃) *δ* 9.11 (s, 1H), 8.97 - 8.96 (m, 2H), 8.78 (s, 2H), 8.67 (d, *J* = 3.3 Hz, 1H), 8.32 - 8.29 (m, 1H), 8.13 (d, *J* = 8.5 Hz, 1H), 3.88 - 3.55 (m, 4H), 2.51 - 2.45 (m, 6H), 1.12 (t, *J* = 7.2 Hz, 3H). HRMS (ESI) calcd 465.1616; found, 465.1614.

### Example 22: Synthesis of tert-butyl 4-(2-((4-(benzothiazole-5-yl)-5-fluoropyrimidine-2-yl)amino)pyrimidine-5-carbonyl)piperazine-1-c arboxylate (P-22)

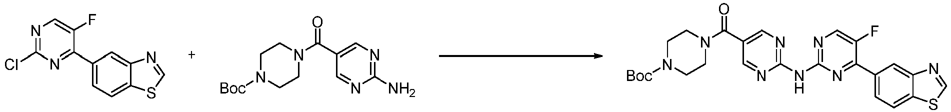

Referring to the synthesis method of Example 1, the yield was 45%. ¹H NMR (300 MHz, CDCl₃) *δ* 9.11 (s, 1H), 8.98 - 8.97 (m, 1H), 8.76 (s, 2H), 8.67 (d, *J* = 3.4 Hz, 1H), 8.57 (s, 1H), 8.31 (d, *J =* 8.7 Hz, 1H), 8.13 (dd, *J=* 8.5, 0.6 Hz, 1H), 3.72 - 3.47 (m, 8H), 1.49 (s, 9H). HRMS (ESI) calcd 537.1827; found, 537.1820.

### Example 23: Synthesis of (2-((4-(benzothiazole-5-yl)-5-fluoropyrimidine-2-yl)amino)pyrimidine-5-yl)(piperazine-1-yl)k etone hydrochloride (P-23)

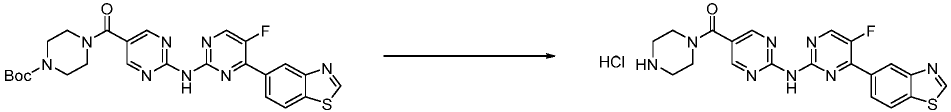

P-22 (100 mg) was dissolved in dichloromethane (15 mL). The mixture was added dropwisely with 3 mL of 2 M EA-HCl solution, and stirred for 8 hours, concentrated to give Compound P-23, yield of 100%, as a white solid. ¹H NMR (400 MHz, DMSO) *δ* 10.98 (s, 1H), 9.56 (d, *J =* 16.5 Hz, 3H), 8.85 (d, *J =* 3.3 Hz, 1H), 8.80 (d, *J =* 1.5 Hz, 1H), 8.75 (s, 2H), 8.39 (d, *J =* 8.5 Hz, 1H), 8.24 (dd, *J =* 8.4, 1.6 Hz, 1H), 3.83 - 3.78 (m, 4H), 3.20 - 3.16 (m, 4H). HRMS (ESI) calcd 437.1303; found, 437.1302.

### Example 24: Synthesis of (5-((4-(benzothiazole-5-yl)-5-fluoropyrimidine-2-yl)amino)pyridine-2-yl)(4-ethylpiperazine-1-yl)ketone (P-24)

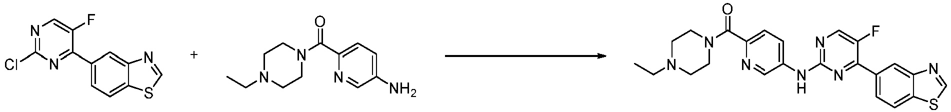

Referring to the synthesis method of Example 1, the yield was 37%, as a white solid. ¹H NMR (400 MHz, CDCl₃) *δ* 9.10 (s, 1H), 8.90 - 8.89 (m, 1H), 8.78 (dd, *J* = 2.7, 0.7 Hz, 1H), 8.45 (d, *J* = 3.3 Hz, 1H), 8.34 (dd, *J =* 8.6, 2.7 Hz, 1H), 8.24 - 8.21 (m, 1H), 8.12 (dd, *J* = 8.5, 0.6 Hz, 1H), 7.74 (d, *J =* 8.5 Hz, 1H), 7.55 (s, 1H), 3.87 - 3.76 (m, 4H), 2.58 - 2.46 (m, 6H), 1.11 (t, *J =* 7.2 Hz, 3H). HRMS (ESI) calcd 464.1663; found, 464.1654.

### Example 25: Synthesis of tert-butyl 4-(5-((4-(benzothiazole-5-yl)-5-fluoropyrimidine-2-yl)amino)pyridine-2-yl)piperazine-1-carboxylat e (P-25)

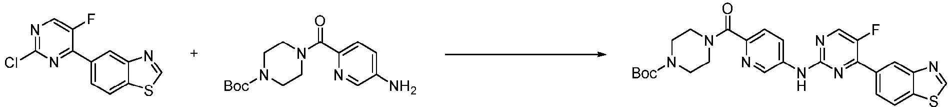

Referring to the synthesis method of Example 1, the yield was 39%, as a white solid. ¹H NMR (400 MHz, CDCl₃) *δ* 9.11 (s, 1H), 8.91 (d, *J =* 1.5 Hz, 1H), 8.78 (d, *J =* 2.6 Hz, 1H), 8.46 (d, *J =* 3.3 Hz, 1H), 8.35 (dd, *J =* 8.6, 2.6 Hz, 1H), 8.24 - 8.22 (m, 1H), 8.13 (d, *J* = 8.5 Hz, 1H), 7.79 (d, *J =* 8.6 Hz, 1H), 7.44 (s, 1H), 3.79 - 3.75 (m, 4H), 3.58 - 3.48 (m, 4H), 1.48 (s, 9H).

### Example 26: Synthesis of (5-((4-(benzothiazole-5-yl)-5-fluoropyrimidine-2-yl)amino)pyridine-2-yl)(piperazine-1-yl)keto ne hydrochloride (P-26):

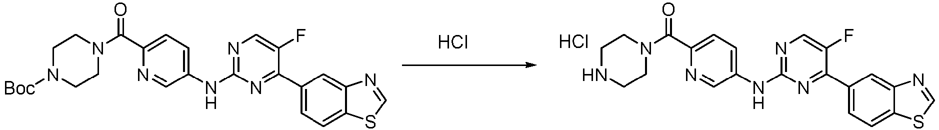

P-25 (100 mg) was dissolved in dichloromethane (15 mL). The mixture was added dropwisely with 3 mL of 2 M EA-HCl solution, and stirred for 8 hours, concentrated to give Compound P-26, yield of 100%, as a white solid. ¹H NMR (400 MHz, DMSO) *δ* 10.44 (s, 1H), 9.55 (s, 1H), 9.48 (d, *J =* 6.5 Hz, 2H), 9.02 (d, *J* = 2.5 Hz, 1H), 8.80 (d, *J* = 3.5 Hz, 1H), 8.74 (t, *J* = 1.3 Hz, 1H), 8.42 (dd, *J =* 8.6, 2.3 Hz, 2H), 8.19 - 8.16 (m, 1H), 7.76 (d, *J =* 8.6 Hz, 1H), 3.91 - 3.88 (m, 4H), 3.19 - 3.14 (m, 4H). HRMS (ESI) calcd 436.1350; found, 436.1350.

### Example 27: Synthesis of (6-((4-(2-(dimethylamino)benzothiazole-5-yl)-5-fluoropyrimidine-2-yl)amino)pyridine-3-yl)(p iperazine-1-yl)ketone hydrochloride (P-27)

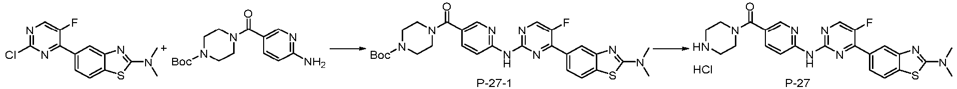

Step 1: Intermediate P-27-1 was synthesized by referring to Example 1, with a yield of 36%, as a white solid. ¹H NMR (300 MHz, CDCl₃) *δ* 8.55 - 8.52 (m, 2H), 8.47 - 8.45 (m, 2H), 8.33 - 8.32 (m, 1H), 7.87 - 7.81 (m, 2H), 7.76 - 7.73 (m, 1H), 3.66 - 3.47 (m, 8H), 3.26 (s, 6H), 1.48 (s, 9H). HRMS (ESI) calcd 579.2297; found, 579.2291.

Step 2: Intermediate P-27-1 (100 mg) was dissolved in dichloromethane (15 mL). The mixture was added dropwisely with 3 mL of 2 M EA-HCl solution, and stirred for 8 hours, concentrated to give Compound P-27, yield of 100%, as a light yellow solid. ¹H NMR (400 MHz, DMSO) *δ* 11.22 (s, 1H), 9.54 (s, 2H), 8.84 (d, *J =* 3.5 Hz, 1H), 8.51 - 8.50 (m, 1H), 8.25 (s, 1H), 8.20 - 8.12 (m, 2H), 8.06 (d, *J =* 8.3 Hz, 1H), 7.88 - 7.85 (m, 1H), 3.80 - 3.76 (m, 4H), 3.27 (s, 6H), 3.19 - 3.16 (m, 4H). HRMS (ESI) calcd 479.1772; found, 479.1774.

### Example 28: Synthesis of (6-((4-(2-(diethylamino)benzothiazole-5-yl)-5-fluoropyrimidine-2-yl)amino)pyridine-3-yl)(pip erazine-1-yl)ketone hydrochloride (P-28)

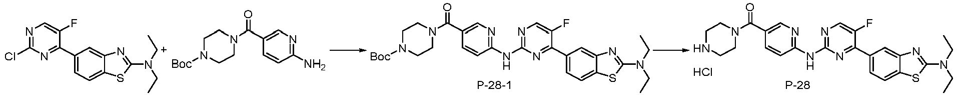

Step 1: Intermediate P-28-1 was synthesized by referring to Example 1, with a yield of 39%, as a white solid. ¹H NMR (400 MHz, CDCl₃) *δ* 9.39 (s, 1H), 8.59 - 8.56 (m, 2H), 8.52 (d, *J =* 3.5 Hz, 1H), 8.29 (t, *J =* 1.4 Hz, 1H), 7.86 - 7.80 (m, 2H), 7.72 (d, *J =* 8.2 Hz, 1H), 3.67 - 3.58 (m, 8H), 3.50 - 3.47 (m, 4H), 1.48 (s, 9H), 1.32 (t, *J =* 7.1 Hz, 6H). HRMS (ESI) calcd 607.2610; found, 607.2600.

Step 2: Intermediate P-28-1 (100 mg) was dissolved in dichloromethane (15 mL). The mixture was added dropwisely with 3 mL of 2 M EA-HCl solution, and stirred for 8 hours, concentrated to give Compound P-28, yield of 100%, as a light yellow solid. ¹H NMR (400 MHz, DMSO) *δ* 11.35 (s, 1H), 9.64 (s, 2H), 8.85 (d, *J =* 3.3 Hz, 1H), 8.53 (s, 1H), 8.28 (s, 1H), 8.18 - 8.04 (m, 3H), 7.87 (d, *J =* 8.3 Hz, 1H), 3.82 - 3.70 (m, 4H), 3.68 - 3.64 (m, 4H), 3.19 - 3.15 (m, 4H), 1.27 (t, *J =* 7.1 Hz, 6H). HRMS (ESI) calcd 507.2085; found, 507.2089.

### Example 29: Synthesis of (5-((4-(2-(dimethylamino)benzothiazole-5-yl)-5-fluoropyrimidine-2-yl)amino)pyridine-2-yl)(4 -ethylpiperazine-1-yl)ketone (P-29)

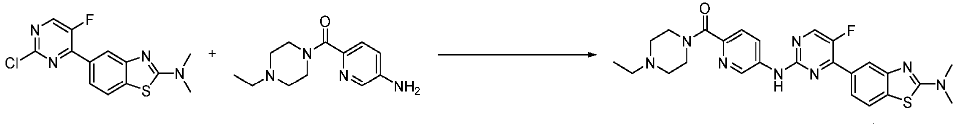

Referring to the synthesis method of Example 1, the yield was 33%, as a white solid. ¹H NMR (400 MHz, CDCl₃) *δ* 8.75 (d, *J =* 2.6 Hz, 1H), 8.39 - 8.34 (m, 2H), 8.31 (t, *J=* 1.4 Hz, 1H), 7.86 - 7.83 (m, 1H), 7.76 - 7.73 (m, 2H), 7.29 (s, 1H), 3.88 - 3.76 (m, 4H), 3.25 (s, 6H), 2.61 - 2.45 (m, 6H), 1.14 - 1.11 (m, 3H). HRMS (ESI) calcd 507.2085; found, 507.2077.

### Example 30: Synthesis of (5-((4-(2-(dimethylamino)benzothiazole-5-yl)-5-fluoropyrimidine-2-yl)amino)pyridine-2-yl)(4 -isopropylpiperazine-1-yl)ketone (P-30)

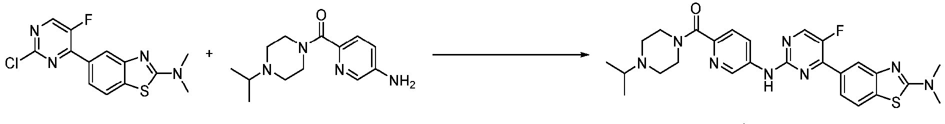

Referring to the synthesis method of Example 1, the yield was 37%. ¹H NMR (400 MHz, CDCl₃) *δ* 8.75 (d, *J =* 2.6 Hz, 1H), 8.39 - 8.34 (m, 2H), 8.31 (s, 1H), 7.84 (d, *J =* 8.3 Hz, 1H), 7.73 (d, *J =* 8.4 Hz, 2H), 7.41 (s, 1H), 3.85 - 3.76 (m, 4H), 3.25 (s, 6H), 2.78 - 2.72 (m, 1H), 2.65 - 2.54 (m, 4H), 1.07 (d, *J =* 6.5 Hz, 6H). HRMS (ESI) calcd 521.2242; found, 521.2240.

### Example 31:Synthesis of tert-butyl 4-(5-((4-(2-(dimethylamino)benzothiazole-5-yl)-5-fluoropyrimidine-2-yl)amino) pyridine-1-yl)piperazine-1-carboxylate (P-31)

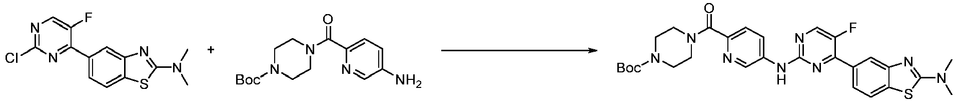

Referring to the synthesis method of Example 1, the yield was 38%, as a white solid. ¹H NMR (400 MHz, CDCl₃) *δ* 8.75 (d, *J =* 2.6 Hz, 1H), 8.40 - 8.35 (m, 2H), 8.32 (d, *J=* 1.7 Hz, 1H), 7.85 - 7.83 (m, 1H), 7.79 - 7.73 (m,2H), 7.33 (s, 1H), 3.79 - 3.75 (m, 4H), 3.57 - 3.48 (m, 4H), 3.26 (s, 6H), 1.48 (s, 9H). HRMS (ESI) calcd 579.2297; found, 579.2290.

### Example 32: Synthesis of (5-((4-(2-(dimethylamino)benzothiazole-5-yl)-5-fluoropyrimidine-2-yl)amino)pyridine-2-yl)(p iperazine-1-yl)ketone hydrochloride (P-32)

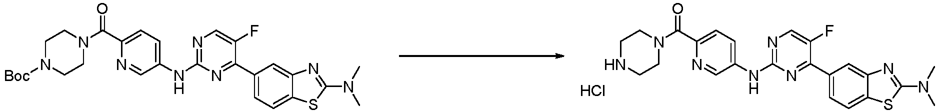

P-31 (100 mg) was dissolved in dichloromethane (15 mL). The mixture was added dropwisely with 3 mL of 2 M EA-HCl solution, and stirred for 8 hours, concentrated to give Compound P-32, yield of 100%, as a light yellow solid. ¹H NMR (400 MHz, DMSO) *δ* 10.42 (s, 1H), 9.63 (s, 2H), 8.98 (d, *J =* 2.5 Hz, 1H), 8.76 (d, *J =* 3.5 Hz, 1H), 8.45 (dd, *J =* 8.7, 2.5 Hz, 1H), 8.25 (s, 1H), 8.07 (d, *J =* 8.4 Hz, 1H), 7.86 - 7.78 (m, 2H), 3.92 - 3.88 (m, 4H), 3.29 (s, 6H), 3.18 - 3.13 (m, 4H). HRMS (ESI) calcd 479.1772; found, 479.1771.

### Example 33: Synthesis of (5-((4-(2-(diethylamino) benzothiazole-5-yl)-5-fluoropyrimidine-2-yl)amino)pyridine-2-yl)(4-ethylpiperazine-1-yl)ketone (P-33)

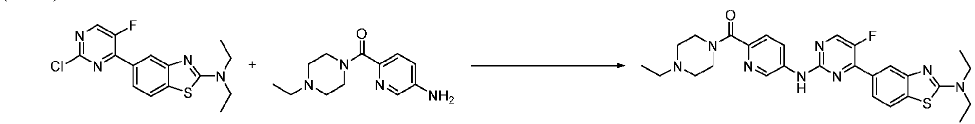

Referring to the synthesis method of Example 1, the yield was 38%. ¹H NMR (400 MHz, CDCl₃) *δ* 8.74 (d, *J* = 2.6 Hz, 1H), 8.39 - 8.35 (m, 2H), 8.29 (s, 1H), 7.82 (d, *J* = 8.3 Hz, 1H), 7.75 - 7.71 (m, 2H), 7.32 (s, 1H), 3.87 - 3.77 (m, 4H), 3.62 (q, *J =* 7.1 Hz, 4H), 2.60 - 2.47 (m, 6H), 1.32 (t, *J =* 7.1 Hz, 6H), 1.12 (t, *J =* 7.2 Hz, 3H). HRMS (ESI) calcd 535.2398; found, 535.2398.

### Example 34: Synthesis of (5-((4-(2-(diethylamino)benzothiazole-5-yl)-5-fluoropyrimidine-2-yl)amino)pyridine-2-yl)(4-is opropylpiperazine-1-yl)ketone (P-34)

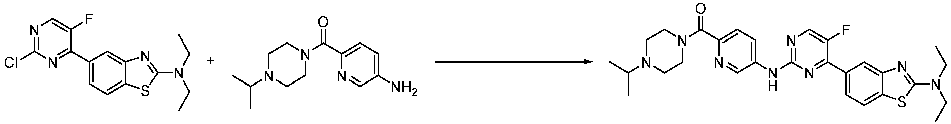

Referring to the synthesis method of Example 1, the yield was 35%, as a white solid. ¹H NMR (400 MHz, CDCl₃) *δ* 8.75 (d, *J =* 2.6 Hz, 1H), 8.38 - 8.35 (m, 2H), 8.28 (s, 1H), 7.81 (d, *J =* 8.3 Hz, 1H), 7.75 - 7.70 (m, 2H), 7.43 (s, 1H), 3.85 - 3.75 (m, 4H), 3.61 (q, *J=* 7.1 Hz, 4H), 2.79 - 2.73 (m, 1H), 2.66 - 2.55 (m, 4H), 1.32 (t, *J* = 7.1 Hz, 6H), 1.07 (d, *J* = 6.5 Hz, 6H). HRMS (ESI) calcd 549.2555; found, 549.2547.

### Example 35: Synthesis of 1-(4-(6-((4-(2-(dimethylamino) benzothiazole-6-yl)-5-fluoropyrimidine-2-yl)amino)nicotinoyl)piperazine-1-yl)ethyl-1-one (P-35)

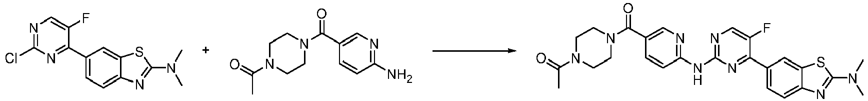

Compound A-12 (309 mg, 1.0 mmol) and B-15 (298 mg, 1.2 mmol) was dissolved in dioxane(10 mL), and then was added with Pd₂(dba)₃(45.8 mg, 0.05 mmol), BINAP(62.3 mg, 0.1 mmol), cesium carbonate (651.6 mg, 2.0 mmol). The system was replaced with argon three times, and was heated to 100°C, in order to react for 12h. The mixture was cooled, filtrated, and concentrated, and purified by column chromatography to give Compound P35 (193 mg, 37% yield), as a white solid. ¹H NMR (300 MHz, DMSO) *δ* 10.36 (s, 1H), 8.70 (d, *J =* 3.8 Hz, 1H), 8.53 (d, *J =* 1.8 Hz, 1H), 8.40 (dd, *J =* 2.4, 0.8 Hz, 1H), 8.31 (d, *J =* 8.7 Hz, 1H), 8.06 (dt, *J =* 8.8, 1.2 Hz, 1H), 7.90 (dd, *J* = 8.7, 2.4 Hz, 1H), 7.59 (d, *J =* 8.6 Hz, 1H), 3.55 - 3.47 (m, 8H), 3.21 (s, 6H), 2.03 (s, 3H). HRMS (ESI) calcd 521.1878; found, 521.1880.

### Example 36: Synthesis of (4-(cyclopropylcarbonyl)piperazine-1-yl)(6-((4-(2-(dimethylamino) benzothiazole-6-yl)-5-fluoropyrimidine-2-yl)amino)pyridine-3-yl)ketone (P-36)

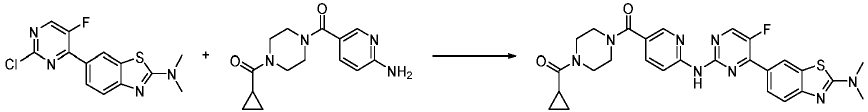

Referring to the synthesis method of Example 35, the yield was 35%. ¹H NMR (300 MHz, DMSO) *δ* 10.33 (s, 1H), 8.70 (d, *J =* 3.8 Hz, 1H), 8.53 (d, *J =* 1.8 Hz, 1H), 8.41 (dd, *J =* 2.4, 0.8 Hz, 1H), 8.34 - 8.31 (m, 1H), 8.08 - 8.05 (m, 1H), 7.91 (dd, *J =* 8.7, 2.4 Hz, 1H), 7.59 (d, *J =* 8.6 Hz, 1H), 3.76 - 3.49 (m, 8H), 3.21 (s, 6H), 2.03 - 1.96 (m, 1H), 0.77 - 0.71 (m, 4H). HRMS (ESI) calcd 547.2034; found, 547.2032.

### Example 37: Synthesis of (6-((4-(2-(dimethylamino)benzothiazole-6-yl)-5-fluoropyrimidine-2-yl)amino)pyridine-3-yl)(4 - aryl piperazine-1-yl)ketone (P-37)

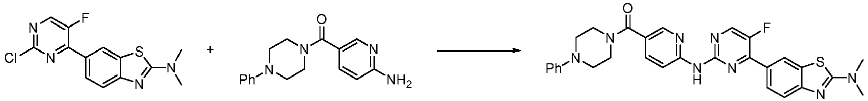

Referring to the synthesis method of Example 35, the yield was 32%. ¹H NMR (400 MHz, CDCl₃) *δ* 8.52 (dd, *J =* 8.7, 0.8 Hz, 1H), 8.48 (dd, *J =* 2.4, 0.8 Hz, 1H), 8.45 - 8.44 (m, 2H), 8.42 (d, *J =* 3.8 Hz, 1H), 8.16 - 8.13 (m, 1H), 7.87 (dd, *J =* 8.7, 2.3 Hz, 1H), 7.65 (d, *J* = 8.6 Hz, 1H), 7.32 - 7.28 (m, 2H), 6.96 - 6.90 (m, 3H), 3.89 - 3.77 (m, 4H), 3.27 (s, 6H), 3.25 - 3.20 (m, 4H). HRMS (ESI) calcd 555.2085; found, 555.2084.

### Example 38: Synthesis of ethyl 4-(6-((4-(2-(dimethylamino)benzothiazole-6-yl)-5-fluoropyrimidine-2-yl)amino)nicotinoyl)piperazi ne-1-carboxylate (P-38)

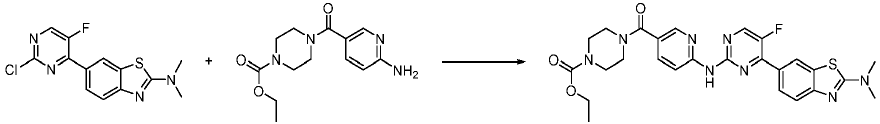

Referring to the synthesis method of Example 35, the yield was 34%. ¹H NMR (300 MHz, CDCl₃) *δ* 8.52 (dd, *J =* 8.7, 0.8 Hz, 1H), 8.47 - 8.42 (m, 4H), 8.17 - 8.13 (m, 1H), 7.84 (dd, *J* = 8.7, 2.3 Hz, 1H), 7.65 (d, *J =* 8.6 Hz, 1H), 4.18 (q, *J =* 7.1 Hz, 2H), 3.70 - 3.52 (m, 8H), 3.28 (s, 6H), 1.31 - 1.26 (m, 3H). HRMS (ESI) calcd 551.1984; found, 551.1984.

### Example 39: Synthesis of 1-(4-(6-((4-(2-(diethylamino)benzothiazole-6-yl)-5-fluoropyrimidine-2-yl)amino)nicotinoyl)pi perazine-1-yl)ethyl-1-one (P-39)

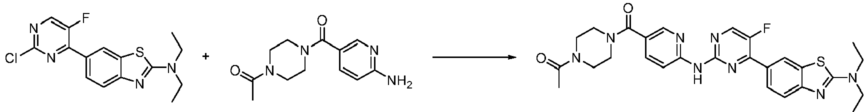

Referring to the synthesis method of Example 35, the yield was 30%. ¹H NMR (300 MHz, CDCl₃) *δ* 8.68 (s, 1H), 8.54 (dd, *J =* 8.8, 0.8 Hz, 1H), 8.45 - 8.41 (m, 3H), 8.15 - 8.12 (m, 1H), 7.86 (dd, *J* = 8.7, 2.3 Hz, 1H), 7.63 (d, *J* = 8.6 Hz, 1H), 3.72 - 3.54 (m, 12H), 2.15 (s, 3H), 1.33 (t, *J* = 7.1 Hz, 6H). HRMS (ESI) calcd 549.2191; found, 549.2188.

### Example 40: Synthesis of (4-(cyclopropylcarbonyl)piperazine-1-yl)(6-((4-(2-(diethylamino) benzothiazole-6-yl)-5-fluoropyrimidine-2-yl)amino)pyridine-3-yl)ketone (P-40)

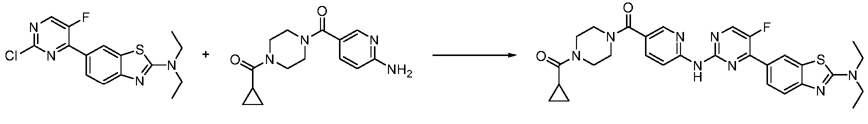

Referring to the synthesis method of Example 35, the yield was 31%. ¹H NMR (300 MHz, CDCl₃) *δ* 8.89 (s, 1H), 8.56 - 8.52 (m, 2H), 8.45 (d, *J =* 3.8 Hz, 1H), 8.43 (d, *J=* 1.8 Hz, 1H), 8.13 (ddd, *J =* 8.6, 1.9, 1.0 Hz, 1H), 7.86 (dd, *J=* 8.7, 2.4 Hz, 1H), 7.63 (d, *J=* 8.6 Hz, 1H), 3.74 - 3.60 (m, 12H), 1.79 - 1.73 (m, 1H), 1.33 (t, *J=* 7.1 Hz, 6H), 1.04 - 1.01 (m, 2H), 0.85 - 0.80 (m, 2H). HRMS (ESI) calcd 575.2347; found, 575.2344.

### Example 41: Synthesis of 1-(4-(6-((4-(2-(cyclopenylamino)benzothiazole-6-yl)-5-fluoropyrimidine-2-yl)amino)nicotinoy 1)piperazine-1-yl)ethyl-1-one (P-41)

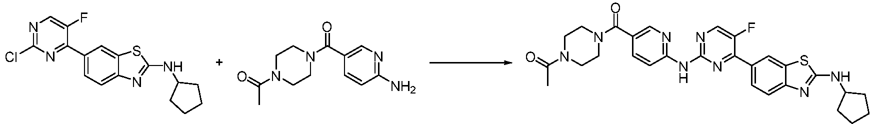

Referring to the synthesis method of Example 35, the yield was 41%. ¹H NMR (400 MHz, CDCl₃) *δ* 8.53 - 8.50 (m, 2H), 8.45 (dd, *J =* 2.4, 0.8 Hz, 1H), 8.43 - 8.42 (m, 2H), 8.13 - 8.10 (m, 1H), 7.84 (dd, *J* = 8.7, 2.4 Hz, 1H), 7.63 (d, *J =* 8.6 Hz, 1H), 5.82 (s, 1H), 4.11 - 4.05 (m, 1H), 3.73 - 3.53 (m, 8H), 2.24 - 2.17 (m, 2H), 2.15 (s, 3H), 1.83 - 1.68 (m, 6H). ¹³C NMR (126 MHz, CDCl₃) *δ* 168.36, 167.73, 167.27, 155.27, 154.98, 153.90, 151.55, 151.05, 150.98, 149.52, 147.08, 146.93, 137.42, 137.31, 130.89, 126.74, 126.69, 124.93, 124.89, 124.31, 121.53, 117.65, 110.89, 55.71, 39.76, 39.60, 39.43, 39.26, 39.10, 23.25, 21.16.

### Example 42: Synthesis of (4-(6-((4-(2-(cyclopenylamino) benzothiazole-6-yl)-5-fluoropyrimidine-2-yl)amino) nicotinoyl)piperazine-1-yl)(cyclopropyl)ketone (P-42)

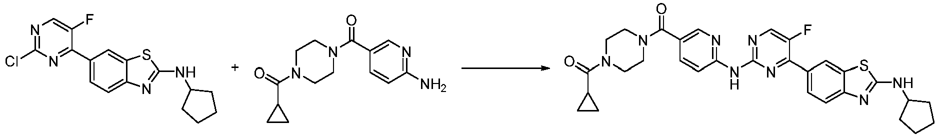

Referring to the synthesis method of Example 35, the yield was 43%. ¹H NMR (400 MHz, CDCl₃) *δ* 8.65 (s, 1H), 8.53 - 8.51 (m, 1H), 8.47 - 8.41 (m, 3H), 8.11 - 8.09 (m, 1H), 7.85 (dd, *J=* 8.7, 2.4 Hz, 1H), 7.63 (d, *J =* 8.6 Hz, 1H), 5.89 (s, 1H), 4.12 - 4.06 (m, 1H), 3.78 - 3.64 (m, 8H), 2.18 - 2.14 (m, 2H), 1.80 - 1.66 (m, 7H), 1.05 - 1.01 (m, 2H), 0.84 - 0.80 (m, 2H). ¹³C NMR (126 MHz, CDCl₃) δ 171.18, 167.71, 167.26, 155.26, 154.99, 153.90, 151.54, 151.03, 150.96, 149.52, 147.13, 146.96, 137.40, 137.28, 130.88, 126.68, 124.93, 124.89, 124.31, 121.50, 117.65, 110.78, 55.71, 32.22, 23.24, 10.27, 7.05.

### Example 43: Synthesis of (6-((4-(benzothiazole-6-yl)-5-fluoropyrimidine-2-yl)amino)pyridine-3-yl)(4-isopropylpiperazi ne-1-yl)ketone (P-43)

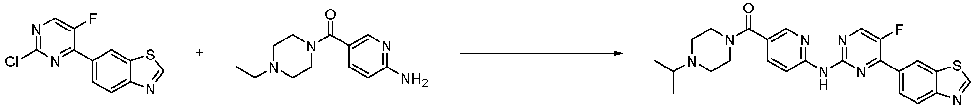

Referring to the synthesis method of Example 35, the yield was 41%, as a white solid. ¹H NMR (400 MHz, CDCl₃) *δ* 9.62 (s, 1H), 9.15 (s, 1H), 8.76 (s, 1H), 8.62 - 8.59 (m, 2H), 8.54 - 8.51 (m, 1H), 8.33 - 8.28 (m, 2H), 7.89 - 7.85 (m, 1H), 3.80 - 3.52 (m, 4H), 2.77 - 2.72 (m, 1H), 2.59 - 2.49 (m, 4H), 1.06 (d, *J* = 6.7 Hz, 6H). ¹³C NMR (126 MHz, DMSO) *δ* 166.77, 159.14, 155.19, 155.17, 154.37, 153.61, 151.74, 150.69, 150.62, 149.71, 147.76, 147.55, 146.85, 137.20, 134.14, 129.91, 126.58, 126.54, 124.83, 123.54, 123.48, 123.16, 110.93, 53.61, 17.97.

### Example 44: Synthesis of 1-(4-(6-((4-(benzothiazole-6-yl)-5-fluoropyrimidine-2-yl)amino) nicotinoyl)piperazine-1-yl)ethyl-1-one (P-44)

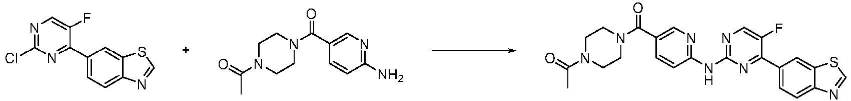

Referring to the synthesis method of Example 35, the yield was 44%. ¹H NMR (400 MHz, CDCl₃) *δ* 9.16 (s, 1H), 8.78 (s, 1H), 8.53 - 8.50 (m, 2H), 8.47 - 8.44 (m, 2H), 8.34 - 8.28 (m, 2H), 7.86 (dd, *J* = 8.7, 2.3 Hz, 1H), 3.73 - 3.53 (m, 9H), 2.15 (s, 3H). ¹³C NMR (101 MHz, DMSO) δ 168.92, 167.80, 159.73, 155.74, 155.71, 154.95, 154.35, 152.58, 151.28, 151.19, 150.04, 148.37, 148.11, 147.60, 137.93, 134.72, 130.46, 130.41, 127.16, 127.10, 125.07, 124.12, 124.05, 123.73, 111.50, 46.01, 21.73.

### Example 45: Synthesis of (4-(6-((4-(benzothiazole-6-yl)-5-fluoropyrimidine-2-yl)amino) nicotinoyl)piperazine-1-yl)(cyclopropyl)ketone (P-45)

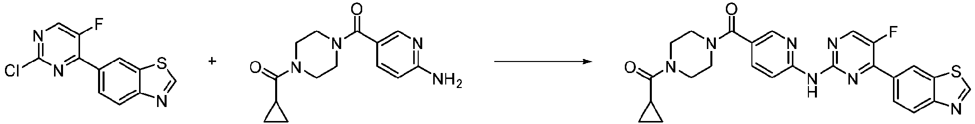

Referring to the synthesis method of Example 35, the yield was 42%. ¹H NMR (400 MHz, CDCl₃) *δ* 9.16 (s, 1H), 8.78 (s, 1H), 8.52 (dd, *J* = 6.0, 2.7 Hz, 2H), 8.46 (d, *J* = 2.3 Hz, 1H), 8.35 - 8.28 (m, 3H), 7.87 (dd, *J =* 8.7, 2.4 Hz, 1H), 3.81 - 3.62 (m, 8H), 1.77 - 1.71 (m, 1H), 1.04 - 1.02 (m, 2H), 0.84 - 0.81 (m, 2H). ¹³C NMR (126 MHz, DMSO) *δ* 171.21, 167.22, 159.11, 155.12, 155.10, 154.38, 153.77, 151.75, 150.70, 150.62, 149.72, 147.73, 147.52, 147.06, 137.44, 134.15, 129.87, 129.83, 126.57, 126.52, 124.48, 123.51, 123.45, 123.14, 110.93, 10.30, 7.06.

### Example 46: Synthesis of 4-(benzothiazole-6-yl)-N-(5-((4-isopropylpiperazine-1-yl)methyl)pyridine-2-yl)-5-methylpyri midine-2-amine (P-46)

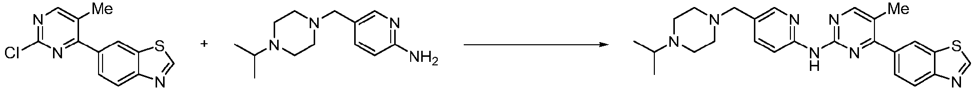

Referring to the synthesis method of Example 35, the yield was 42%. ¹H NMR (400 MHz, CDCl₃) *δ* 9.12 (s, 1H), 8.45 (s, 1H), 8.42 (d, *J* = 8.6 Hz, 1H), 8.30 - 8.24 (m, 4H), 7.82 (dd, *J* = 8.5, 1.7 Hz, 1H), 7.61 (d, *J =* 8.6 Hz, 1H), 3.55 (s, 2H), 3.33 - 2.86 (m, 9H), 2.34 (s, 3H), 1.38 (d, *J =* 6.8 Hz, 6H).

### Example 47: Synthesis of tert-butyl 4-(6-((4-(benzothiazole-6-yl)-5-methylpyrimidine-2-yl)amino) nicotinoyl)piperazine-1-carboxylate (P-47)

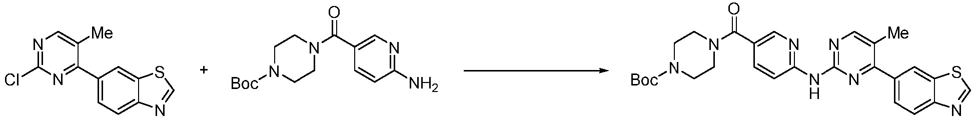

Referring to the synthesis method of Example 35, the yield was 34%. ¹H NMR (400 MHz, CDCl₃) *δ* 9.12 (s, 1H), 8.53 (dd, *J =* 8.7, 0.8 Hz, 1H), 8.48 - 8.46 (m, 2H), 8.44 (dd, *J =* 2.4, 0.8 Hz, 1H), 8.28 - 8.26 (m, 2H), 7.82 (dd, *J* = 8.6, 1.7 Hz, 1H), 7.76 (dd, *J* = 8.7, 2.3 Hz, 1H), 3.66 - 3.45 (m, 8H), 2.36 (s, 3H), 1.48 (s, 9H).

### Example 48: Synthesis of (6-((4-(benzothiazole-6-yl)-5-methylpyrimidine-2-yl)amino)pyridine-3-yl)(4-isopropylpiperazi ne-1-yl)ketone (P-48)

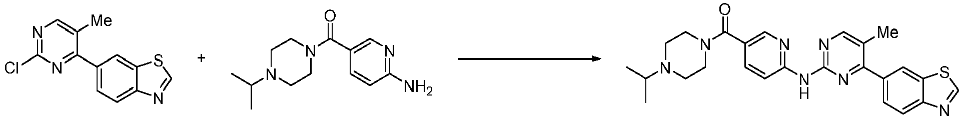

Referring to the synthesis method of Example 35, the yield was 39%. ¹H NMR (400 MHz, CDCl₃) *δ* 9.12 (s, 1H), 8.51 - 8.49 (m, 1H), 8.46 (s, 1H), 8.42 (d, *J =* 2.2 Hz, 1H), 8.28 - 8.26 (m, 3H), 7.82 (dd, *J =* 8.6, 1.6 Hz, 1H), 7.77 (dd, *J =* 8.7, 2.3 Hz, 1H), 3.75 - 3.55 (m, 4H), 2.77 - 2.71 (m, 1H), 2.59 - 2.36 (m, 4H), 2.36 (s, 3H), 1.06 (d, *J =* 6.5 Hz, 6H).

### Example 49: Synthesis of (6-((4-(benzothiazole-6-yl)-5-methylpyrimidine-2-yl)amino) pyridine-3-yl)(piperazine-1-yl)ketone hydrochloride (P-49)

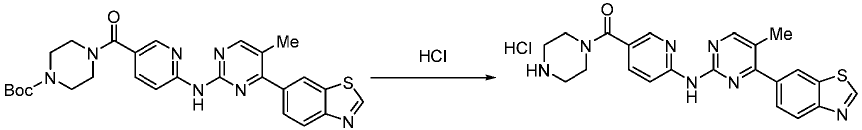

P47 (100 mg) was dissolved in dichloromethane (15 mL). The mixture was added dropwisely with 3 mL of 2 M EA-HCl solution, and stirred for 8 hours, concentrated to give Compound P49, yield of 100%, as a white solid. ¹H NMR (400 MHz, DMSO) *δ* 11.83 (s, 1H), 9.68 (s, 2H), 9.56 (s, 1H), 8.73 (s, 1H), 8.62 (d, *J =* 1.7 Hz, 1H), 8.56 (d, *J =* 2.2 Hz, 1H), 8.27 (d, *J =* 8.5 Hz, 1H), 8.20 (dd, *J* = 8.9, 2.1 Hz, 1H), 7.96 - 7.91 (m, 2H), 3.80 - 3.76 (m, 4H), 3.18 - 3.16 (m, 4H), 2.42 (s, 3H).

### Example 50: Synthesis of 4-(benzothiazole-6-yl)-N-(5-((4-isopropylpiperazine-1-yl)methyl)pyridine-2-yl)-5-methoxypyr imidine-2-amine (P-50)

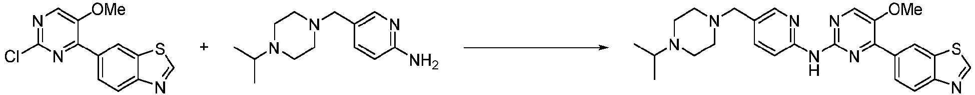

Referring to the synthesis method of Example 35, the yield was 52%. H NMR (400 MHz, CDCl₃) *δ* 9.10 (s, 1H), 8.80 (dd, *J* = 1.7, 0.7 Hz, 1H), 8.37 (dd, *J* = 8.6, 0.9 Hz, 1H), 8.35 - 8.32 (m, 2H), 8.24 (dd, *J =* 8.6, 0.6 Hz, 1H), 8.21 - 8.20 (m, 1H), 8.03 (s, 1H), 7.68 (dd, *J =* 8.6, 2.3 Hz, 1H), 3.95 (s, 3H), 3.48 (s, 2H), 2.68 - 2.49 (m, 9H), 1.05 (d, *J =* 6.5 Hz, 6H).

### Example 51: Synthesis of tert-butyl 4-(6-((4-(benzothiazole-6-yl)-5-methoxypyrimidine-2-yl)amino) nicotinoyl)piperazine-1-carboxylate (P-51)

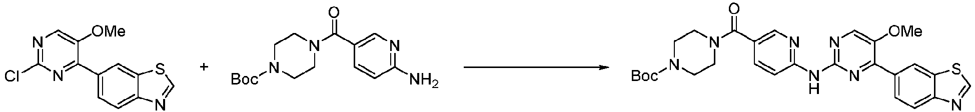

Referring to the synthesis method of Example 35, the yield was 43%. ¹H NMR (400 MHz, CDCl₃) *δ* 9.12 (s, 1H), 8.79 (d, *J =* 1.7 Hz, 1H), 8.53 - 8.49 (m, 2H), 8.45 (dd, *J* = 2.4, 0.9 Hz, 1H), 8.39 (s, 1H), 8.32 (dd, *J =* 8.7, 1.7 Hz, 1H), 8.25 (d, *J =* 8.7 Hz, 1H), 7.80 (dd, *J =* 8.7, 2.4 Hz, 1H), 3.97 (s, 3H), 3.65 - 3.46 (m, 8H), 1.48 (s, 9H).

### Example 52: Synthesis of (6-((4-(benzothiazole-6-yl)-5-methoxypyrimidine-2-yl)amino)pyridine-3-yl)(4-isopropylpipera zine-1-yl)ketone (P-52)

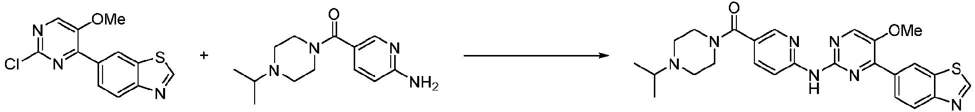

Referring to the synthesis method of Example 35, the yield was 45%. ¹H NMR (400 MHz, CDCl₃) *δ* 9.11 (s, 1H), 8.80 - 8.79 (m, 1H), 8.48 (dd, *J =* 8.7, 0.9 Hz, 1H), 8.44 (dd, *J* = 2.4, 0.8 Hz, 1H), 8.40 (s, 1H), 8.38 (s, 1H), 8.32 (dd, *J =* 8.6, 1.7 Hz, 1H), 8.25 (dd, *J =* 8.7, 0.6 Hz, 1H), 7.81 (dd, *J =* 8.7, 2.3 Hz, 1H), 3.96 (s, 3H), 3.81 - 3.56 (m, 4H), 2.79 - 2.71 (m, 1H), 2.61 - 2.52 (m, 4H), 1.07 (d, *J =* 6.5 Hz, 6H).

### Example 53: Synthesis of (6-((4-(benzothiazole-6-yl)-5-methoxypyrimidine-2-yl)amino) pyridine-3-yl)(piperazine-1-yl)ketone hydrochloride (P-53)

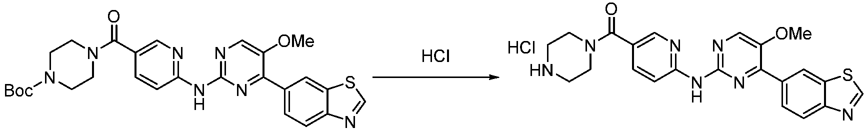

P51 (100 mg) was dissolved in dichloromethane (15 mL). The mixture was added dropwisely with 3 mL of 2 M EA-HCl solution, and stirred for 8 hours, concentrated to give Compound P53, yield of 100%, as a white solid. ¹H NMR (400 MHz, DMSO) *δ* 12.05 (s, 1H), 9.76 (s, 2H), 9.58 (s, 1H), 8.95 (d, *J =* 1.5 Hz, 1H), 8.66 (s, 1H), 8.58 (d, *J =* 2.2 Hz, 1H), 8.30 - 8.24 (m, 3H), 7.91 (d, *J* = 9.0 Hz, 1H), 4.06 (s, 3H), 3.82 - 3.76 (m, 4H), 3.19 - 3.16 (m, 4H).

### Example 54: Synthesis of (6-((4-(benzothiazole-6-yl)pyrimidine-2-yl)amino)pyridine-3-yl)(4-isopropylpiperazine-1-yl)k etone (P-54) Synthesis

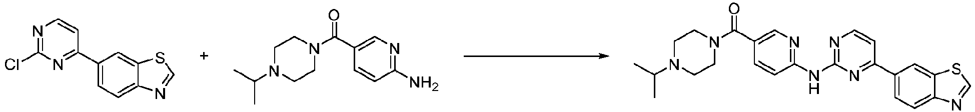

Referring to the synthesis method of Example 35, the yield was 42%. ¹H NMR (400 MHz, CDCl₃) *δ* 9.13 (s, 1H), 8.76 (d, *J =* 1.6 Hz, 1H), 8.62 (d, *J =* 5.3 Hz, 1H), 8.59 (d, *J =* 8.6 Hz, 1H), 8.44 (d, *J* = 2.3 Hz, 1H), 8.27 (d, *J* = 8.6 Hz, 1H), 8.22 (dd, *J* = 8.6, 1.6 Hz, 1H), 7.86 (dd, *J* = 8.6, 2.3 Hz, 1H), 7.37 (d, *J =* 5.2 Hz, 1H), 3.80 - 3.55 (m, 4H), 2.79 - 2.72 (m, 1H), 2.60 - 2.52 (m, 4H), 1.07 (d, *J =* 6.5 Hz, 6H). ¹³C NMR (126 MHz, DMSO) *δ* 167.38, 163.81, 159.73, 159.60, 159.16, 155.23, 154.24, 147.44, 137.84, 134.95, 134.07, 125.64, 125.34, 123.79, 122.10, 112.14, 110.35, 54.29, 18.50.

### Example 55: Synthesis of 5-fluoro-N-(5-((4-isopropylpiperazine-1-yl)methyl)pyridine-2-yl)-4-(2-methylbenzothiazole-6-yl)pyrimidine-2-amine (P-55)

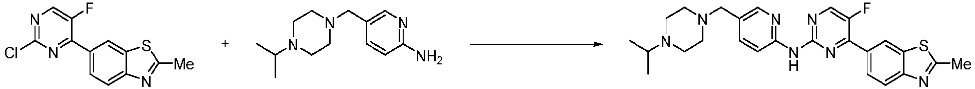

Referring to the synthesis method of Example 35, the yield was 51%. ¹H NMR (400 MHz, CDCl₃) *δ* 8.64 (s, 1H), 8.46 (d, *J =* 3.5 Hz, 1H), 8.38 (d, *J =* 8.5 Hz, 1H), 8.33 - 8.21 (m, 3H), 8.08 (d, *J =* 8.6 Hz, 1H), 7.70 (d, *J =* 8.6 Hz, 1H), 3.54 (s, 2H), 2.91 (s, 3H), 2.88 - 2.50 (m, 9H), 1.24 (d, *J* = 17.2 Hz, 6H).

### Example 56: Synthesis of (6-((5-fluoro-4-(2-methylbenzothiazole-6-yl)pyrimidine-2-yl)amino) pyridine-3-yl)(4isopropylpiperazine-1-yl)ketone (P-56)

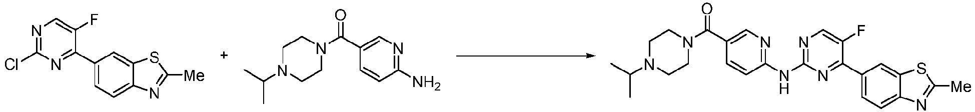

Referring to the synthesis method of Example 35, the yield was 42%. ¹H NMR (400 MHz, CDCl₃) *δ* 8.64 (d, *J =* 1.7 Hz, 1H), 8.48 - 8.46 (m, 2H), 8.44 (dd, *J =* 2.3, 0.8 Hz, 1H), 8.30 (s, 1H), 8.26 - 8.24 (m, 1H), 8.09 (d, *J =* 8.6 Hz, 1H), 7.84 (dd, *J =* 8.6, 2.3 Hz, 1H), 3.80 - 3.55 (m, 4H), 2.91 (s, 3H), 2.78 - 2.72 (m, 1H), 2.61 - 2.50 (m, 4H), 1.07 (d, *J* = 6.4 Hz, 6H). ¹³C NMR (126 MHz, CDCl₃) *δ* 170.03, 167.97, 155.12, 155.10, 153.82, 152.28, 151.79, 151.72, 150.24, 147.54, 147.35, 147.14, 137.92, 136.27, 129.74, 129.69, 126.80, 126.75, 125.03, 122.65, 122.59, 122.57, 111.03, 54.58, 20.43, 18.37.

### Example 57: Synthesis of 1-(4-(6-((5-fluoro-4-(2-methylbenzothiazole-6-yl)pyrimidine-2-yl)amino) nicotinoyl)piperazine-1-yl)ethyl-1-one (P-57)

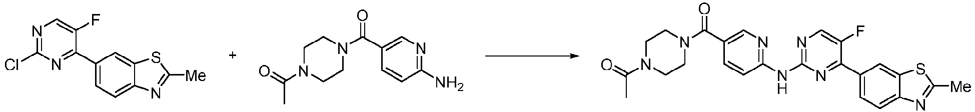

Referring to the synthesis method of Example 35, the yield was 38%. ¹H NMR (400 MHz, CDCl₃) *δ* 8.64 (d, *J =* 1.7 Hz, 1H), 8.53 - 8.46 (m, 4H), 8.26 - 8.24 (m, 1H), 8.09 (d, *J =* 8.6 Hz, 1H), 7.86 (dd, *J=* 8.7, 2.4 Hz, 1H), 3.76 - 3.54 (m, 8H), 2.91 (s, 3H), 2.15 (s, 3H). ¹³C NMR (126 MHz, CDCl₃) *δ* 170.13, 169.22, 168.49, 155.06, 154.33, 152.28, 151.81, 151.74, 150.23, 147.68, 147.38, 147.17, 137.98, 136.25, 129.67, 129.62, 126.77, 126.72, 124.07, 122.65, 122.59, 122.56, 111.16, 46.16, 41.49, 21.39, 20.42.

### Example 58: Synthesis of (4-(cyclopropylcarbonyl)piperazine-1-yl)(6-((5-fluoro-4-(2-methylbenzothiazole-6-yl)pyrimidi ne-2-yl)amino)pyridine-3-yl)ketone (P-58) Synthesis

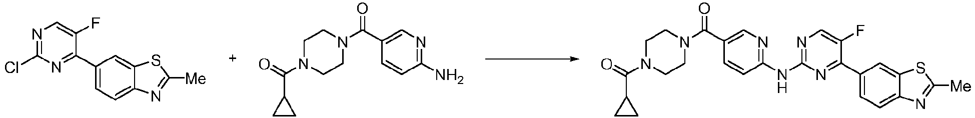

Referring to the synthesis method of Example 35, the yield was 30%. ¹H NMR (400 MHz, CDCl₃) *δ* 8.70 (s, 1H), 8.64 (d, *J* = 1.7 Hz, 1H), 8.54 - 8.50 (m, 3H), 8.26 - 8.23 (m, 1H), 8.09 (d, *J* = 8.6 Hz, 1H), 7.87 (dd, *J* = 8.7, 2.4 Hz, 1H), 3.79 - 3.63 (m, 8H), 2.91 (s, 3H), 1.78 - 1.72 (m, 1H), 1.05 - 1.01 (m, 2H), 0.85 - 0.80 (m, 2H). ¹³C NMR (101 MHz, DMSO) *δ* 170.86, 167.79, 155.71, 155.68, 155.00, 154.37, 154.37, 152.54, 151.31, 151.22, 150.01, 148.26, 147.62, 137.92, 136.29, 129.71, 129.66, 127.07, 127.01, 125.04, 123.49, 123.41, 122.60, 111.47, 21.23, 10.83, 7.62.

### Biological Assay Experiments:

### I. DYRK2 kinase activity analysis assay

To evaluate the inhibitory effect of the example compound P-1 to P-40 on DYRK2 kinase, we first prepared a reaction buffer containing 20 mM HEPES (pH 7.5), 10 mM MgCl₂, 1 mM EGTA, 0.01% Brij35, 0.02 mg/mL BSA, 0.1 mM Na₃VO₄, 2 mM DTT, and 1% DMSO. Subsequently, DYRK2 kinase and DYRKtide substrates were added to the reaction buffer and mixed gently. The compound to be tested was added to the reaction mixture and ³³P-ATP was added to initiate the reaction. The reaction was then observed on P81 ion exchange paper and incubated for an additional 2 hours at room temperature. Finally, the residual DYRK2 activity of the test compound group relative to the DMSO control group was calculated by measuring the residual hyperphosphorylated substrate on the filter paper.

The measured IC₅₀ values are shown in Table 1 below. From the experimental results, it can be seen that the compounds of the examples of the present invention have strong inhibitory activity against DYRK2 kinase activity.

**Table 1 IC₅₀ measured values of the compounds of the invention against DYRK2 kinase activity**

| Example | IC₅₀ (nM) | Example | IC₅₀ (nM) | Example | IC₅₀ (nM) |
|---|---|---|---|---|---|
| 1 | 1474 | 21 | 1053 | 41 | 85 |
| 2 | 1415 | 22 | 535 | 42 | 128 |
| 3 | 498 | 23 | 695 | 43 | 44 |
| 4 | 268 | 24 | 15 | 44 | 26 |
| 5 | 357 | 25 | 16 | 45 | 40 |
| 6 | 774 | 26 | 4 | 46 | 7761 |
| 7 | 779 | 27 | 18 | 47 | >10,000 |
| 8 | 636 | 28 | 35 | 48 | 1723 |
| 9 | 67 | 29 | 11 | 49 | 2107 |
| 10 | 99 | 30 | 13 | 50 | >10,000 |
| 11 | 190 | 31 | 590 | 51 | >10,000 |
| 12 | 430 | 32 | 9 | 52 | >10,000 |
| 13 | 216 | 33 | 29 | 53 | 8,519 |
| 14 | 22 | 34 | 45 | 54 | 58 |
| 15 | 28 | 35 | 22 | 55 | 203 |
| 16 | 33 | 36 | 16 | 56 | 147 |
| 17 | 56 | 37 | 683 | 57 | 30 |
| 18 | 29 | 38 | 91 | 58 | 58 |
| 19 | 70 | 39 | 15 | | |
| 20 | 0.6 | 40 | 14 | | |

Most of the compounds prepared by the invention have strong inhibitory activity on DYRK2 kinase, wherein the IC₅₀ measured values of most of the compounds on DYRK2 kinase are less than 1000nM, and the IC₅₀ measured values of Examples 20, 24, 25, 26, 27, 29, 30, 32, 36, 39 and 40 are less than 20nM. As shown in the table, the compounds of the invention have very good inhibitory activity on DYRK2 kinase.

### II. Cell proliferation inhibition activity assay

The inhibitory effect of the compound on the proliferation of prostate cancer DU145 and 22Rv1 cells was measured according to the CCK-8 method. The preferred compound Example 20 or Example 40 or Example 39 was incubated with DU145 or 22Rv1 cells in a 96-well plate for 72 hours, followed by the addition of CCK8 reagent and incubation at 37 °C, under 5% CO₂ for 2 hours. After the incubation, the absorbance value at 450 nm was measured by a microplate reader, and the half inhibitory concentration IC₅₀ of the compound against cell proliferation activity was fitted by GraphPad Prism 6.

| Compound | IC₅₀(µM) | |
|---|---|---|
| | DU145 | 22Rv1 |
| Example 20 | 3.255 ± 0.060 | 2.552 ± 0.046 |
| Example 40 | 7.461 ± 0.31 | 4.38 ± 0.51 |
| Example 39 | 16.04±0.256 | 13.85±2.61 |

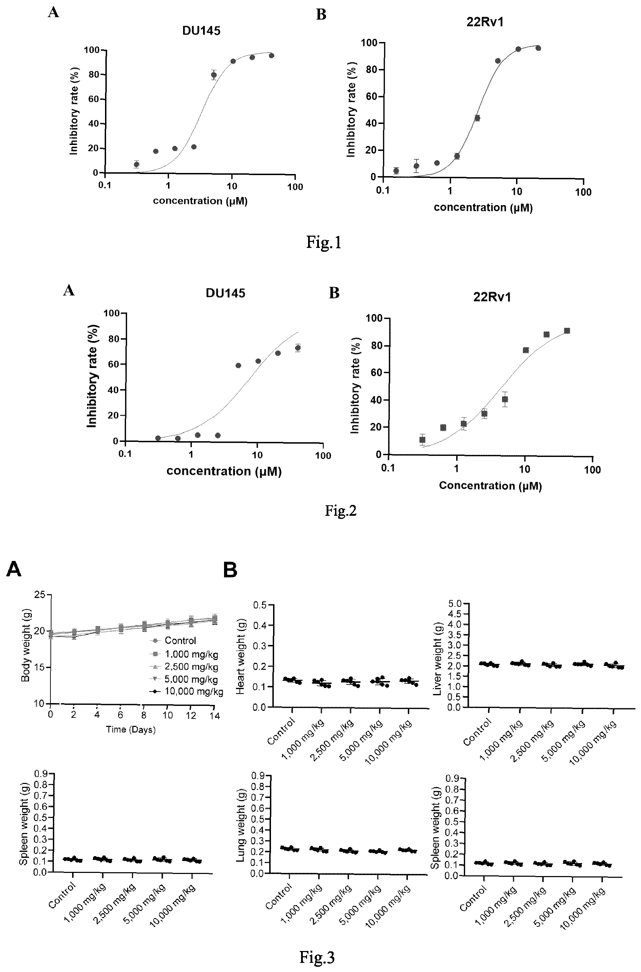

As can be seen from that table, the compound of Example 20 and Example 40 significantly inhibit the proliferation of prostate cancer DU145 and 22Rv1 cell, see Figures 1 and 2.

### III. Acute toxicity test

### 1. Acute toxicity t of the compound of Example 20

The ICR mice were divided into five groups, one control group and four dosing groups. Example 20 was given by gavage, with six mice in each group. The control group was given the same amount of normal saline, and the four dosing groups were given the compound of Example 20 by gavage once at the doses of 1000mg/kg, 2500mg/kg, 5000mg/kg, and 10000mg/kg, respectively. The poisoning symptoms and death of the mice were recorded, and the dead animals were subjected to autopsy. The observation period was 14 days.

The results showed that no abnormality was observed within 12 hours after dosing in mice of each group. No animals died within 24 hours of dosing and no animals died after day 14 of dosing. The mice were dissected and the main organs including heart, liver, spleen, lung and kidney were weighed. It was found that there was no difference in the weight of main organs between the administration group and the control group. Therefore, Example 20 has no obvious toxic or side effects and is good in safety, as shown in Figure 3.

### 2. Acute toxicity t of the compound of Example 40

The ICR mice were divided into five groups, one control group and four dosing groups. Example 40 was given by gavage, with six mice in each group. The control group was given the same amount of normal saline, and the four dosing groups were given the compound of Example 40 by gavage once at the doses of 1000mg/kg, 2500mg/kg, 5000mg/kg, and 10000mg/kg, respectively. The poisoning symptoms and death of the mice were recorded, and the dead animals were subjected to autopsy. The observation period was 14 days.

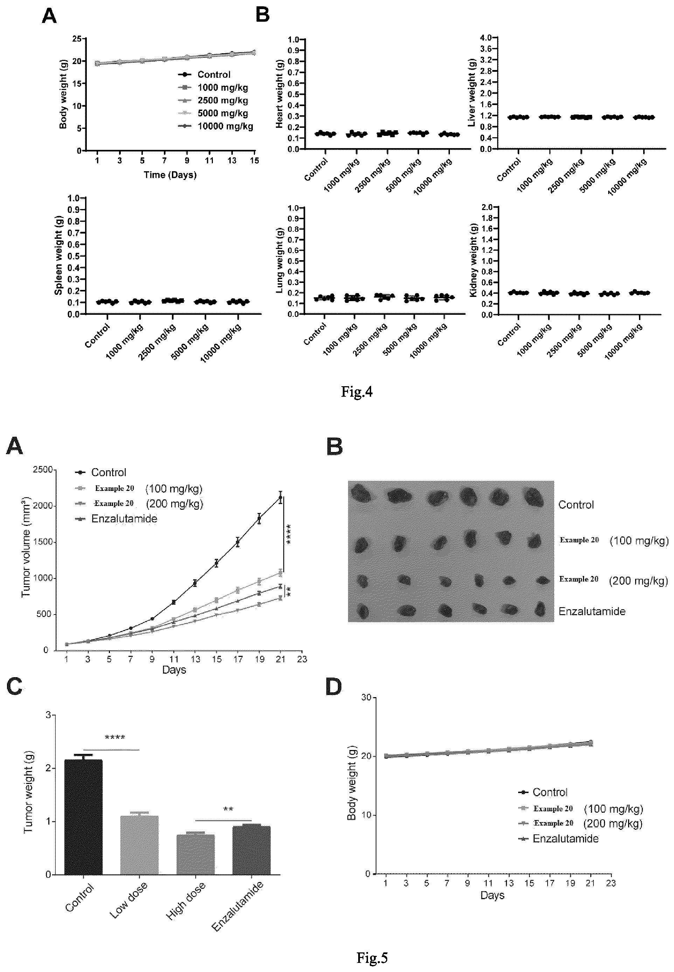

The results showed that no abnormality was observed within 12 hours after dosing in mice of each group. No animals died within 24 hours of dosing and no animals died after day 14 of dosing. The mice were dissected and the main organs including heart, liver, spleen, lung and kidney were weighed. It was found that there was no difference in the weight of main organs between the administration group and the control group. Therefore, Example 40 has no obvious toxic or side effects and is good in safety, as shown in Figure 4.

### IV. In vivo efficacy assay against prostate cancer

### 1. In vivo efficacy assay of Example 20 against prostate cancer

DU145 cells were inoculated subcutaneously at the right axilla of BALB/c nude mice. When the tumor grew to about 85 mm³, 24 tumor-bearing nude mice in good growth condition and with good tumor size uniformity were selected and divided into four groups, with six mice in each group, i.e., the model group, the low-dose group of Example 20 (100 mg/kg), the high-dose group of Example 20 (200 mg/kg), and the positive drug enzalutamide group (100 mg/kg). They were administered once a day. Tumor diameters were measured every other day and nude mice were weighed while tumor diameters were measured. The nude mice were euthanized 21 days after dosing and tumor blocks were surgically removed and weighed.

The experimental result show that that T/C(%) of human prostate cancer cell DU145 xenografts in nude mice in the low dose group of Example 20 is 50.9%, and the tumor inhibition rate is 48.7%; Example 20 T/C(%) of human prostate cancer cell DU145 xenografts in nude mice in the high dose group was 34.4%, and the tumor inhibition rate was 65.3%. The T/C(%) of human prostate cancer cell DU145 xenografts in nude mice treated with the positive drug enzalutamide was 42.0%, and the tumor inhibition rate was 57.8%. Moreover, no significant decrease in body weight was observed in nude mice of each group during dosing, as shown in Figure 5.

### 2. In vivo efficacy assay of Example 40 against prostate cancer

DU145 cells were inoculated subcutaneously at the right axilla of BALB/c nude mice. When the tumor grew to about 85 mm³, 28 tumor-bearing nude mice in good growth condition and with good tumor size uniformity were selected and divided into four groups, with seven mice in each group, i.e., the model group, the low-dose group of Example 40 (80 mg/kg), the high-dose group of Example 40 (200 mg/kg), and the positive drug enzalutamide group (80 mg/kg). They were administered once a day. Tumor diameters were measured every other day and nude mice were weighed while tumor diameters were measured. The nude mice were euthanized 21 days after dosing and tumor blocks were surgically removed and weighed.

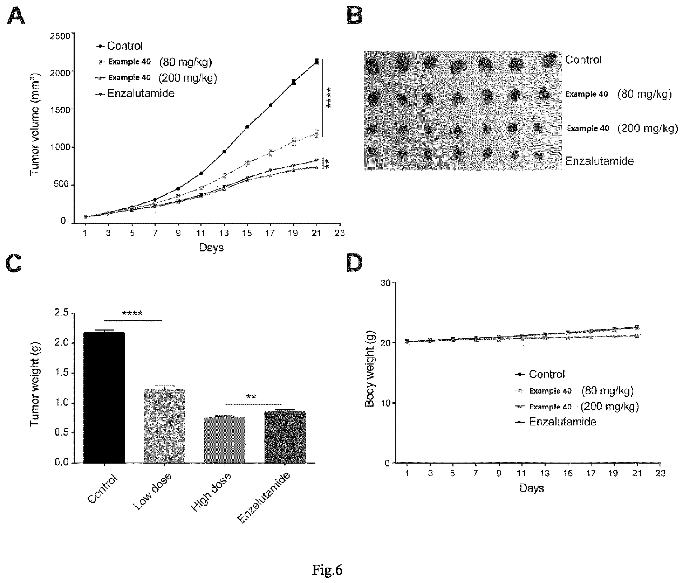

The experimental result show that that T/C(%) of human prostate cancer cell DU145 xenografts in nude mice in the low dose group of Example 40 is 55.3%, and the tumor inhibition rate is 43.6%; Example 40 T/C(%) of human prostate cancer cell DU145 xenografts in nude mice in the high dose group was 34.8%, and the tumor inhibition rate was 65.0%. The T/C(%) of human prostate cancer cell DU145 xenografts in nude mice treated with the positive drug enzalutamide was 38.8%, and the tumor inhibition rate was 60.9%, as shown in Figure 6.

## Claims

1. A compound represented by Formula (P), or a stereoisomer thereof, or a pharmaceutically acceptable salt thereof 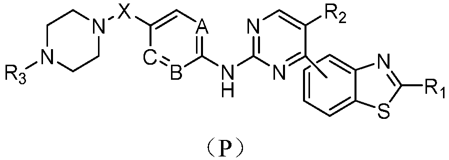 wherein
the said X is each independently selected from: O, NH, S(O)₂, C(O) or -(CH₂)ₙ-; n is 0, or 1, or 2;
the said A, B, C is each independently selected from C or N;
the said R₁ is each independently selected from: hydrogen, deuterium, halogen, hydroxy, mercapto, cyano, nitro, C₂-C₈ alkenyl, C₂-C₈ alkynyl, C₁-C₈ alkyl, halo-C₁-C₈ alkyl, C₁-C₈ alkoxy, C₃-C₈ cycloalkyl, C₆-C₁₀ aryl, C₅-C₁₀ heteroaryl, C₄-C₈ heterocyclyl or -NR₄R₅, wherein the said R₄, R₅ is each independently selected from: H, C₁-C₈ alkyl, C₃-C₈ cycloalkyl, C₆-C₁₀ aryl, C₅-C₁₀ heteroaryl or C₄-C₈ heterocyclyl;
the said R₂ is each independently selected from hydrogen, deuterium, hydroxy, mercapto, cyano, halogen, nitro, C₃-C₈ cycloalkyl, C₁-C₈ alkyl or C₁-C₈ alkoxy ;
the said R₃ is each independently selected from hydrogen, deuterium, C₁-C₈ alkyl, halo-C₁-C₈ alkyl, C₁-C₈ alkoxy, C₃-C₈ cycloalkyl, -C(O)OC₁-C₈ alkyl, -C(O)C₃-C₈ cycloalkyl, C₆-C₁₀ aryl, C₃-C₁₀ heteroaryl, C₄-C₈ heterocyclyl or -C(O) R₆;
the said R₆ is each independently selected from C₁-C₈ alkyl, C₁-C₈ alkoxy, C₃-C₈ cycloalkyl or C₆-C₁₀ aryl.

2. The compound according to claim 1, or a stereoisomer thereof, or a pharmaceutically acceptable salt thereof, wherein the said compound is a compound represented by Formula (P-I) or (P-II), or a stereoisomer thereof, or a pharmaceutically acceptable salt thereof: 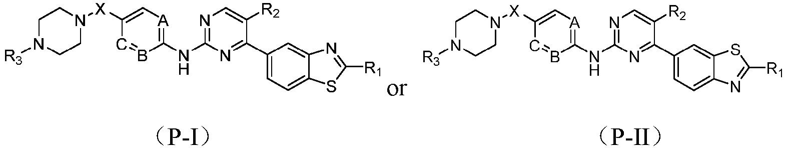 wherein
the said X is each independently selected from O, NH, S(O)₂, C(O) or -(CH₂)ₙ-; n is 0, or 1, or 2;
the said A, B, C is each independently selected from C or N;
the said R₁ is each independently selected from: hydrogen, deuterium, halogen, hydroxy, mercapto, cyano, nitro, C₂-C₈ alkenyl, C₂-C₈ alkynyl, C₁-C₈ alkyl, halo-C₁-C₈ alkyl, C₁-C₈ alkoxy, C₃-C₈ cycloalkyl, C₆-C₁₀ aryl, C₃-C₁₀ heteroaryl, C₄-C₈ heterocyclyl or -NR₄R₅, wherein R₄, R₅ is each independently selected from: H, C₁-C₈ alkyl, C₃-C₈ cycloalkyl, C₆-C₁₀ aryl, C₃-C₁₀ heteroaryl or C₄-C₈ heterocyclyl;
the said R₂ is each independently selected from hydrogen, deuterium, hydroxy, mercapto, cyano, halogen, nitro, C₃-C₈ cycloalkyl, C₁-C₈ alkyl or C₁-C₈ alkoxy ;
the said R₃ is each independently selected from hydrogen, deuterium, C₁-C₈ alkyl, halo-C₁-C₈ alkyl, C₁-C₈ alkoxy, C₃-C₈ cycloalkyl, -C(O)OC₁-C₈ alkyl, -C(O)C₃-C₈ cycloalkyl, C₆-C₁₀ aryl, C₃-C₁₀ heteroaryl, C₄-C₈ heterocyclyl or -C(O) R₆;
the said R₆ is each independently selected from C₁-C₈ alkyl, C₁-C₈ alkoxy, C₃-C₈ cycloalkyl or C₆-C₁₀ aryl.

3. The compound according to claim 2, or a stereoisomer thereof, or a pharmaceutically acceptable salt thereof, wherein
the said X is each independently selected from C(O) or -(CH₂)ₙ-; n is 1;
the said A, B, C is each independently selected from C or N;
the said R₁ is each independently selected from hydrogen, halogen, C₁-C₈ alkyl, hydroxy, -NR₄R₅, wherein R₄, R₅ is selected from H, C₁-C₆ alkyl or C₄-C₈ heterocyclyl;
the said R₂ is each independently selected from hydrogen, halogen, hydroxy, C₁-C₆ alkyl or C₁-C₆ alkoxy ;
the said R₃ is each independently selected from hydrogen, C₁-C₆ alkyl, -C(O)OC₁-C₆ alkyl, -C(O)C₃-C₆ cycloalkyl, C₆-C₁₀ aryl, or -C(O) R₆;
the said R₆ is each independently selected from C₁-C₆ alkyl, C₁-C₆ alkoxy, C₃-C₆ cycloalkyl or C₆-C₁₀ aryl.

4. The compound according to claim 2, or a stereoisomer thereof, or a pharmaceutically acceptable salt thereof, wherein
the said X is each independently selected from C(O) or -CH₂-;
the said A, B, C is each independently selected from C or N;
the said R₁ is selected from hydrogen, methyl or -NR₄R₅, wherein R₄, R₅ is each independently selected from hydrogen, methyl, ethyl, propyl, isopropyl, cyclopentyl or cyclohexyl;
the said R₂ is each independently selected from hydrogen, methyl, ethyl, propyl, methoxy, fluoro or isopropyl;
the said R₃ is each independently selected from hydrogen, methyl, ethyl, propyl, isopropyl, n-butyl, isobutyl, -C(O)OC₁-C₆ alkyl, phenyl or -C(O)R₆;
the said R₆ is each independently selected from methyl, ethyl, methoxy, ethoxy or cyclopropyl.

5. The compound according to any one of claims 1 to 4, or a stereoisomer thereof, or a pharmaceutically acceptable salt thereof, wherein the said compound is selected from the compounds with following structures, or a stereoisomer thereof, or a pharmaceutically acceptable salt thereof:
| Structure Formula | Structure Formula |
|---|---|
| 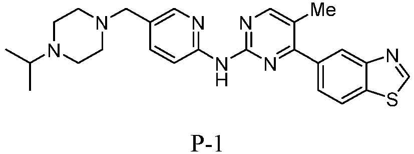 | 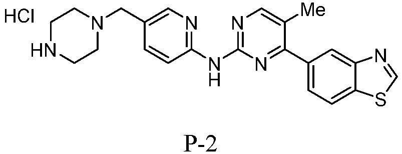 |
| 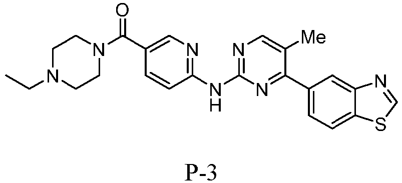 | 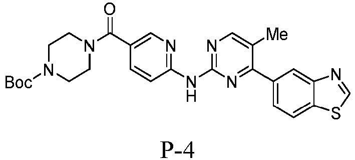 |
| 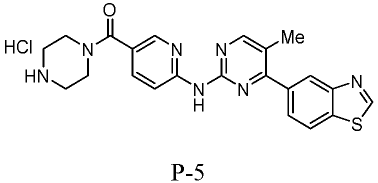 | 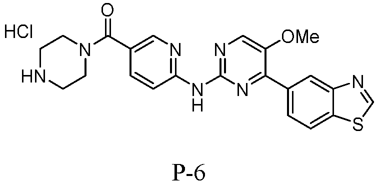 |
| 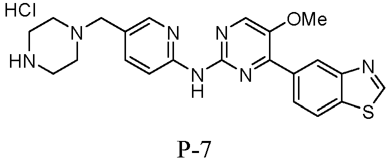 | 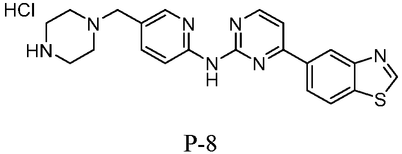 |
| 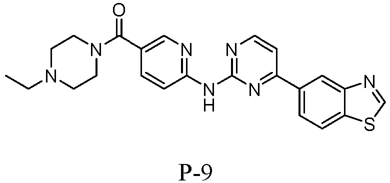 | 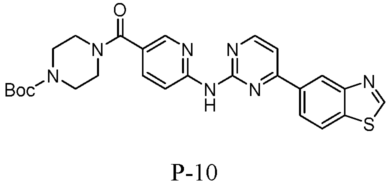 |
| 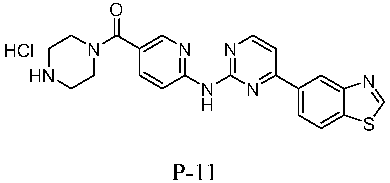 | 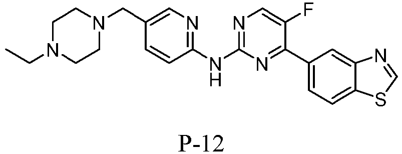 |
| 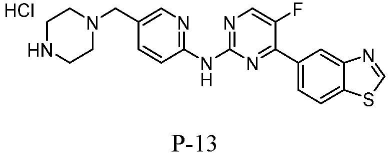 | 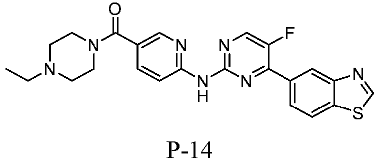 |
| 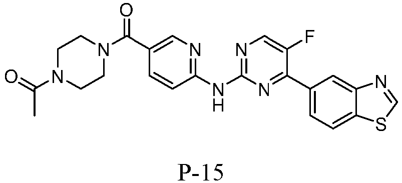 | 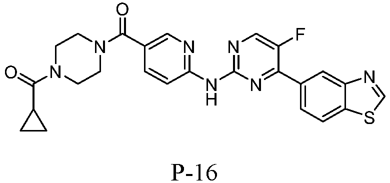 |
| 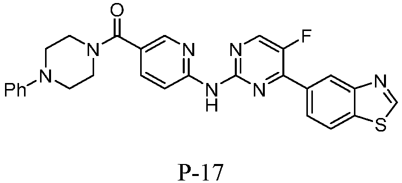 | 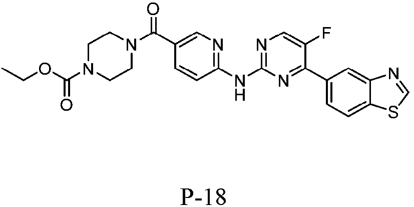 |
| 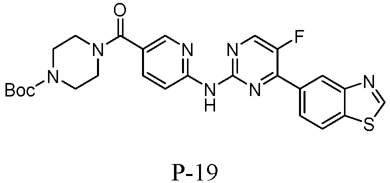 | 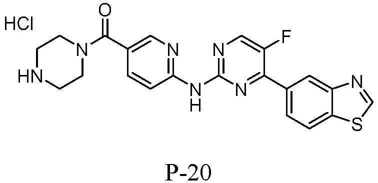 |
| 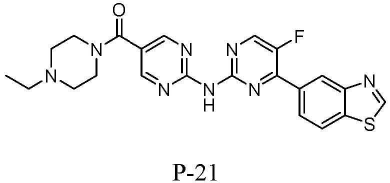 | 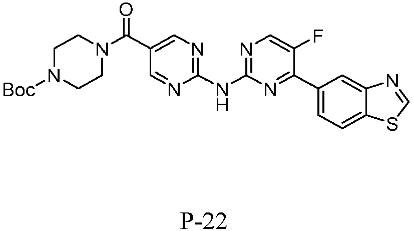 |
| 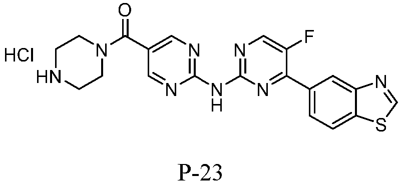 | 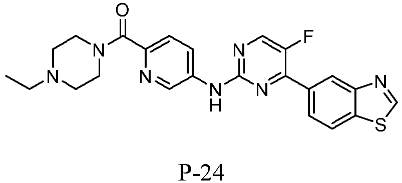 |
| 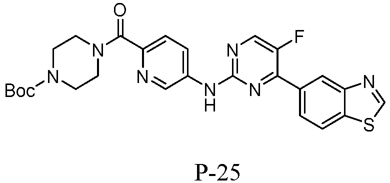 | 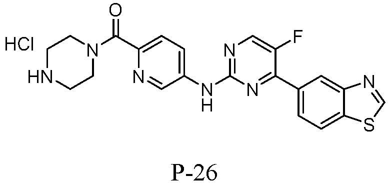 |
| 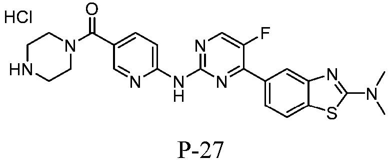 | 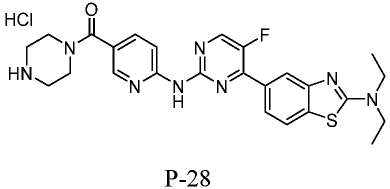 |
| 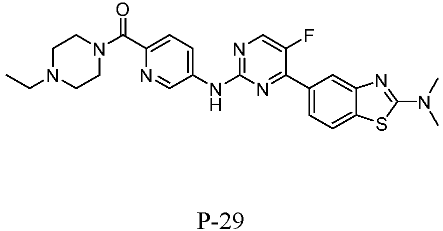 | 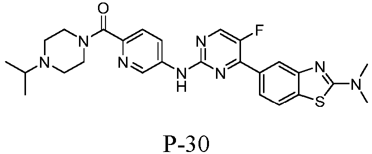 |
| 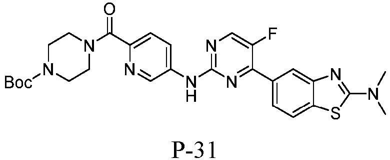 | 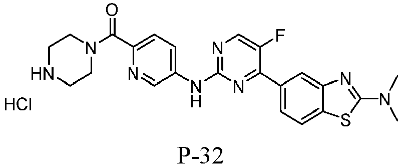 |
| 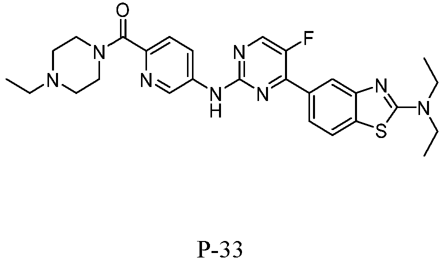 | 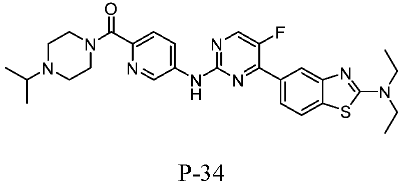 |
| 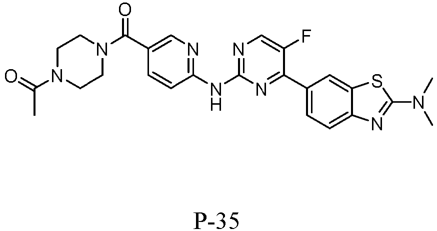 | 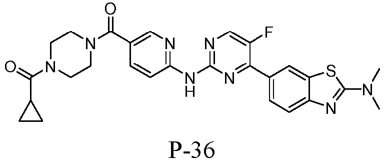 |
| 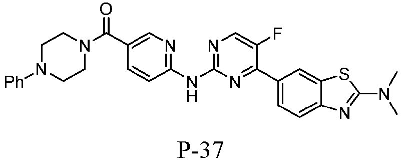 | 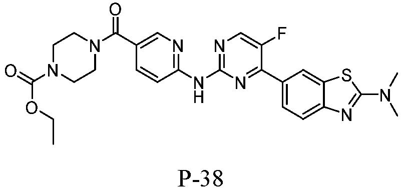 |
| 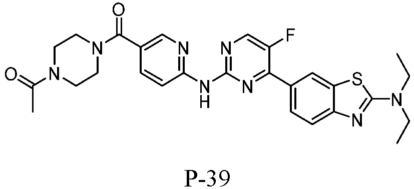 | 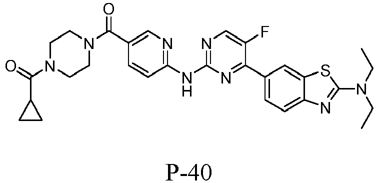 |
| 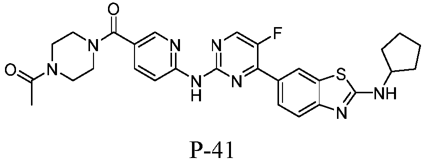 | 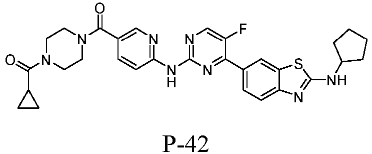 |
| 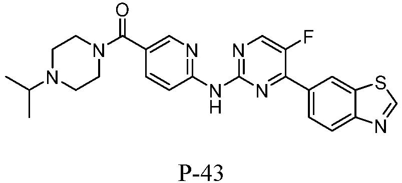 | 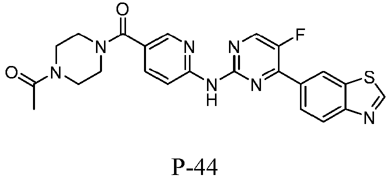 |
| 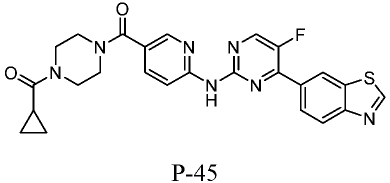 | 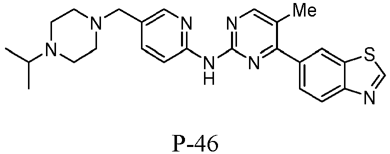 |
| 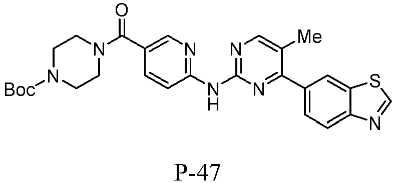 | 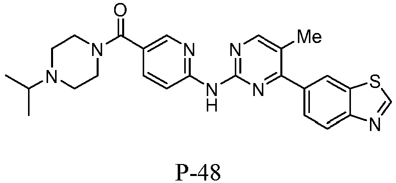 |
| 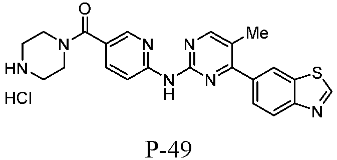 | 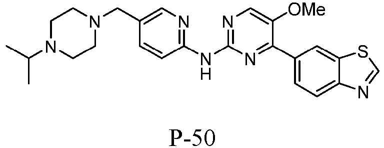 |
| 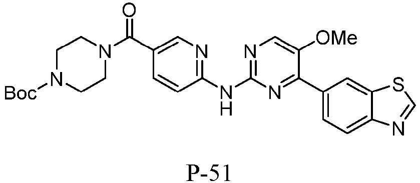 | 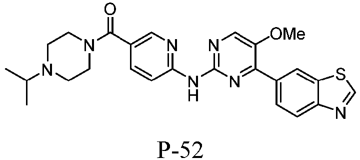 |
| 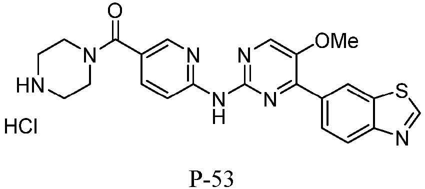 | 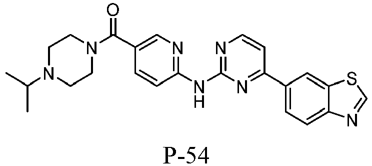 |
| 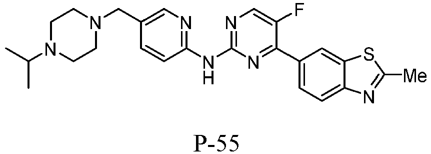 | 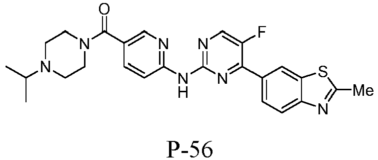 |
| 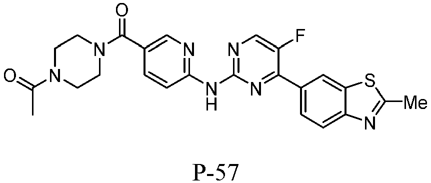 | 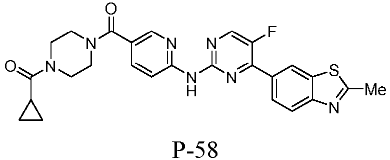 |

6. The compound according to any one of claims 1 to 5, or a stereoisomer thereof, or a pharmaceutically acceptable salt thereof, wherein the pharmaceutically acceptable salt is a salt formed by the compound represented by Formula (P) and hydrochloric acid, sulfuric acid, phosphoric acid, methanesulfonic acid, acetic acid, trichloroacetic acid, propionic acid, butyric acid, maleic acid, p-toluenesulfonic acid, malic acid, malonic acid, cinnamic acid, citric acid, fumaric acid, camphoric acid, gluconic acid, aspartic acid and tartaric acid.

7. A preparation method for the compound of any one of claims 1 to 6, or a stereoisomer thereof, or a pharmaceutically acceptable salt thereof, including a step of subjecting Intermediate Compound (A) and Intermediate Compound (B) to a coupling reaction under a catalyst: 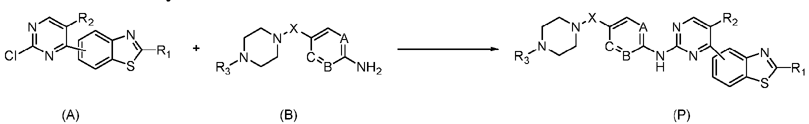
wherein, the definitions of the said substituents X, A, B, C, R₁, R₂ and R₃ are identical to any one of claims 1 to 6;
the said catalyst is a palladium catalyst;
the said reaction is conducted under argon protection atmosphere; and
the reaction temperature is 55 to 125°C, and the reaction time is 5 to24 hours.

8. A pharmaceutical composition, comprising the compound of any one of claims 1 to 6, or a stereoisomer thereof, or a pharmaceutically acceptable salt thereof.

9. A use of the compound of any one of claims 1 to 6, or a stereoisomer thereof, or a pharmaceutically acceptable salt thereof, or the pharmaceutical composition of claim 8 in the preparation of a medicament of an inhibitor targeting DYRK2.

10. A use of the compound of any one of claims 1 to 6, or a stereoisomer thereof, or a pharmaceutically acceptable salt thereof, or the pharmaceutical composition of claim 8 in the preparation of a medicament for treating cancer or tumor related disease, wherein the said cancer or tumor related disease is lung cancer, leukemia, breast cancer, prostate cancer, multiple myeloma, liver cancer, gastric cancer, bone cancer, brain cancer, head and neck cancer, intestinal cancer, pancreatic cancer, bladder cancer, testicular cancer, ovarian cancer or endometrial cancer.
